# EUROPÄISCHE PATENTSCHRIFT

(11) **EP 3 017 016 B1**
(45) Veröffentlichungstag und Bekanntmachung des Hinweises auf die Patenterteilung: **15.04.2020**
(21) Anmeldenummer: 14733521.0
(22) Anmeldetag: 11.06.2014
(51) Int. Cl.: C09K 11/06, C07D 471/00, C07D 487/00, H05B 33/10

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 02.07.2013 EP 13003344
(43) Veröffentlichungstag der Anmeldung: 11.05.2016
(73) Patentinhaber: Merck Patent GmbH, 64293 Darmstadt (DE)
(72) Erfinder: JATSCH, Anja, 60489 Frankfurt am Main (DE); PFLUMM, Christof, 64291 Darmstadt (DE); EBERLE, Thomas, 76829 Landau (DE); PARHAM, Amir, Hossain, 60486 Frankfurt am Main (DE); KROEBER, Jonas, Valentin, 60311 Frankfurt am Main (DE); STOESSEL, Philipp, 60487 Frankfurt am Main (DE); KAISER, Joachim, 64289 Darmstadt (DE)
(86) Internationale Anmeldenummer: PCT/EP2014/001587
(87) Internationale Veröffentlichungsnummer: WO 2015/000549

(56) Entgegenhaltungen:
- EP-A1- 1 956 022
- DE-A1-102009 048 791
- YIHUA JIANG ET AL: "Multibranched triarylamine end-capped triazines with aggregation-induced emission and large two-photon absorption cross-sections", CHEMICAL COMMUNICATIONS, Bd. 46, Nr. 26, 1. Januar 2010 (2010-01-01), Seite 4689, XP55130977, ISSN: 1359-7345, DOI: 10.1039/c0cc00803f

## Beschreibung

Die vorliegende Erfindung betrifft eine Verbindung einer Formel (I), in der eine elektronenarme Gruppe und eine Arylaminogruppe über eine Zwischengruppe miteinander verbunden sind. Die Verbindung der Formel (I) eignet sich als Funktionsmaterial in elektronischen Vorrichtungen.

Unter elektronischen Vorrichtungen im Sinne dieser Anmeldung werden insbesondere sogenannte organische elektronische Vorrichtungen verstanden (organic electronic devices), welche organische Halbleitermaterialien als Funktionsmaterialien enthalten. Nochmals insbesondere werden darunter organische Elektrolumineszenzvorrichtungen (OLEDs) und andere elektronische Vorrichtungen verstanden, welche im Folgenden bei der detaillierten Beschreibung der Erfindung aufgeführt sind.

Allgemein wird unter der Bezeichnung OLED eine elektronische Vorrichtung verstanden, welche mindestens ein organisches Material enthält und unter Anlegen von elektrischer Spannung Licht emittiert. Der genaue Aufbau von OLEDs ist unter anderem in US 4539507, US 5151629, EP 0676461 und WO 98/27136 beschrieben.

Bei elektronischen Vorrichtungen, insbesondere OLEDs, besteht großes Interesse an der Verbesserung der Leistungsdaten, insbesondere Lebensdauer und Effizienz und Betriebsspannung. Eine wichtige Rolle spielen dabei organische Emitterschichten, insbesondere die darin enthaltenen Matrixmaterialien, und organische Schichten mit elektronentransportierender Funktion.

Zur Lösung dieser technischen Aufgabe werden kontinuierlich neue Materialien gesucht, die sich zur Verwendung als Matrixmaterialien in emittierenden Schichten, insbesondere phosphoreszierenden emittierenden Schichten, eignen. Weiterhin werden Materialien mit elektronentransportierenden Eigenschaften zur Verwendung in entsprechenden Funktionsschichten gesucht.

Phosphoreszierende emittierende Schichten im Sinne der vorliegenden Anmeldung sind solche organischen Schichten, welche mindestens eine phosphoreszierende Emitterverbindung enthalten.

Vom Begriff phosphoreszierende Emitter sind gemäß der vorliegenden Anmeldung Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, wie einem Quintett-Zustand.

Unter einem Matrixmaterial wird für den Fall der emittierenden Schicht ein Material verstanden, welches keine Emitterverbindung ist. Emitterverbindungen einer emittierenden Schicht sind Verbindungen, welche bei Betrieb der Vorrichtung Licht emittieren.

Allgemein, insbesondere bei anderen Funktionsschichten als emittierenden Schichten, wird unter einem Matrixmaterial in einem System enthaltend zwei Materialien das Material verstanden, dessen Anteil in der Mischung der größere ist. Entsprechend wird unter einem Dotanden in einem System enthaltend zwei Materialien das Material verstanden, dessen Anteil in der Mischung der kleinere ist.

Im Stand der Technik bekannt ist die Verwendung von Verbindungen in OLEDs, welche eine Triazingruppe und eine Arylaminogruppe enthalten, wobei zwischen den beiden Gruppen bestimmte verbindende Gruppen vorliegen, beispielsweise Biphenylengruppen (vgl. JP 2002-193952, JP 2010-134121 und Q. Wang et al., J. Mat. Chem. C, 2013, 1, 2224-2232). Die Verbindungen sind dadurch gekennzeichnet, dass am Stickstoffatom nur kleine aromatische Ringsysteme gebunden sind, wie beispielsweise Phenyl. Im Stand der Technik sind auch Verbindungen bekannt, welche eine Triazingruppe und eine Triarylaminogruppe enthalten, wobei die drei Arylgruppen der Triarylaminogruppe miteinander verbunden sind (vgl. DE 102009048791, EP1956022). Auch bekannt im Stand der Technik sind Verbindungen, welche eine Triazingruppe und mindestens drei Arylaminogruppen enthalten (vgl. Y. Jiang et al, Chem. Comm., Bd. 46, Nr. 26, 2010, 4689).

Es besteht weiterhin Bedarf an alternativen Verbindungen, die sich als Funktionsmaterialien in elektronischen Vorrichtungen eignen. Insbesondere besteht Bedarf an Verbindungen, welche als Funktionsmaterialien in elektronischen Vorrichtungen eine hohe Lebensdauer und eine hohe Leistungseffizienz der Vorrichtungen bewirken, insbesondere bei Verwendung als Matrixmaterialien in phosphoreszierenden emittierenden Schichten. Weiterhin besteht Bedarf an Materialien, die eine geringe Betriebsspannung bei Verwendung in elektronischen Vorrichtungen bewirken. Nochmals weiterhin besteht Bedarf an Materialien, die einen geringen Roll-off, d.h. einen geringen Abfall der Leistungseffizienz der Vorrichtung bei hohen Leuchtdichten, bewirken.

Es wurde nun unerwarteterweise gefunden, dass Verbindungen, die eine Triazingruppe und eine Arylaminogruppe und eine diese Gruppen verbindende Gruppe enthalten, wobei die Aminogruppe mit mindestens einem großen aromatischen oder heteroaromatischen Ringsystem substituiert ist, eine oder mehrere der oben genannten technischen Aufgaben, bevorzugt alle der oben genannten technischen Aufgaben, lösen.

Gegenstand der vorliegenden Erfindung ist daher eine Verbindung der Formel (I) 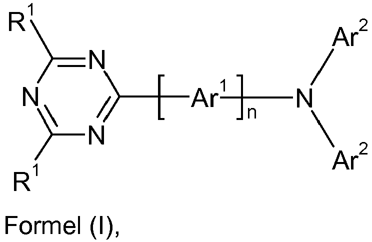 wobei gilt:
- Ar¹: ist bei jedem Auftreten gleich oder verschieden ein aromatisches oder heteroaromatisches Ringsystem mit 5 bis 24 aromatischen Ringatomen, das mit einem oder mehreren Resten R² substituiert sein kann;
- Ar²: ist bei jedem Auftreten gleich oder verschieden ein aromatisches oder heteroaromatisches Ringsystem mit 5 bis 24 aromatischen Ringatomen, das mit einem oder mehreren Resten R² substituiert sein kann, wobei mindestens eine Gruppe Ar² in der Verbindung der Formel (I) eine Gruppe Ar²* darstellt;
- Ar²*: ist bei jedem Auftreten gleich oder verschieden ein aromatisches oder heteroaromatisches Ringsystem mit 12 bis 24 aromatischen Ringatomen, das mit einem oder mehreren Resten R² substituiert sein kann, wobei das aromatische Ringsystem keine kondensierte Aryl- oder Heteroarylgruppe mit mehr als 10 aromatischen Ringatomen umfasst;
- R¹: ist bei jedem Auftreten gleich oder verschieden gewählt aus einem aromatischen oder heteroaromatischen Ringsystem mit 5 bis 30 aromatischen Ringatomen, das mit einem oder mehreren Resten R³ substituiert sein kann;
- R²: ist bei jedem Auftreten gleich oder verschieden gewählt aus H, einer geradkettigen Alkylgruppe mit 1 bis 10 C-Atomen oder einer verzweigten Alkylgruppe mit 3 bis 10 C-Atomen, wobei die oben genannten Gruppen jeweils mit einem oder mehreren Resten R³ substituiert sein können, oder einem aromatischen oder heteroaromatischen Ringsystem mit 5 bis 20 aromatischen Ringatomen, das jeweils mit einem oder mehreren Resten R³ substituiert sein kann;
- R³: ist bei jedem Auftreten gleich oder verschieden gewählt aus H, einer geradkettigen Alkylgruppe mit 1 bis 10 C-Atomen oder einer verzweigten Alkylgruppe mit 3 bis 10 C-Atomen, oder einem aromatischen oder heteroaromatischen Ringsystem mit 5 bis 20 aromatischen Ringatomen;
- n: ist gleich 1, 2, 3 oder 4;
wobei für n=1 die Gruppe Ar¹ nicht wahlweise mit R² substituiertes Phenylen oder wahlweise mit R² substituiertes Carbazol ist; und
wobei Ar¹ nicht über die Positionen 9 und 9' gebundenes, wahlweise mit R² substituiertes Fluoren ist; und
wobei die Verbindung der Formel (I) genau eine Aminogruppe enthält.

Unter den Positionen 9 und 9' des Fluorens werden die untenstehend markierten Bindungspositionen verstanden:

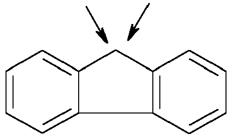

Eine Arylgruppe im Sinne dieser Offenbarung enthält 6 bis 60 aromatische Ringatome; eine Heteroarylgruppe im Sinne dieser Offenbarung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Offenbarung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Offenbarung aus zwei oder mehr miteinander kondensierten einfachen aromatischen bzw. heteroaromatischen Cyclen, und wird entsprechend auch als kondensierte Arylgruppe bzw. kondensierte Heteroarylgruppe bezeichnet.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Offenbarung enthält 6 bis 60 C-Atome im Ringsystem. Ein heteroaromatisches Ringsystem im Sinne dieser Offenbarung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Offenbarung soll ein System verstanden werden, das nicht notwendigerweise nur Aryl- oder Heteroarylgruppen enthält, sondern in dem auch mehrere Aryl- oder Heteroarylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Offenbarung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Offenbarung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von den oben unter Aryl- und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol oder Kombinationen dieser Gruppen.

Im Rahmen der vorliegenden Offenbarung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt enthält die Verbindung der Formel (I) genau eine Triazingruppe.

Bevorzugt enthält die Verbindung der Formel (I) keine kondensierte Arylgruppe mit mehr als 14 aromatischen Ringatomen, besonders bevorzugt keine kondensierte Arylgruppe mit mehr als 10 aromatischen Ringatomen.

Bevorzugt enthält die Verbindung der Formel (I) keine kondensierte Heteroarylgruppe mit mehr als 14 aromatischen Ringatomen, besonders bevorzugt keine kondensierte Heteroarylgruppe mit mehr als 10 aromatischen Ringatomen.

Es ist bevorzugt, dass Index n gleich 1, 2 oder 3 ist, besonders bevorzugt gleich 1 oder 2 ist, ganz besonders bevorzugt gleich 1 ist.

Weiterhin ist es bevorzugt, dass Ar¹ bei jedem Auftreten gleich oder verschieden ein aromatisches oder heteroaromatisches Ringsystem mit 5 bis 20 aromatischen Ringatomen ist, das mit einem oder mehreren Resten R² substituiert sein kann. Besonders bevorzugt ist Ar¹ bei jedem Auftreten gleich oder verschieden gewählt aus wahlweise mit einem oder mehreren Resten R² substituiertem Phenyl, Biphenyl, Terphenyl, Fluorenyl, Spirobifluorenyl, Indenofluorenyl, Naphthyl, Anthracenyl, Phenanthrenyl, Chrysenyl, Benzanthracenyl, Pyrenyl, Fluoranthenyl, Triphenylenyl, Furanyl, Benzofuranyl, Dibenzofuranyl, Thiophenyl, Benzothiophenyl, Dibenzothiophenyl, Indolyl, Carbazolyl, Indolocarbazolyl, Indenocarbazolyl, Pyridyl, Chinolinyl, Acridyl, Dihydroacridyl, Pyrazolyl, Imidazolyl, Benzimidazolyl, Pyridazyl, Pyrimidyl, Pyrazinyl, Phenanthrolyl, Diphenylether, Diphenylthioether, Diphenylsilylen und Diphenylmethylen.

Ganz besonders bevorzugt ist die Einheit -(Ar¹)ₙ- gewählt aus wahlweise mit einem oder mehreren Resten R² substituiertem Fluorenyl, Spirobifluorenyl, Indenofluorenyl, Naphthyl, Anthracenyl, Phenanthrenyl, Chrysenyl, Benzanthracenyl, Pyrenyl, Fluoranthenyl, Furanyl, Benzofuranyl, Dibenzofuranyl, Thiophenyl, Benzothiophenyl, Dibenzothiophenyl, Indolyl, Indolocarbazolyl, Indenocarbazolyl, Pyridyl, Chinolinyl, Acridyl, Dihydroacridyl, Pyrazolyl, Imidazolyl, Benzimidazolyl, Pyridazyl, Pyrimidyl, Pyrazinyl und Phenanthrolyl, Diphenylether, Diphenylthioether, Diphenylsilylen, Diphenylmethylen und aus Kombinationen enthaltend eine oder mehrere der oben genannten Gruppen und eine oder mehrere weitere Gruppen, gewählt aus wahlweise mit einem oder mehreren Resten R² substituiertem Phenyl und Carbazolyl.

Es ist für die Einheit -(Ar¹)ₙ- bevorzugt, dass sie eine vollständig konjugierte ungesättigte divalente Gruppe darstellt. Dies bedeutet, dass sie bevorzugt keine konjugationsunterbrechenden Gruppen wie beispielsweise Alkylengruppen aufweist, die das konjugierte System, das sich von der einen Seite zur anderen Seite der divalenten Einheit -(Ar¹)ₙ- erstreckt, unterbrechen. Bevorzugt weist die Einheit -(Ar¹)ₙ- ein durchgehend konjugiertes pi-Elektronensystem von der einen Seite zur anderen Seite der Einheit auf, so dass Triazingruppe und Aminogruppe konjugiert miteinander verbunden sind.

Besonders bevorzugte Einheiten -(Ar¹)ₙ- entsprechen den folgenden Formeln (L-1) bis (L-368), wobei die Gruppen Ar¹ entsprechend hintereinander gebunden sind und den Formeln (Ar¹-1) bis (Ar¹-17), wie unten angegeben, entsprechen:

| | n | Ar¹ | Ar¹ | Ar¹ |
|---|---|---|---|---|
| (L-1) | 1 | (Ar¹-2) | -- | -- |
| (L-2) | " | "-3) | -- | -- |
| (L-3) | " | "-4) | -- | -- |
| (L-4) | " | "-5) | -- | -- |
| (L-5) | " | "-6) | -- | -- |
| (L-6) | " | "-7) | -- | -- |
| (L-7) | " | "-8) | -- | -- |
| (L-8) | " | "-9) | -- | -- |
| (L-9) | " | "-10) | -- | -- |
| (L-10) | " | "-11) | -- | -- |
| (L-11) | " | "-12) | -- | -- |
| (L-12) | " | "-13) | -- | -- |
| (L-13) | " | "-15) | -- | -- |
| (L-14) | " | "-16) | -- | -- |
| (L-15) | " | "-17) | -- | -- |
| (L-16) | 2 | "-1) | (Ar¹-1) | -- |
| (L-17) | " | " | "-2 | -- |
| (L-18) | " | " | "-3) | -- |
| (L-19) | " | " | "-4 | -- |
| (L-20) | " | " | "-5) | -- |
| (L-21) | " | " | "-6) | -- |
| (L-22) | " | " | "-7) | -- |
| (L-23) | " | " | "-8) | -- |
| (L-24) | " | " | "-9) | -- |
| (L-25) | " | " | "-10) | -- |
| (L-26) | " | " | "-11) | -- |
| (L-27) | " | " | "-12) | -- |
| (L-28) | " | " | "-13) | -- |
| (L-29) | " | " | "-14) | -- |
| (L-30) | " | " | "-15) | -- |
| (L-31) | " | " | "-16) | -- |
| (L-32) | " | " | "-17) | -- |
| (L-33) | " | "-2) | "-1) | -- |
| (L-34) | " | " | "-2) | -- |
| (L-35) | " | " | "-3) | -- |
| (L-36) | " | " | "-4) | -- |
| (L-37) | " | " | "-5) | -- |
| (L-38) | " | " | "-6) | -- |
| (L-39) | " | " | "-7) | -- |
| (L-40) | " | " | "-8) | -- |
| (L-41) | " | " | "-9) | -- |
| (L-42) | " | " | "-10) | -- |
| (L-43) | " | " | "-11) | -- |
| (L-44) | " | " | "-12) | -- |
| (L-45) | " | " | "-13) | -- |
| (L-46) | " | " | "-14) | -- |
| (L-47) | " | " | "-15) | -- |
| (L-48) | " | " | "-16) | -- |
| (L-49) | " | " | "-17) | -- |
| (L-50) | " | "-3) | "-1) | -- |
| (L-51) | " | " | "-2) | -- |
| (L-52) | " | " | "-3) | -- |
| (L-53) | " | " | "-4) | -- |
| (L-54) | " | " | "-5) | -- |
| (L-55) | " | " | "-6) | -- |
| (L-56) | " | " | "-7) | -- |
| (L-57) | " | " | "-8) | -- |
| (L-58) | " | " | "-9) | -- |
| (L-59) | " | " | "-10) | -- |
| (L-60) | " | " | "-11) | -- |
| (L-61) | " | " | "-12) | -- |
| (L-62) | " | " | "-13) | -- |
| (L-63) | " | " | "-14) | -- |
| (L-64) | " | " | "-15) | -- |
| (L-65) | " | " | "-16) | -- |
| (L-66) | " | " | "-17) | -- |
| (L-67) | " | "-4) | "-1) | -- |
| (L-68) | " | " | "-2) | -- |
| (L-69) | " | " | "-3) | -- |
| (L-70) | " | " | "-4) | -- |
| (L-71) | " | " | "-5) | -- |
| (L-72) | " | " | "-6) | -- |
| (L-73) | " | " | "-7) | -- |
| (L-74) | " | " | "-8) | -- |
| (L-75) | " | " | "-9) | -- |
| (L-76) | " | " | "-10) | -- |
| (L-77) | " | " | "-11) | -- |
| (L-78) | " | " | "-12) | -- |
| (L-79) | " | " | "-13) | -- |
| (L-80) | " | " | "-14) | -- |
| (L-81) | " | " | "-15) | -- |
| (L-82) | " | " | "-16) | -- |
| (L-83) | " | " | "-17) | -- |
| (L-84) | " | "-5) | "-1) | -- |
| (L-85) | " | " | "-2) | -- |
| (L-86) | " | " | "-3) | -- |
| (L-87) | " | " | "-4) | -- |
| (L-88) | " | " | "-5) | -- |
| (L-89) | " | " | "-6) | -- |
| (L-90) | " | " | "-7) | -- |
| (L-91) | " | " | "-8) | -- |
| (L-92) | " | " | "-9) | -- |
| (L-93) | " | " | "-10) | -- |
| (L-94) | " | " | "-11) | -- |
| (L-95) | " | " | "-12) | -- |
| (L-96) | " | " | "-13) | -- |
| (L-97) | " | " | "-14) | -- |
| (L-98) | " | " | "-15) | -- |
| (L-99) | " | " | "-16) | -- |
| (L-100) | " | " | "-17) | -- |
| (L-101) | " | "-6) | "-1) | -- |
| (L-102) | " | " | "-2) | -- |
| (L-103) | " | " | "-3) | -- |
| (L-104) | " | " | "-4) | -- |
| (L-105) | " | " | "-5) | -- |
| (L-106) | " | " | "-6) | -- |
| (L-107) | " | " | "-7) | -- |
| (L-108) | " | " | "-8) | -- |
| (L-109) | " | " | "-9) | -- |
| (L-110) | " | " | "-10) | -- |
| (L-111) | " | " | "-11) | -- |
| (L-112) | " | " | "-12) | -- |
| (L-113) | " | " | "-13) | -- |
| (L-114) | " | " | "-14) | -- |
| (L-115) | " | " | "-15) | -- |
| (L-116) | " | " | "-16) | -- |
| (L-117) | " | " | "-17) | -- |
| (L-118) | " | "-7) | "-1) | -- |
| (L-119) | " | " | "-2) | -- |
| (L-120) | " | " | "-3) | -- |
| (L-121) | " | " | "-4) | -- |
| (L-122) | " | " | "-5) | -- |
| (L-123) | " | " | "-6) | -- |
| (L-124) | " | " | "-7) | -- |
| (L-125) | " | " | "-8) | -- |
| (L-126) | " | " | "-9) | -- |
| (L-127) | " | " | "-10) | -- |
| (L-128) | " | " | "-11) | -- |
| (L-129) | " | " | "-12) | -- |
| (L-130) | " | " | "-13) | -- |
| (L-131) | " | " | "-14) | -- |
| (L-132) | " | " | "-15) | -- |
| (L-133) | " | " | "-16) | -- |
| (L-134) | " | " | "-17) | -- |
| (L-135) | " | "-8) | "-1) | -- |
| (L-136) | " | " | "-2) | -- |
| (L-137) | " | " | "-3) | -- |
| (L-138) | " | " | "-4) | -- |
| (L-139) | " | " | "-5) | -- |
| (L-140) | " | " | "-6) | -- |
| (L-141) | " | " | "-7) | -- |
| (L-142) | " | " | "-8) | -- |
| (L-143) | " | " | "-9) | -- |
| (L-144) | " | " | "-10) | -- |
| (L-145) | " | " | "-11) | -- |
| (L-146) | " | " | "-12) | -- |
| (L-147) | " | " | "-13) | -- |
| (L-148) | " | " | "-14) | -- |
| (L-149) | " | " | "-15) | -- |
| (L-150) | " | " | "-16) | -- |
| (L-151) | " | " | "-17) | -- |
| (L-152) | " | "-9) | "-1) | -- |
| (L-153) | " | " | "-2) | -- |
| (L-154) | " | " | "-3) | -- |
| (L-155) | " | " | "-4) | -- |
| (L-156) | " | " | "-5) | -- |
| (L-157) | " | " | "-6) | -- |
| (L-158) | " | " | "-7) | -- |
| (L-159) | " | " | "-8) | -- |
| (L-160) | " | " | "-9) | -- |
| (L-161) | " | " | "-10) | -- |
| (L-162) | " | " | "-11) | -- |
| (L-163) | " | " | "-12) | -- |
| (L-164) | " | " | "-13) | -- |
| (L-165) | " | " | "-14) | -- |
| (L-166) | " | " | "-15) | -- |
| (L-167) | " | " | "-16) | -- |
| (L-168) | " | " | "-17) | -- |
| (L-169) | " | "-10) | "-1) | -- |
| (L-170) | " | " | "-2) | -- |
| (L-171) | " | " | "-3) | -- |
| (L-172) | " | " | "-4) | -- |
| (L-173) | " | " | "-5) | -- |
| (L-174) | " | " | "-6) | -- |
| (L-175) | " | " | "-7) | -- |
| (L-176) | " | " | "-8) | -- |
| (L-177) | " | " | "-9) | -- |
| (L-178) | " | " | "-10) | -- |
| (L-179) | " | " | "-11) | -- |
| (L-180) | " | " | "-12) | -- |
| (L-181) | " | " | "-13) | -- |
| (L-182) | " | " | "-14) | -- |
| (L-183) | " | " | "-15) | -- |
| (L-184) | " | " | "-16) | -- |
| (L-185) | " | " | "-17) | -- |
| (L-186) | " | "-11) | "-1) | -- |
| (L-187) | " | " | "-2) | -- |
| (L-188) | " | " | "-3) | -- |
| (L-189) | " | " | "-4) | -- |
| (L-190) | " | " | "-5) | -- |
| (L-191) | " | " | "-6) | -- |
| (L-192) | " | " | "-7) | -- |
| (L-193) | " | " | "-8) | -- |
| (L-194) | " | " | "-9) | -- |
| (L-195) | " | " | "-10) | -- |
| (L-196) | " | " | "-11) | -- |
| (L-197) | " | " | "-12) | -- |
| (L-198) | " | " | "-13) | -- |
| (L-199) | " | " | "-14) | -- |
| (L-200) | " | " | "-15) | -- |
| (L-201) | " | " | "-16) | -- |
| (L-202) | " | " | "-17) | -- |
| (L-203) | " | "-12) | "-1) | -- |
| (L-204) | " | " | "-2) | -- |
| (L-205) | " | " | "-3) | -- |
| (L-206) | " | " | "-4) | -- |
| (L-207) | " | " | "-5) | -- |
| (L-208) | " | " | "-6) | -- |
| (L-209) | " | " | "-7) | -- |
| (L-210) | " | " | "-8) | -- |
| (L-211) | " | " | "-9) | -- |
| (L-212) | " | " | "-10) | -- |
| (L-213) | " | " | "-11) | -- |
| (L-214) | " | " | "-12) | -- |
| (L-215) | " | " | "-13) | -- |
| (L-216) | " | " | "-14) | -- |
| (L-217) | " | " | "-15) | -- |
| (L-218) | " | " | "-16) | -- |
| (L-219) | " | " | "-17) | -- |
| (L-220) | " | "-13) | "-1) | -- |
| (L-221) | " | " | "-2) | -- |
| (L-222) | " | " | "-3) | -- |
| (L-223) | " | " | "-4) | -- |
| (L-224) | " | " | "-5) | -- |
| (L-225) | " | " | "-6) | -- |
| (L-226) | " | " | "-7) | -- |
| (L-227) | " | " | "-8) | -- |
| (L-228) | " | " | "-9) | -- |
| (L-229) | " | " | "-10) | -- |
| (L-230) | " | " | "-11) | -- |
| (L-231) | " | " | "-12) | -- |
| (L-232) | " | " | "-13) | -- |
| (L-233) | " | " | "-14) | -- |
| (L-234) | " | " | "-15) | -- |
| (L-235) | " | " | "-16) | -- |
| (L-236) | " | " | "-17) | -- |
| (L-237) | " | "-14) | "-1) | -- |
| (L-238) | " | " | "-2) | -- |
| (L-239) | " | " | "-3) | -- |
| (L-240) | " | " | "-4) | -- |
| (L-241) | " | " | "-5) | -- |
| (L-242) | " | " | "-6) | -- |
| (L-243) | " | " | "-7) | -- |
| (L-244) | " | " | "-8) | -- |
| (L-245) | " | " | "-9) | -- |
| (L-246) | " | " | "-10) | -- |
| (L-247) | " | " | "-11) | -- |
| (L-248) | " | " | "-12) | -- |
| (L-249) | " | " | "-13) | -- |
| (L-250) | " | " | "-14) | -- |
| (L-251) | " | " | "-15) | -- |
| (L-252) | " | " | "-16) | -- |
| (L-253) | " | " | "-17) | -- |
| (L-254) | " | "-15) | "-1) | -- |
| (L-255) | " | " | "-2) | -- |
| (L-256) | " | " | "-3) | -- |
| (L-257) | " | " | "-4) | -- |
| (L-258) | " | " | "-5) | -- |
| (L-259) | " | " | "-6) | -- |
| (L-260) | " | " | "-7) | -- |
| (L-261) | " | " | "-8) | -- |
| (L-262) | " | " | "-9) | -- |
| (L-263) | " | " | "-10) | -- |
| (L-264) | " | " | "-11) | -- |
| (L-265) | " | " | "-12) | -- |
| (L-266) | " | " | "-13) | -- |
| (L-267) | " | " | "-14) | -- |
| (L-268) | " | " | "-15) | -- |
| (L-269) | " | " | "-16) | -- |
| (L-270) | " | " | "-17) | -- |
| (L-271) | " | "-16) | "-1) | -- |
| (L-272) | " | " | "-2) | -- |
| (L-273) | " | " | "-3) | -- |
| (L-274) | " | " | "-4) | -- |
| (L-275) | " | " | "-5) | -- |
| (L-276) | " | " | "-6) | -- |
| (L-277) | " | " | "-7) | -- |
| (L-278) | " | " | "-8) | -- |
| (L-279) | " | " | "-9) | -- |
| (L-280) | " | " | "-10) | -- |
| (L-281) | " | " | "-11) | -- |
| (L-282) | " | " | "-12) | -- |
| (L-283) | " | " | "-13) | -- |
| (L-284) | " | " | "-14) | -- |
| (L-285) | " | " | "-15) | -- |
| (L-286) | " | " | "-16) | -- |
| (L-287) | " | " | "-17) | -- |
| (L-288) | " | "-17) | "-1) | -- |
| (L-289) | " | " | "-2) | -- |
| (L-290) | " | " | "-3) | -- |
| (L-291) | " | " | "-4) | -- |
| (L-292) | " | " | "-5) | -- |
| (L-293) | " | " | "-6) | -- |
| (L-294) | " | " | "-7) | -- |
| (L-295) | " | " | "-8) | -- |
| (L-296) | " | " | "-9) | -- |
| (L-297) | " | " | "-10) | -- |
| (L-298) | " | " | "-11) | -- |
| (L-299) | " | " | "-12) | -- |
| (L-300) | " | " | "-13) | -- |
| (L-301) | " | " | "-14) | -- |
| (L-302) | " | " | "-15) | -- |
| (L-303) | " | " | "-16) | -- |
| (L-304) | " | " | "-17) | -- |
| (L-305) | 3 | "-1) | "-1) | "-1) |
| (L-306) | " | " | " | "-2) |
| (L-307) | " | " | " | "-3) |
| (L-308) | " | " | " | "-4) |
| (L-309) | " | " | "-2) | "-1) |
| (L-310) | " | " | " | "-2) |
| (L-311) | " | " | " | "-3) |
| (L-312) | " | " | " | "-4) |
| (L-313) | " | " | "-3) | "-1) |
| (L-314) | " | " | " | "-2) |
| (L-315) | " | " | " | "-3) |
| (L-316) | " | " | " | "-4) |
| (L-317) | " | " | "-4) | "-1) |
| (L-318) | " | " | " | "-2) |
| (L-319) | " | " | " | "-3) |
| (L-320) | " | " | " | "-4) |
| (L-321) | " | "-2) | "-1) | "-1) |
| (L-322) | " | | " | "-2) |
| (L-323) | " | | " | "-3) |
| (L-324) | " | | " | "-4) |
| (L-325) | " | | "-2) | "-1) |
| (L-326) | " | | " | "-2) |
| (L-327) | " | | " | "-3) |
| (L-328) | " | | " | " |
| (L-329) | " | | "-3) | "-1) |
| (L-330) | " | | " | "-2) |
| (L-331) | " | | " | "-3) |
| (L-332) | " | | " | "-4) |
| (L-333) | " | | "-4) | "-1) |
| (L-334) | " | | " | "-2) |
| (L-335) | " | | " | "-3) |
| (L-336) | " | | " | "-4) |
| (L-337) | " | "-3) | "-1) | "-1) |
| (L-338) | " | " | " | "-2) |
| (L-339) | " | " | " | "-3) |
| (L-340) | " | " | " | "-4) |
| (L-341) | " | " | "-2) | "-1) |
| (L-342) | " | " | " | "-2) |
| (L-343) | " | " | " | "-3) |
| (L-344) | " | " | " | "-4) |
| (L-345) | " | " | "-3) | "-1) |
| (L-346) | " | " | " | "-2) |
| (L-347) | " | " | " | "-3) |
| (L-348) | " | " | " | "-4) |
| (L-349) | " | " | "-4) | "-1) |
| (L-350) | " | " | " | "-2) |
| (L-351) | " | " | " | "-3) |
| (L-352) | " | " | " | "-4) |
| (L-353) | " | "-4) | "-1) | "-1) |
| (L-354) | " | | " | "-2) |
| (L-355) | " | | " | "-3) |
| (L-356) | " | | " | "-4) |
| (L-357) | " | | "-2) | "-1) |
| (L-358) | " | | " | "-2) |
| (L-359) | " | | " | "-3) |
| (L-360) | " | | " | "-4) |
| (L-361) | " | | "-3) | "-1) |
| (L-362) | " | | " | "-2) |
| (L-363) | " | | " | "-3) |
| (L-364) | " | | " | "-4) |
| (L-365) | " | | "-4) | "-1) |
| (L-366) | " | | " | "-2) |
| (L-367) | " | | " | "-3) |
| (L-368) | " | | " | "-4) |

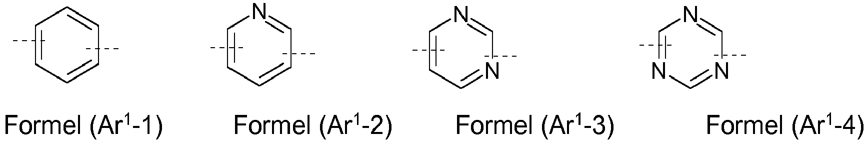

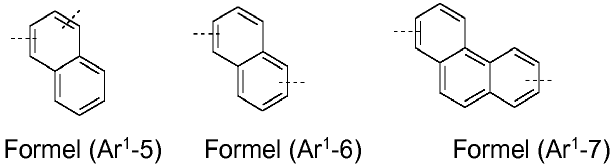

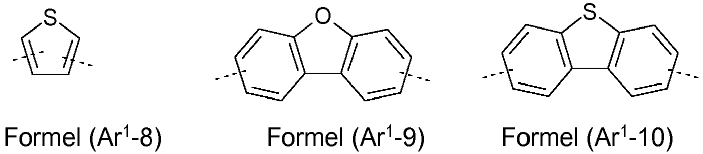

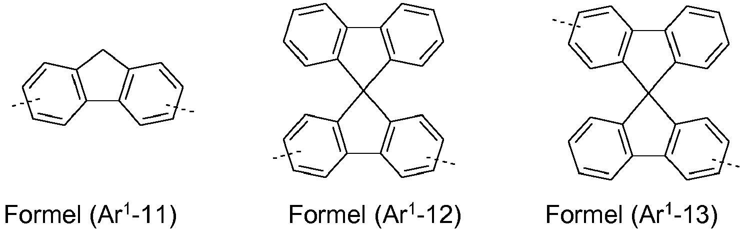

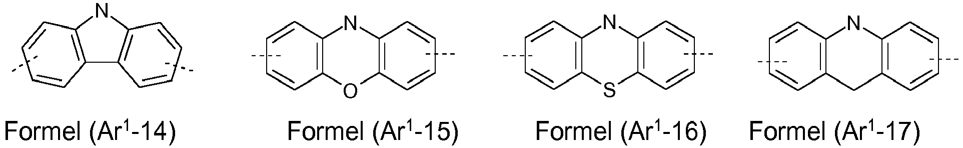

wobei die Gruppen der Formeln (Ar¹-1) bis (Ar¹-14) wahlweise mit einem oder mehreren Resten R² substituiert sein können.

Bevorzugt sind beide Gruppen Ar² gewählt aus gleichen oder verschiedenen Gruppen Ar²*.

Bevorzugt ist Ar² bei jedem Auftreten gleich oder verschieden gewählt aus einem aromatischen oder heteroaromatischen Ringsystem mit 5 bis 20 aromatischen Ringatomen, das mit einem oder mehreren Resten R² substituiert sein kann. Besonders bevorzugt ist Ar² bei jedem Auftreten gleich oder verschieden gewählt aus wahlweise mit einem oder mehreren Resten R² substituiertem Phenyl, Biphenyl, Terphenyl, Quaterphenyl, Fluorenyl, Spirobifluorenyl, Indenofluorenyl, Naphthyl, Anthracenyl, Phenanthrenyl, Chrysenyl, Benzanthracenyl, Pyrenyl, Triphenylenyl, Fluoranthenyl, Furanyl, Benzofuranyl, Dibenzofuranyl, Thiophenyl, Benzothiophenyl, Dibenzothiophenyl, Indolyl, Carbazolyl, Indolocarbazolyl, Indenocarbazolyl, Pyridyl, Chinolinyl, Acridyl, Dihydroacridyl, Pyrazolyl, Imidazolyl, Benzimidazolyl, Pyridazyl, Pyrimidyl, Pyrazinyl und Phenanthrolyl.

Bevorzugt ist Ar² bei jedem Auftreten gleich oder verschieden gewählt aus Gruppen der folgenden Formeln (Ar²-1) bis (Ar²-42) 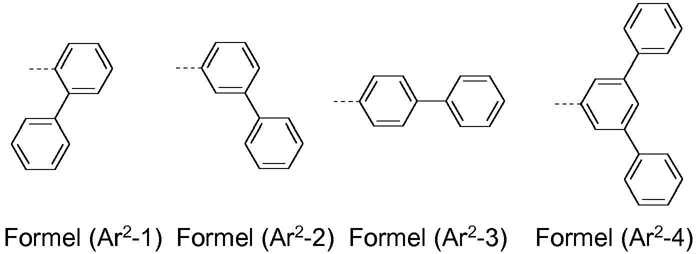 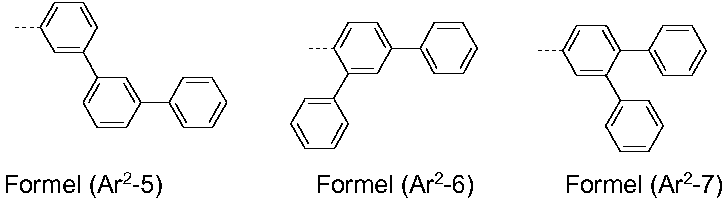 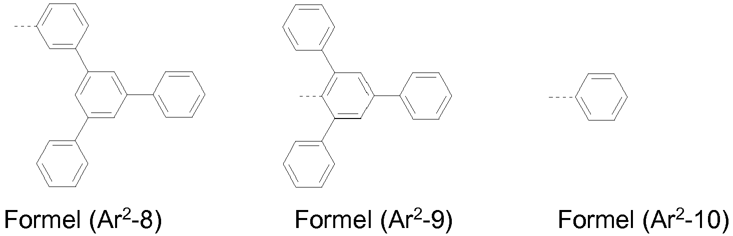 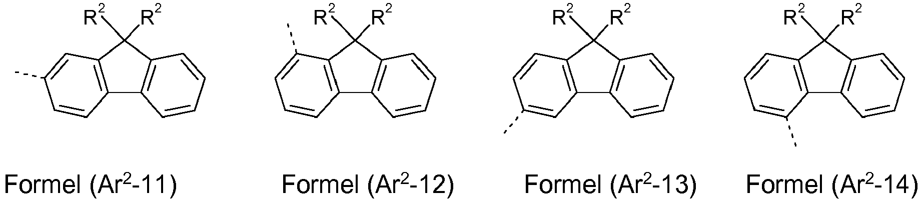 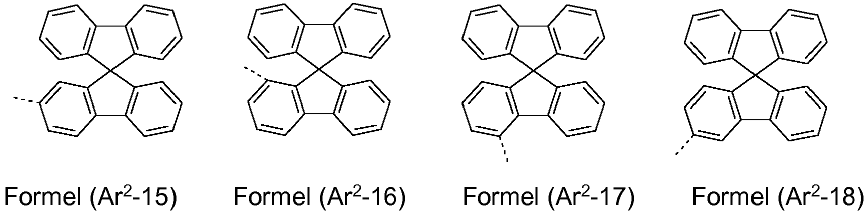 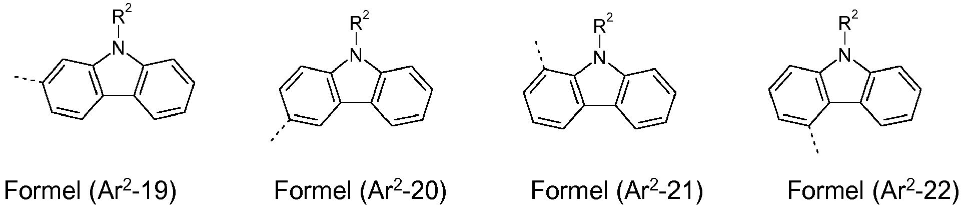 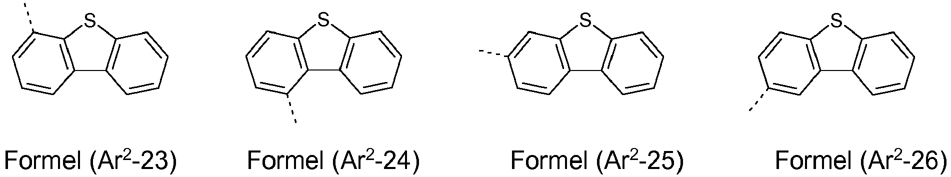 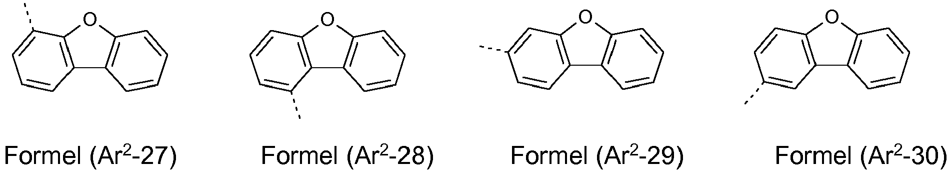 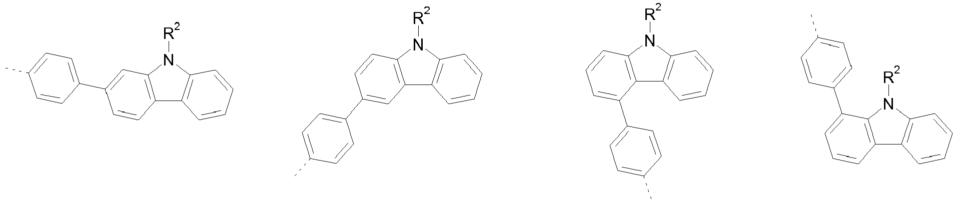 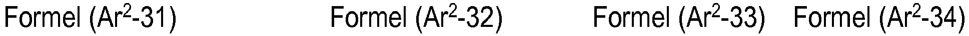 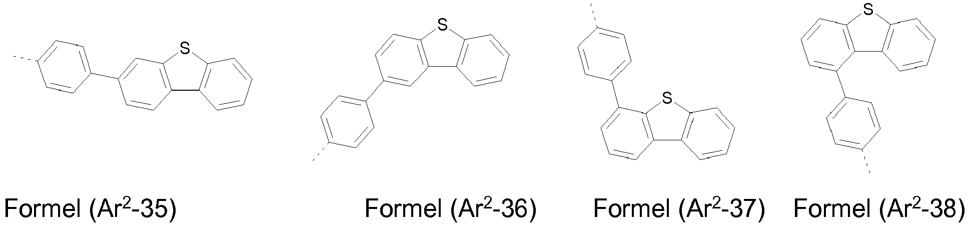 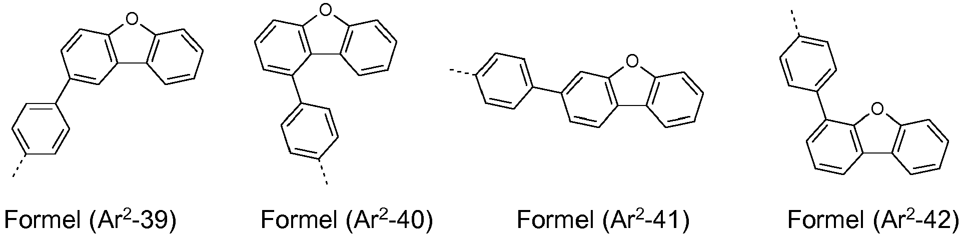 welche mit einem oder mehreren Resten R² substituiert sein können, und wobei ein oder mehrere C-Atome in den aromatischen Ringen durch N ersetzt sein können. Bevorzugt sind keine C-Atome in den aromatischen Ringen der oben genannten Gruppen durch N ersetzt.

Bevorzugt ist Ar²* gewählt aus Gruppen der Formeln (Ar²-1) bis (Ar²-9), (Ar²-11) bis (Ar²-18) und (Ar²-31) bis (Ar²-42).

Es ist für Verbindungen der Formel (I) bevorzugt, dass die bevorzugten Ausführungsformen der Gruppen miteinander kombiniert auftreten. Insbesondere bevorzugt ist es, dass die bevorzugten Ausführungsformen der Gruppen Ar¹ und der Gruppen Ar² in Formel (I) miteinander kombiniert auftreten.

Folgende Verbindungen sind Beispiele für Verbindungen gemäß Formel (I).

| | |
|---|---|
| 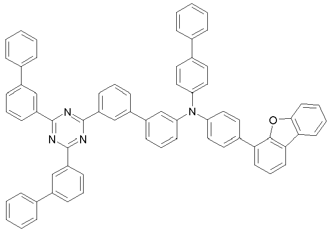 | 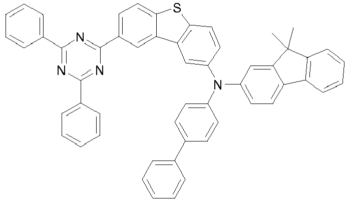 |
| 1 | 2 |
| 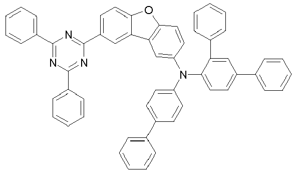 | 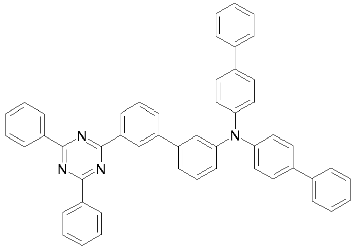 |
| 3 | 4 |
| 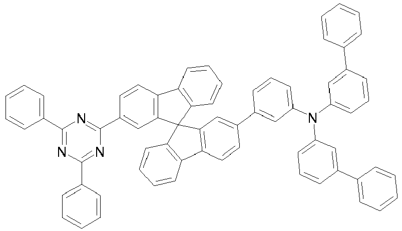 | 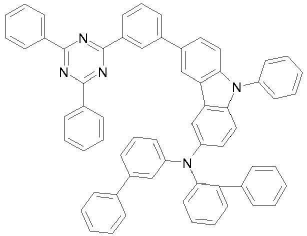 |
| 5 | 6 |
| 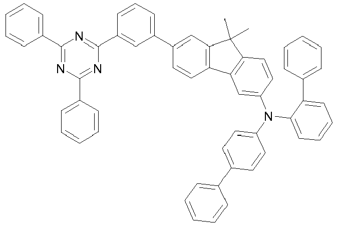 | 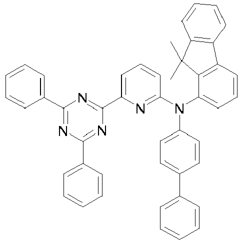 |
| 7 | 8 |
| 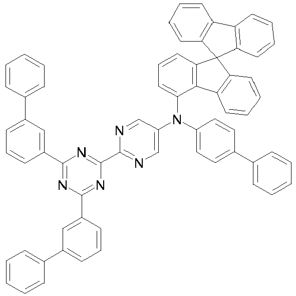 | 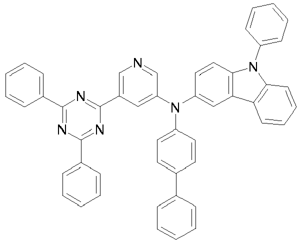 |
| 9 | 10 |
| 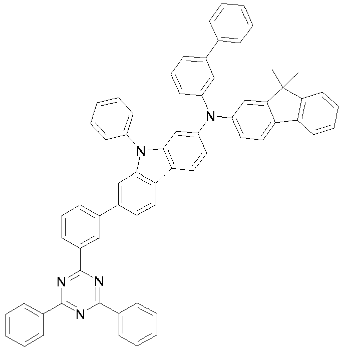 | 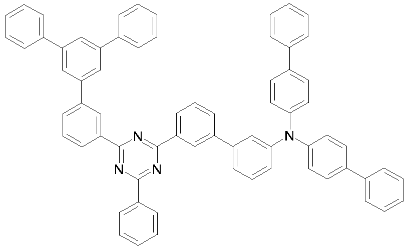 |
| 11 | 12 |
| 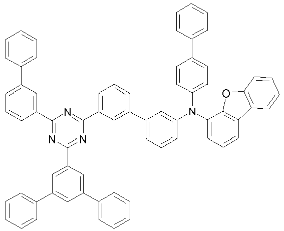 | 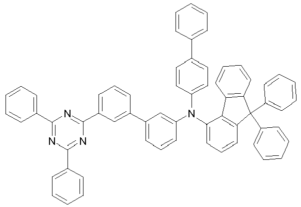 |
| 13 | 14 |
| 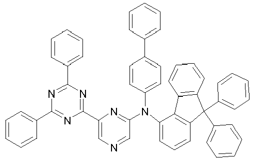 | 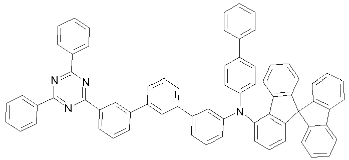 |
| 15 | 16 |
| 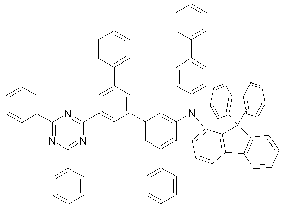 | 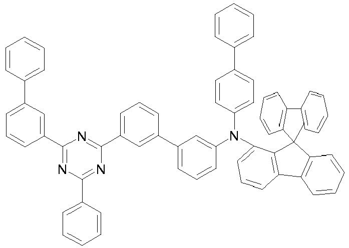 |
| 17 | 18 |
| 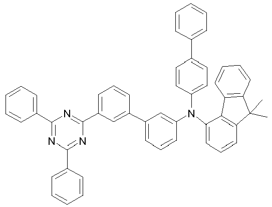 | 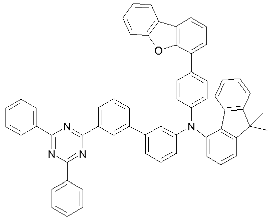 |
| 19 | 20 |
| 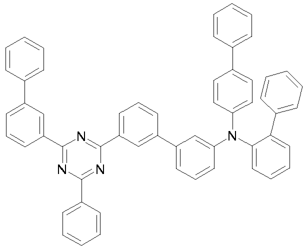 | 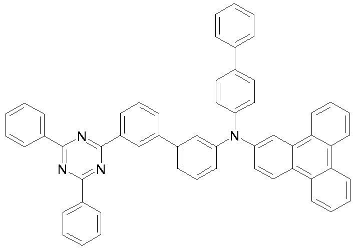 |
| 21 | 22 |
| 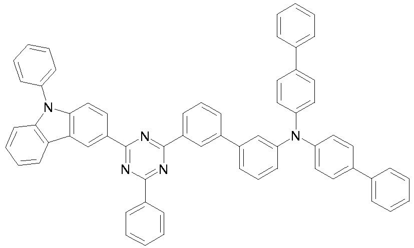 | 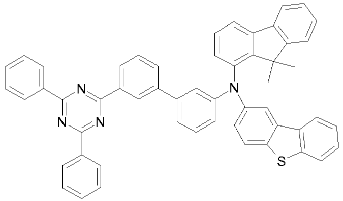 |
| 23 | 24 |
| 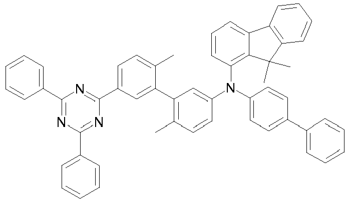 | 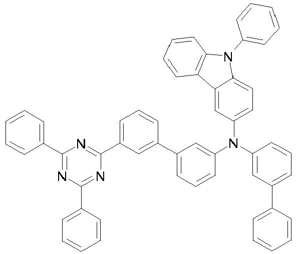 |
| 25 | 26 |
| 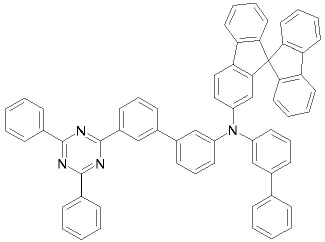 | 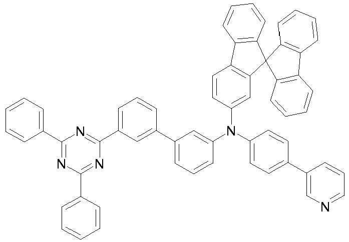 |
| 27 | 28 |
| 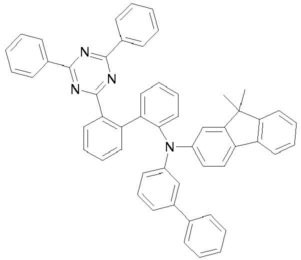 | 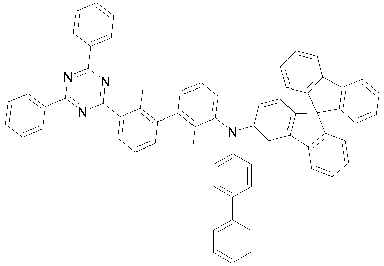 |
| 29 | 30 |
| 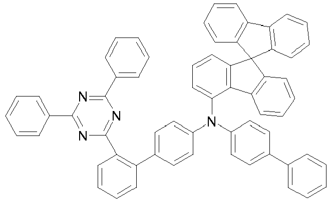 | 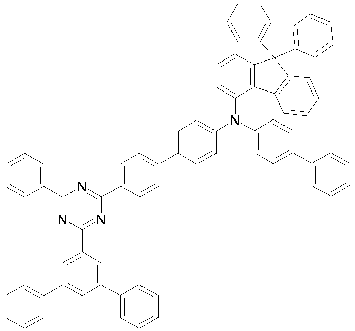 |
| 31 | 32 |
| 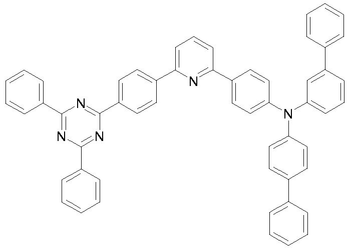 | 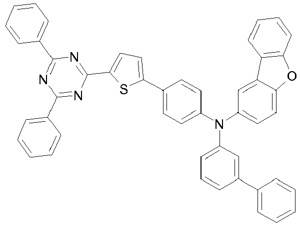 |
| 33 | 34 |
| 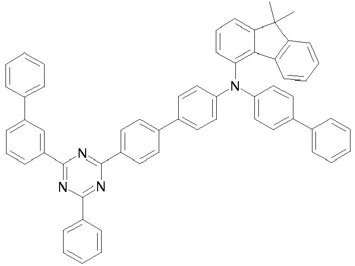 | 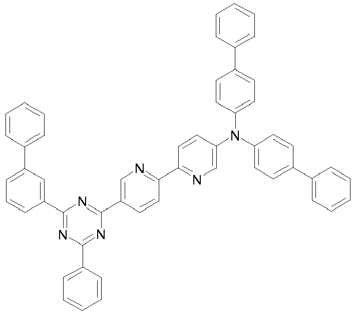 |
| 35 | 36 |
| 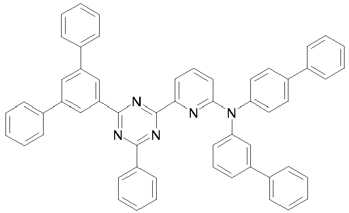 | 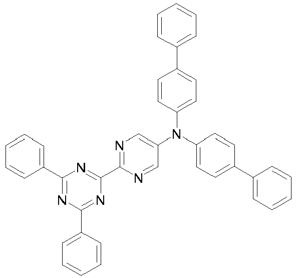 |
| 37 | 38 |
| 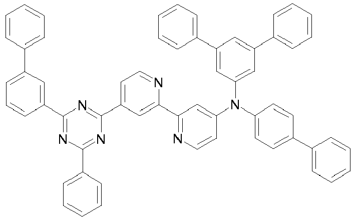 | 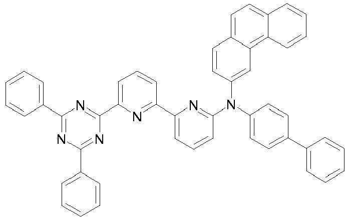 |
| 39 | 40 |
| 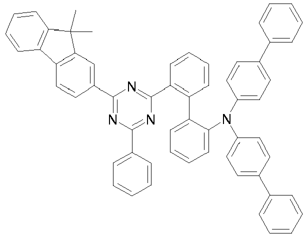 | 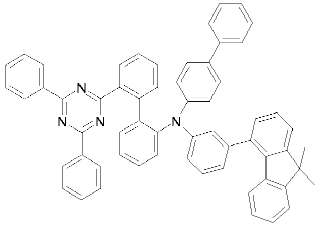 |
| 41 | 42 |
| 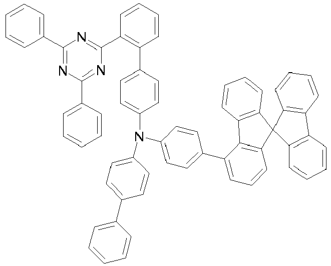 | 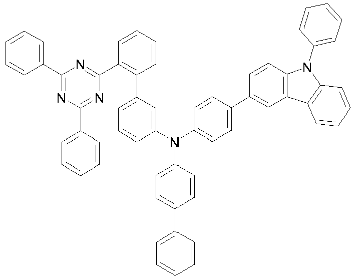 |
| 43 | 44 |
| 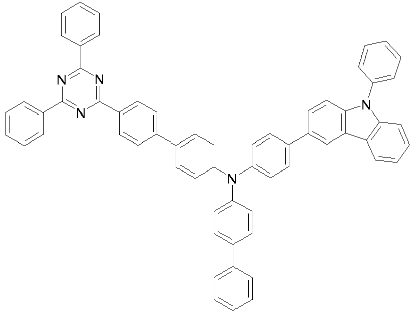 | 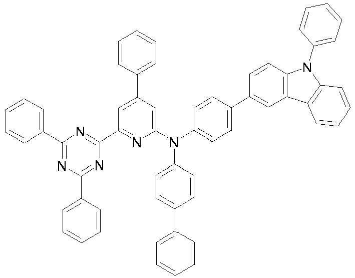 |
| 45 | 46 |
| 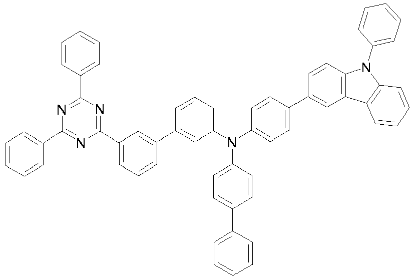 | 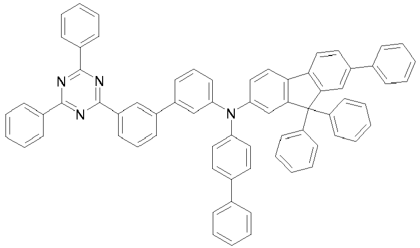 |
| 47 | 48 |
| 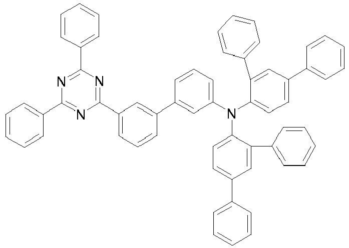 | 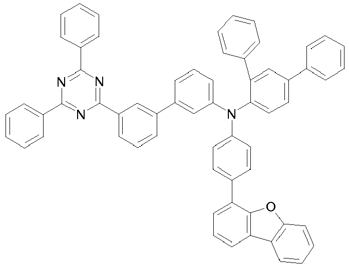 |
| 49 | 50 |
| 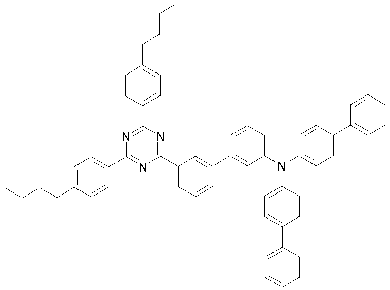 | 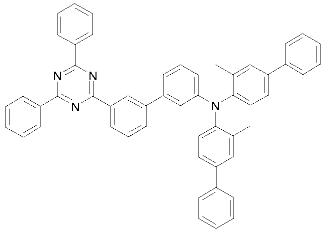 |
| 51 | 52 |
| 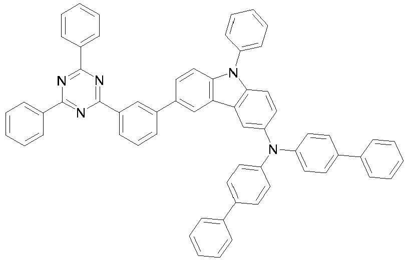 | 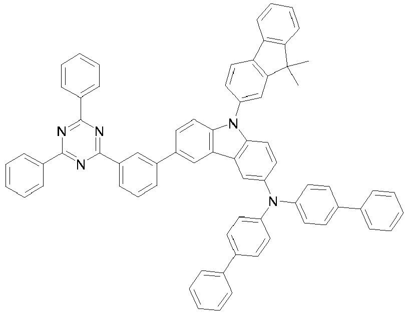 |
| 53 | 54 |
| 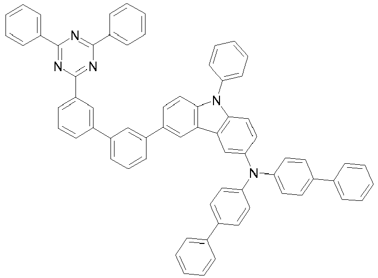 | 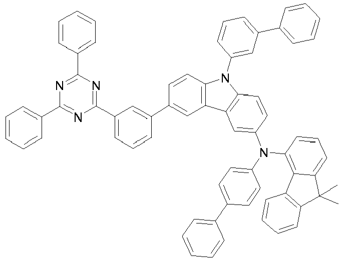 |
| 55 | 56 |
| 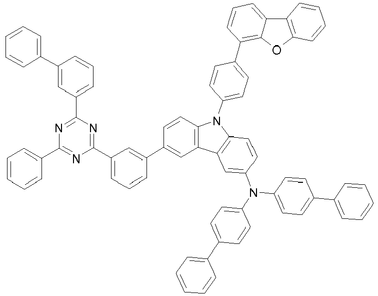 | 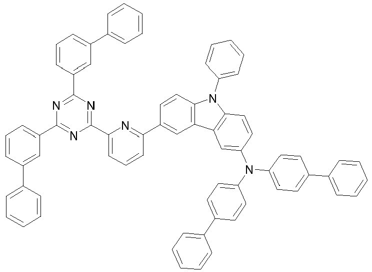 |
| 57 | 58 |
| 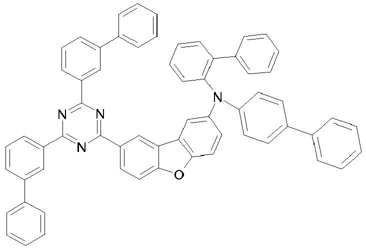 | 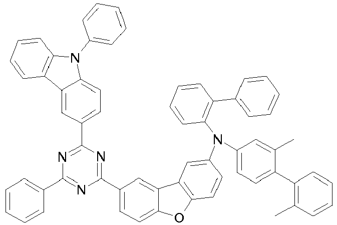 |
| 59 | 60 |
| 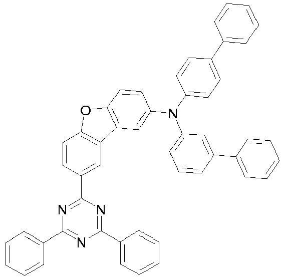 | 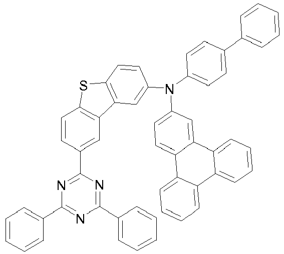 |
| 61 | 61 |
| 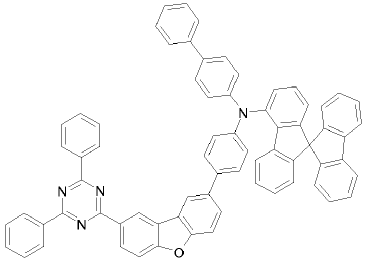 | 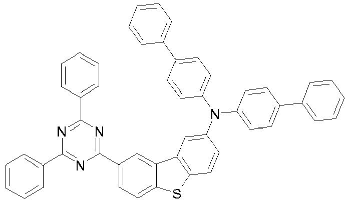 |
| 63 | 64 |
| 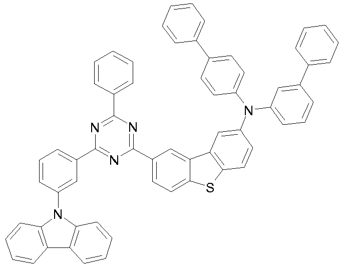 | 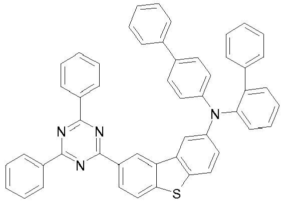 |
| 65 | 66 |
| 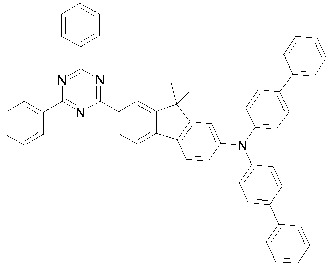 | 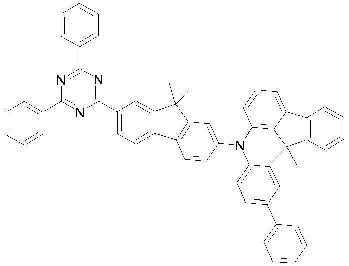 |
| 67 | 68 |
| 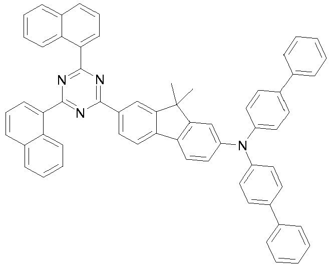 | 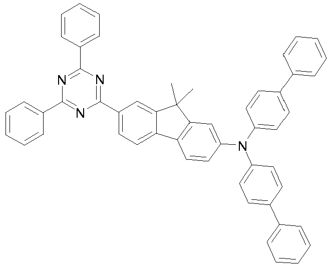 |
| 69 | 70 |
| 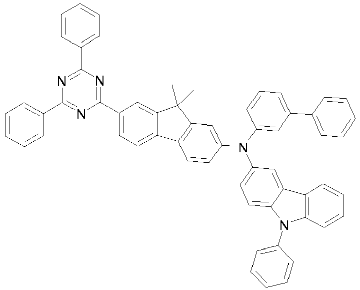 | 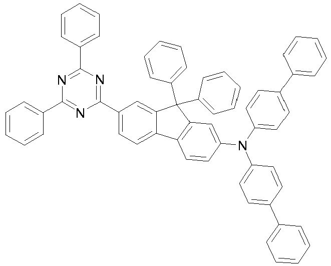 |
| 71 | 72 |
| 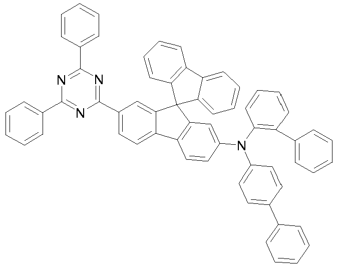 | 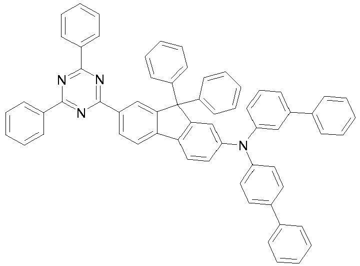 |
| 73 | 74 |
| 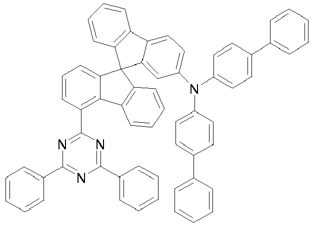 | 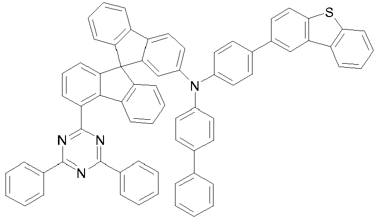 |
| 75 | 76 |
| 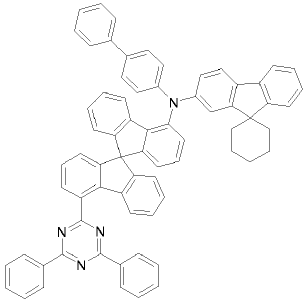 | 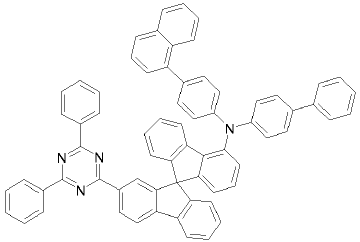 |
| 77 | 78 |
| 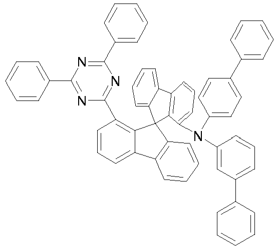 | 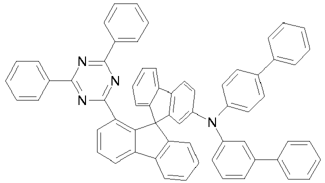 |
| 79 | 80 |
| 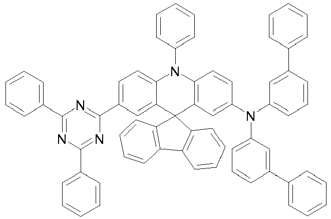 | 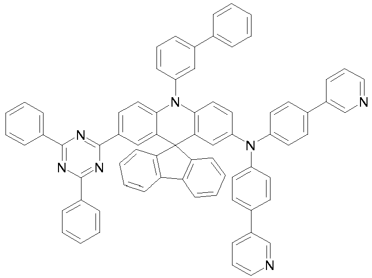 |
| 81 | 82 |
| 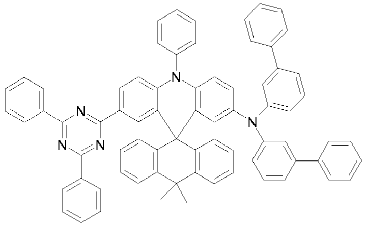 | 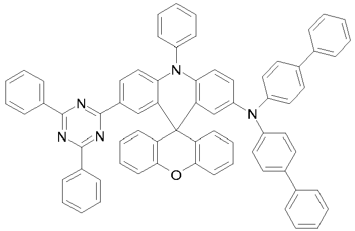 |
| 83 | 84 |
| 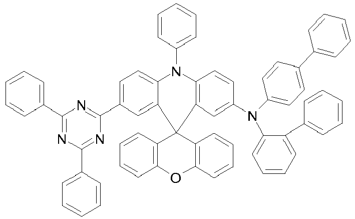 | 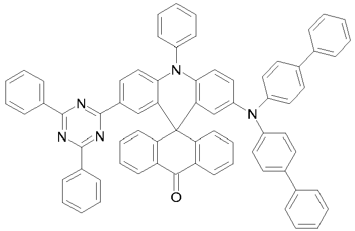 |
| 85 | 86 |
| 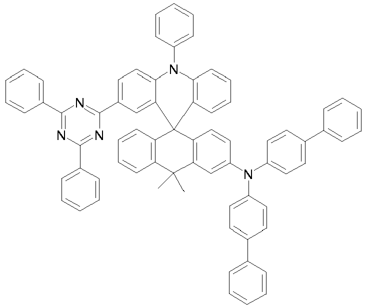 | 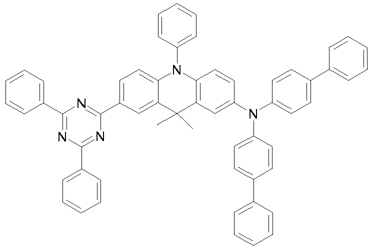 |
| 87 | 88 |
| 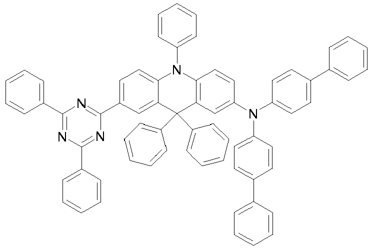 | 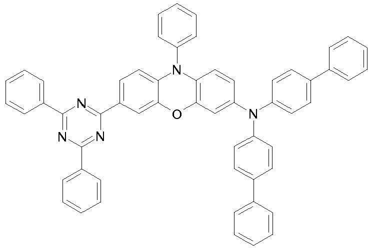 |
| 89 | 90 |
| 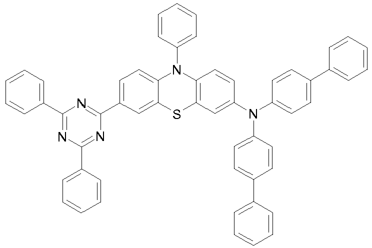 | 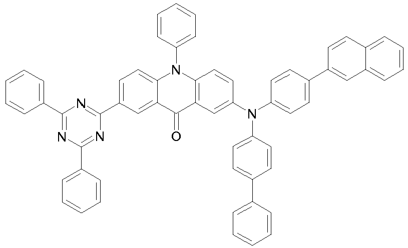 |
| 91 | 92 |
| 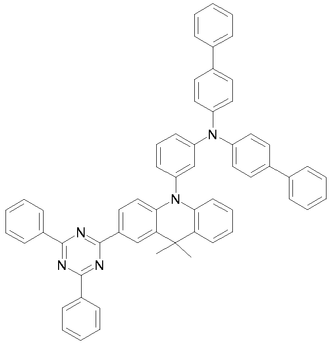 | 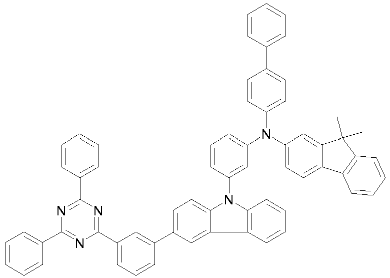 |
| 93 | 94 |
| 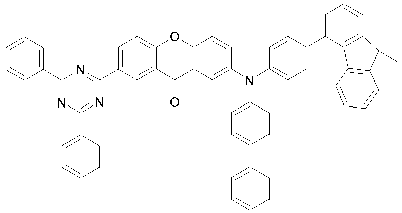 | 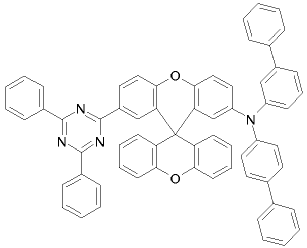 |
| 95 | 96 |
| 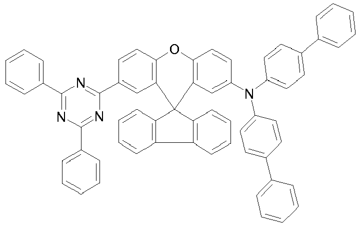 | 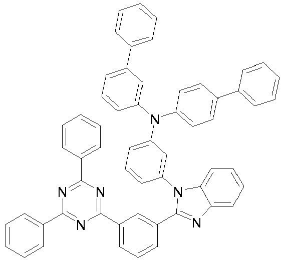 |
| 97 | 98 |
| 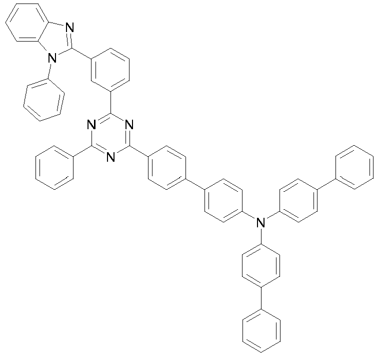 | 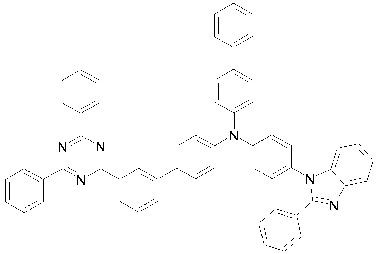 |
| 99 | 100 |
| 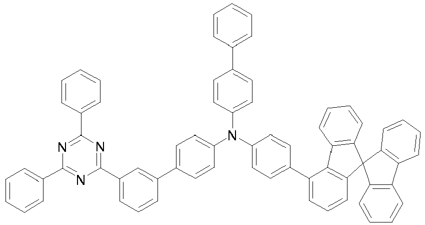 | 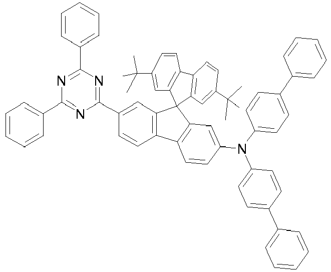 |
| 101 | 102 |
| 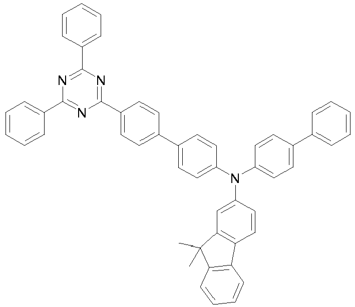 | 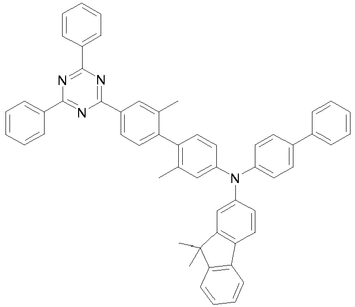 |
| 103 | 104 |
| 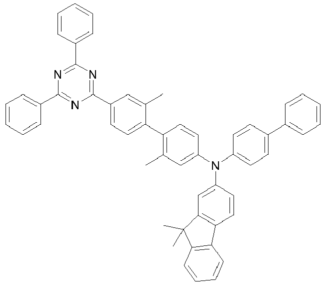 | 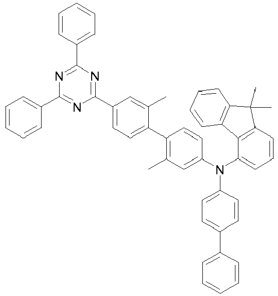 |
| 105 | 106 |
| 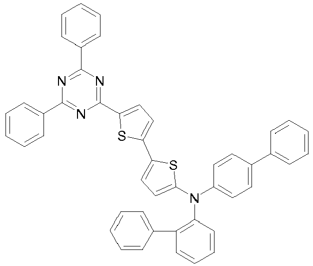 | 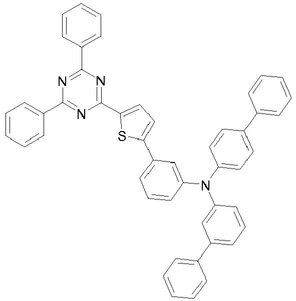 |
| 107 | 108 |
| 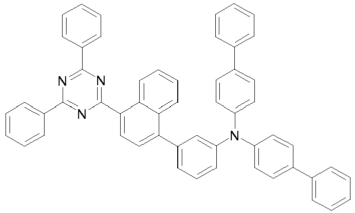 | 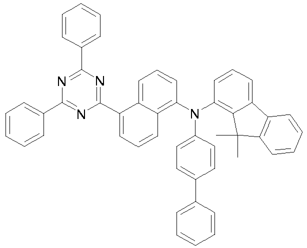 |
| 109 | 110 |
| 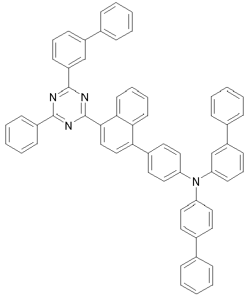 | 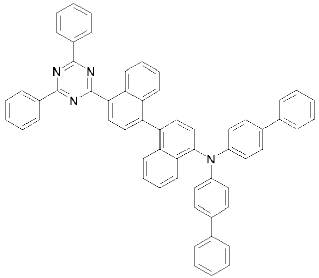 |
| 111 | 112 |
| 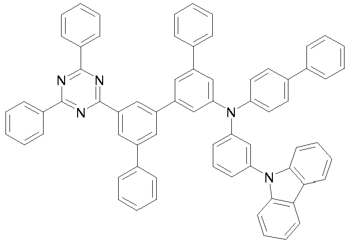 | 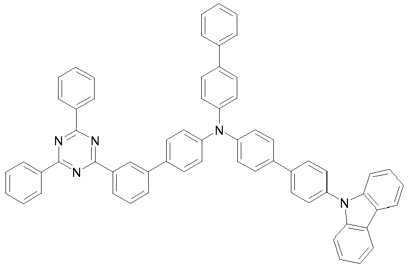 |
| 113 | 114 |
| 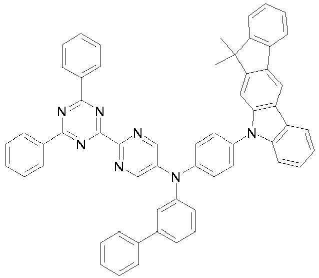 | 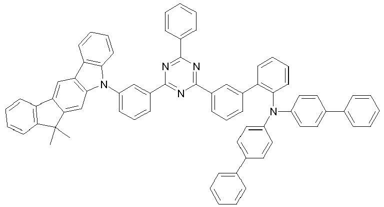 |
| 115 | 116 |
| 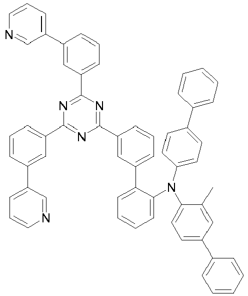 | 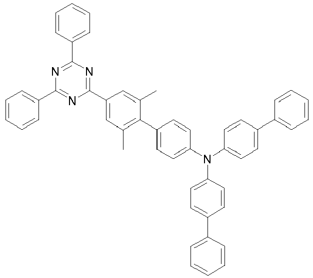 |
| 117 | 118 |
| 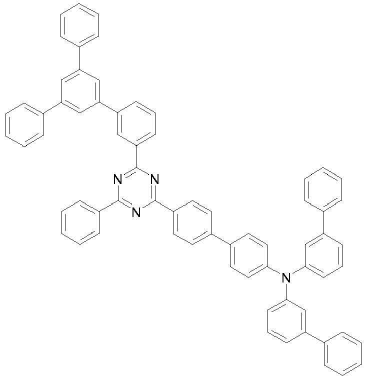 | 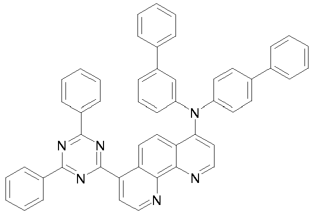 |
| 119 | 120 |
| 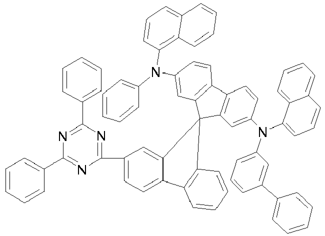 | 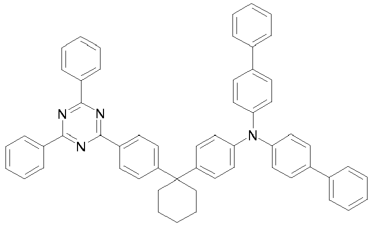 |
| 121* | 122 |
| 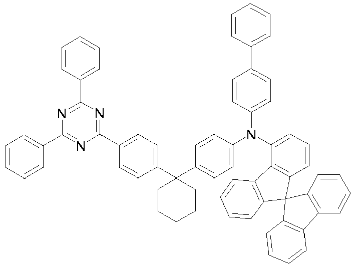 | 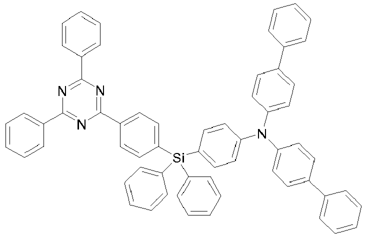 |
| 123 | 124 |
| 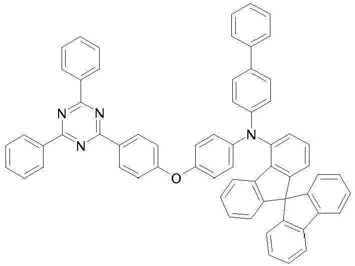 | 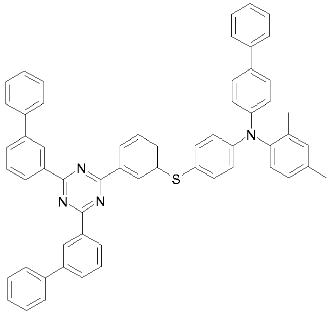 |
| 125 | 126 |
| 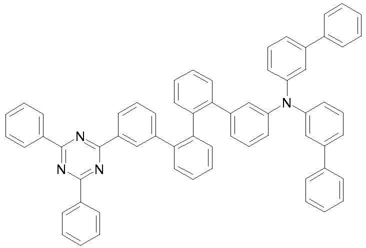 | 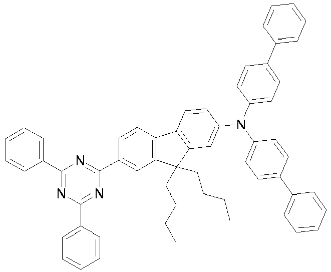 |
| 127 | 128 |
| 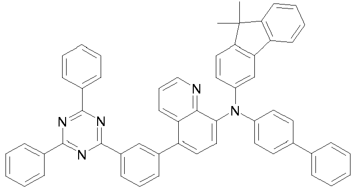 | 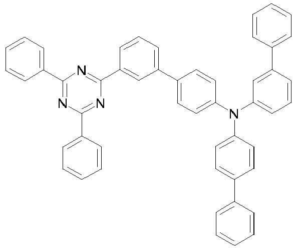 |
| 129 | 130 |
| 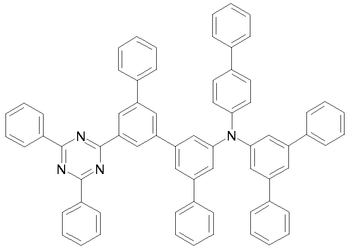 | 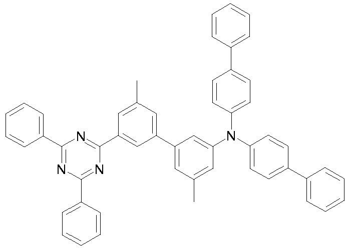 |
| 131 | 132 |
| 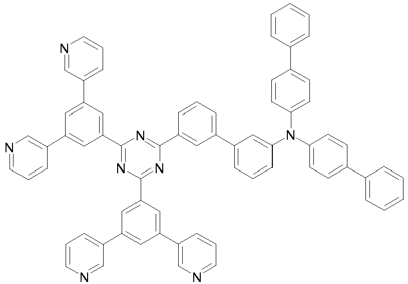 | 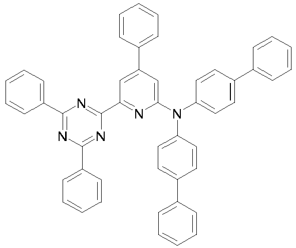 |
| 133 | 134 |
| 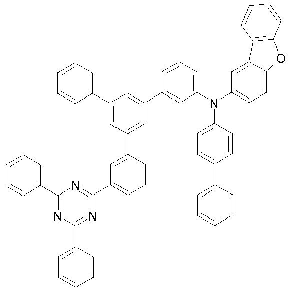 | 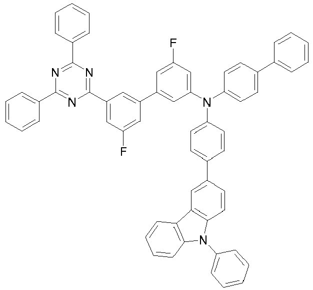 |
| 135 | 136 |
| 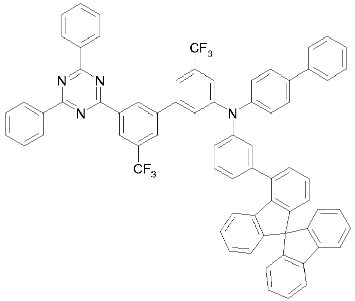 | 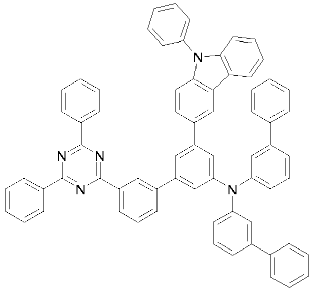 |
| 137 | 138 |
| 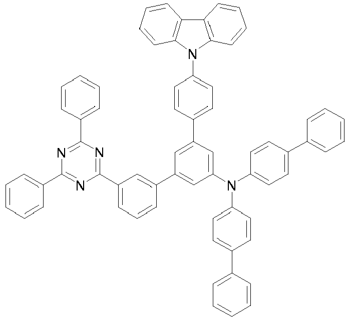 | 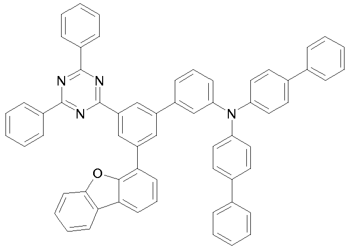 |
| 139 | 140 |
| 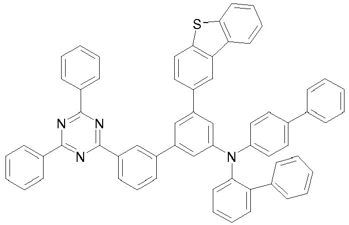 | 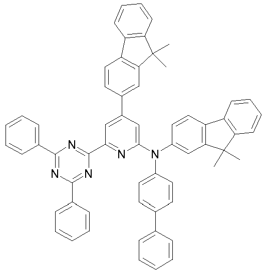 |
| 141 | 142 |
| 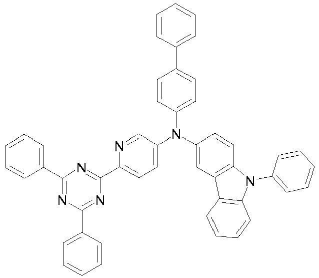 | 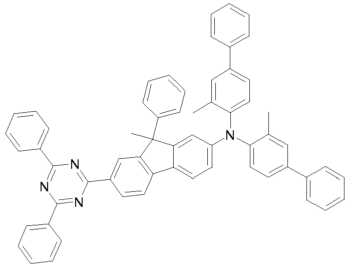 |
| 143 | 144 |
| 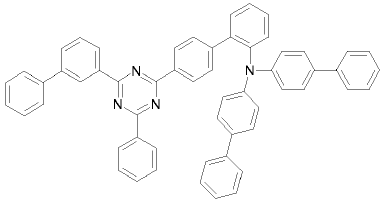 | 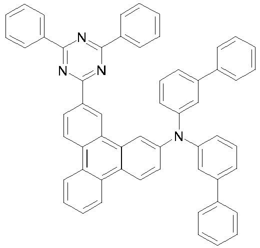 |
| 145 | 146 |
| 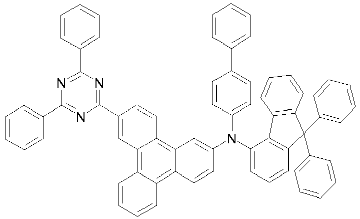 | 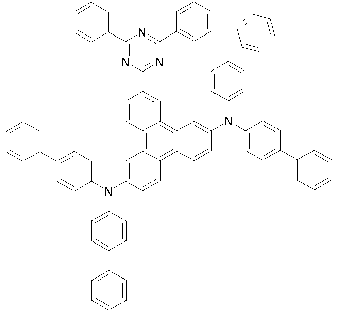 |
| 147 | 148* |
| 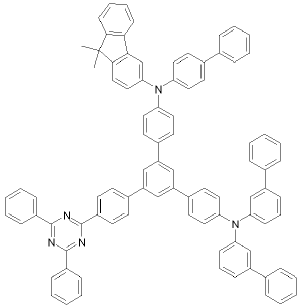 | |
| 149* | |

| | |
|---|---|
| ***Nicht erfindungsgemäß** | |

Die Verbindungen der Formel (I) können mittels bekannter organischchemischer Reaktionen erhalten werden, beispielsweise durch Suzuki-Kupplung, Buchwald-Kupplung, Ullmann-Kupplung, Bromierung und Boronierung.

Ein bevorzugtes Verfahren zur Herstellung der Verbindungen der Formel (I) ist im Folgenden dargestellt (Schema 1). Es wird nur in groben Zügen erläutert. Detaillierte Synthesevorschriften zur Herstellung von Verbindungen der Formel (I), die auf diesem Verfahren basieren, sind in den Ausführungsbeispielen angegeben.

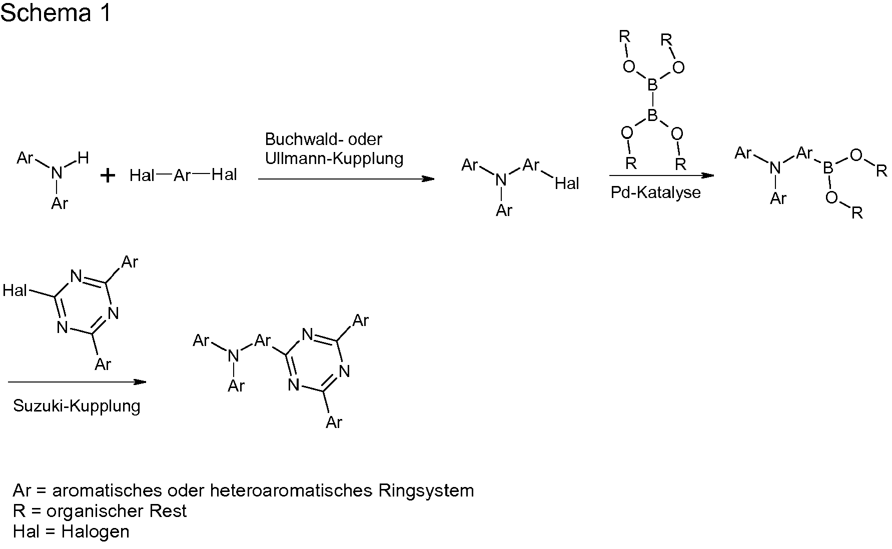

Dazu wird ein Diarylaminoderivat zunächst in einer metallorganischen Kupplungsreaktion, beispielsweise einer Buchwald- oder Ullmann-Kupplung, mit einem Aryl-Halogen-Derivat umgesetzt. Anschließend erfolgt eine Boronierungsreaktion. Das erhaltene Borsäure-Derivat wird dann in einer metallorganischen Kupplungsreaktion, bevorzugt einer Suzuki-Kupplung, mit einem Triazin-Derivat zur Reaktion gebracht. Die dabei erhaltene Verbindung kann gegebenfalls weiter umgesetzt werden, beispielsweise in einer Funktionalisierungsreaktion.

Nach einem alternativen Verfahren (Schema 2) wird ein Triazin-Arylenderivat entweder durch Kondensationsreaktion hergestellt oder ausgehend von Halogen-substituiertem Triazin und einer Arylboronsäure durch Suzuki-Kupplung. In einem weiteren Schritt wird dann die Arylaminogruppe durch Buchwald-Kupplung eingeführt.

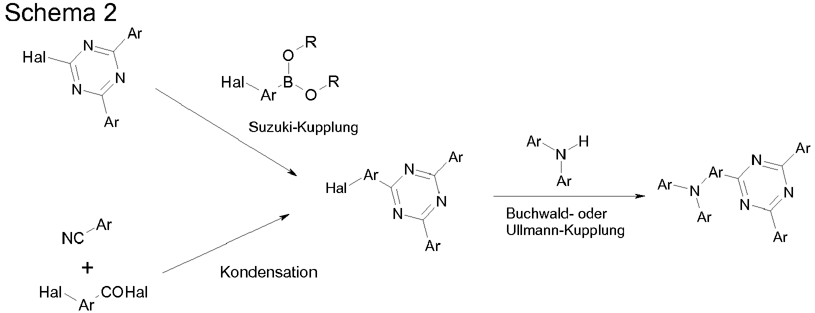

Die angegebenen Verfahren stellen beispielhafte, in vielen Fällen besonders geeignete Verfahren zur Herstellung von Verbindungen der Formel (I) dar. Der Fachmann kann sie unverändert verwenden, oder er kann sie im Rahmen seines allgemeinen Fachwissens anpassen oder durch geeignetere Verfahren ersetzen, wenn die Umstände des konkreten Falls es erfordern.

Gegenstand der Offenbarung ist somit ein Verfahren zur Herstellung einer Verbindung der Formel (I), dadurch gekennzeichnet, dass mindestens eine metallorganische Kupplungsreaktion verwendet wird, bevorzugt mindestens eine Buchwald-Kupplungsreaktion.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Offenbarung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Offenbarung wird eine Verbindung verstanden, welche aus mindestens drei Monomereinheiten aufgebaut ist. Unter einem Polymer im Sinne der Offenbarung wird eine Verbindung verstanden, die aus mindestens zehn Monomereinheiten aufgebaut ist. Die Polymere, Oligomere oder Dendrimere können konjugiert, teilkonjugiert oder nichtkonjugiert sein. Die Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein. Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die Polymere, Oligomere und Dendrimere weisen vorteilhafte Eigenschaften, insbesondere hohe Lebensdauern, hohe Effizienzen und gute Farbkoordinaten auf.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in organischen Elektrolumineszenzvorrichtungen (OLEDs). Die Verbindungen können, unter anderem abhängig von der Substitution, in unterschiedlichen Funktionen und in unterschiedlichen Schichten eingesetzt werden. Bevorzugt werden die Verbindungen als Matrixmaterialien, bevorzugt als Matrixmaterialien für phosphoreszierende Emitter, eingesetzt.

Ein weiterer Gegenstand der Erfindung ist die Verwendung der Verbindungen gemäß Formel (I) in organischen Elektrolumineszenzvorrichtungen (OLEDs).

Gegenstand der Erfindung ist weiterhin eine organische Elektrolumineszenzvorrichtung, enthaltend Anode, Kathode sowie mindestens eine organische Schicht, wobei die organische Schicht mindestens eine Verbindung der Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer), Auskopplungsschichten und/oder organischen oder anorganischen p/n-Übergängen. Die in den jeweiligen Schichten und Funktionen bevorzugt eingesetzten Verbindungen werden in späteren Abschnitten explizit offenbart.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Alternativ und/oder zusätzlich können die erfindungsgemäßen Verbindungen auch in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

In einer bevorzugten Ausführungsform der vorliegenden Erfindung werden die Verbindungen der Formel (I) als Matrixmaterial in einer emittierenden Schicht in Kombination mit einer oder mehreren Emitterverbindungen, vorzugsweise phosphoreszierenden Emitterverbindungen, eingesetzt.

Vom Begriff phosphoreszierende Emitter sind gemäß der vorliegenden Anmeldung Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, wie einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Offenbarung alle lumineszierenden Iridium-, Platin- oder Kupferkomplexe als phosphoreszierende Verbindungen angesehen.

Beispiele für phosphoreszierende Emitterverbindungen können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen. Weitere Beispiele für geeignete phosphoreszierende Emitter können der in einem späteren Abschnitt folgenden Tabelle entnommen werden.

Der Anteil des Matrixmaterials in der emittierenden Schicht beträgt in der erfindungsgemäßen Vorrichtung bevorzugt zwischen 50.0 und 99.9 %, besonders bevorzugt zwischen 80.0 und 99.5 % und ganz besonders bevorzugt für fluoreszierende emittierende Schichten zwischen 92.0 und 99.5 % sowie für phosphoreszierende emittierende Schichten zwischen 85.0 und 97.0 %. Entsprechend beträgt der Anteil der emittierenden Verbindung bevorzugt zwischen 0.1 und 50.0 %, besonders bevorzugt zwischen 0.5 und 20.0 % und besonders ganz bevorzugt für fluoreszierende emittierende Schichten zwischen 0.5 und 8.0 % sowie für phosphoreszierende emittierende Schichten zwischen 3.0 und 15.0 %.

Vorliegend wird unter der Angabe der relativen Anteile verschiedener Verbindungen in einer Schicht in % bei Herstellung der Vorrichtung aus Lösung Gew.-% verstanden, während bei Herstellung durch einen Gasphasenprozess darunter Vol.-% verstanden wird.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit unter anderem lochtransportierenden Eigenschaften und das andere Material ein Material mit unter anderem elektronentransportierenden Eigenschaften dar. Die Verbindung der Formel (I) stellt dabei ein Matrixmaterial mit elektronentransportierenden und lochtransportierenden Eigenschaften dar. Die beiden unterschiedlichen Matrixmaterialien können in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen. Die eine oder die mehreren emittierenden Verbindungen zusammen haben erfindungsgemäß einen Anteil von 0.1 bis 50.0 % an der Gesamtmischung und bevorzugt einen Anteil von 0.5 bis 20.0 % an der Gesamtmischung. Entsprechend haben die Matrixkomponenten zusammen einen Anteil von 50.0 bis 99.9 % an der Gesamtmischung und bevorzugt einen Anteil von 80.0 bis 99.5 % an der Gesamtmischung.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den angegebenen bevorzugten Matrixmaterialien für phosphoreszierende Emitter oder den bevorzugten Matrixmaterialien für fluoreszierende Emitter, je nachdem welche Art von Emitter im Mixed-Matrix-System eingesetzt wird.

Im Folgenden werden die in der erfindungsgemäßen organischen Elektrolumineszenzvorrichtung bevorzugt eingesetzten weiteren Funktionsmaterialien aufgeführt.

Die in der folgenden Tabelle aufgeführten Verbindungen stellen besonders geeignete phosphoreszierende Emitter dar.

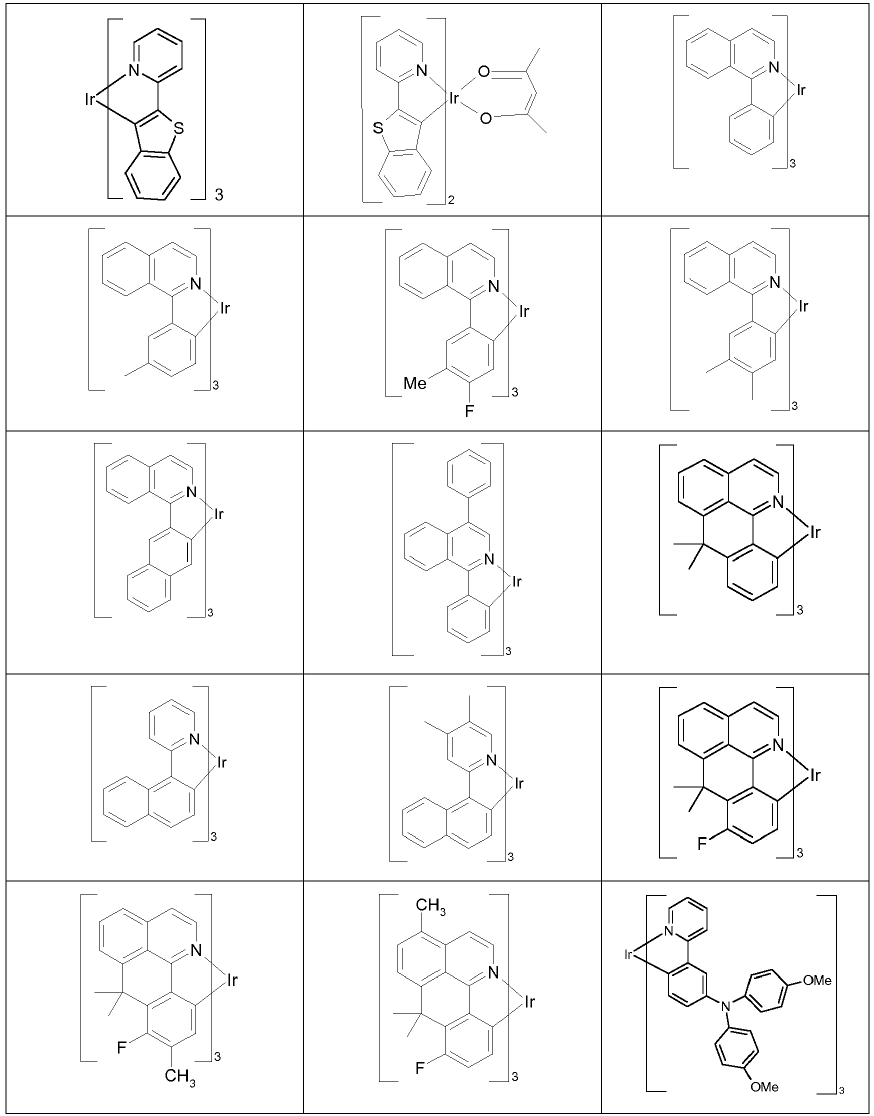

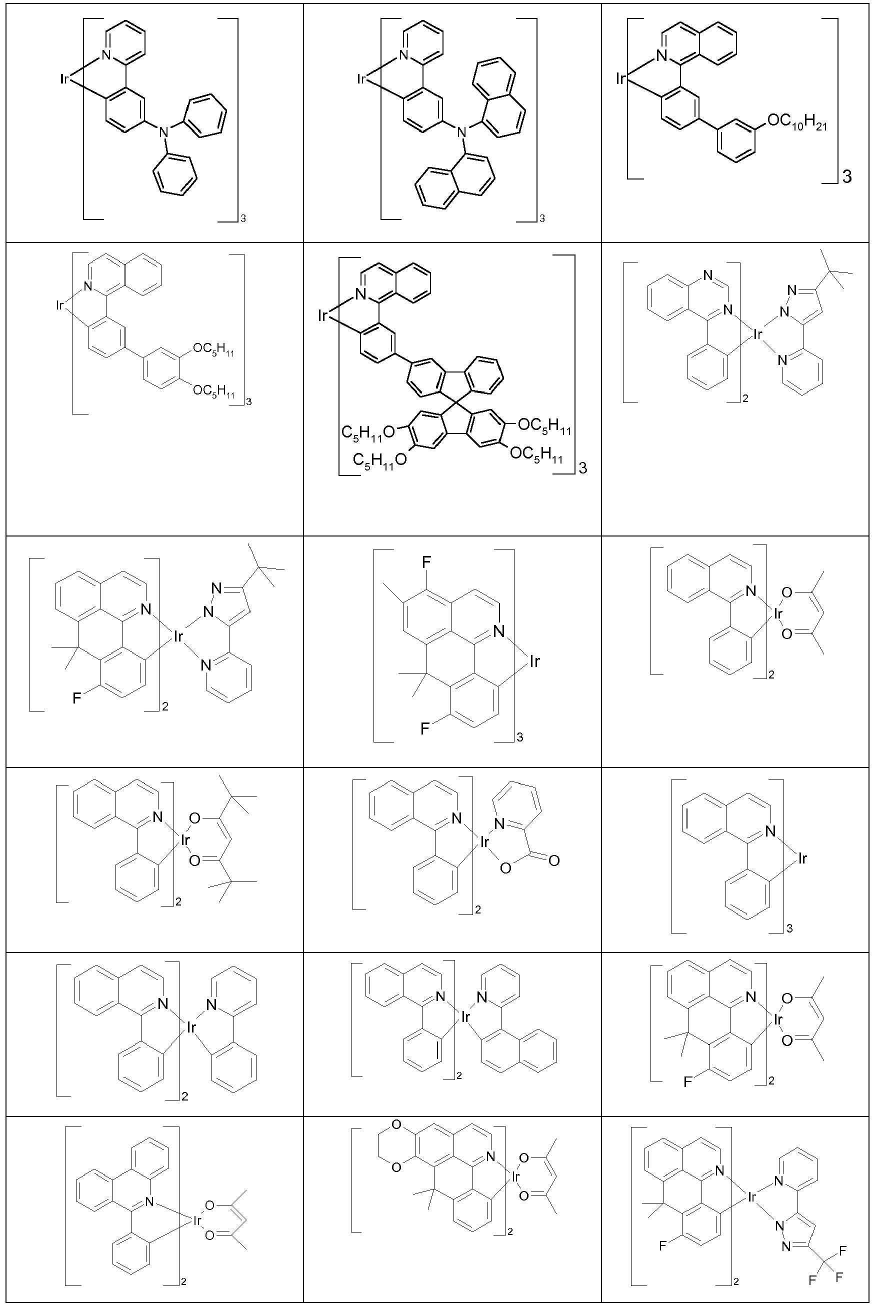

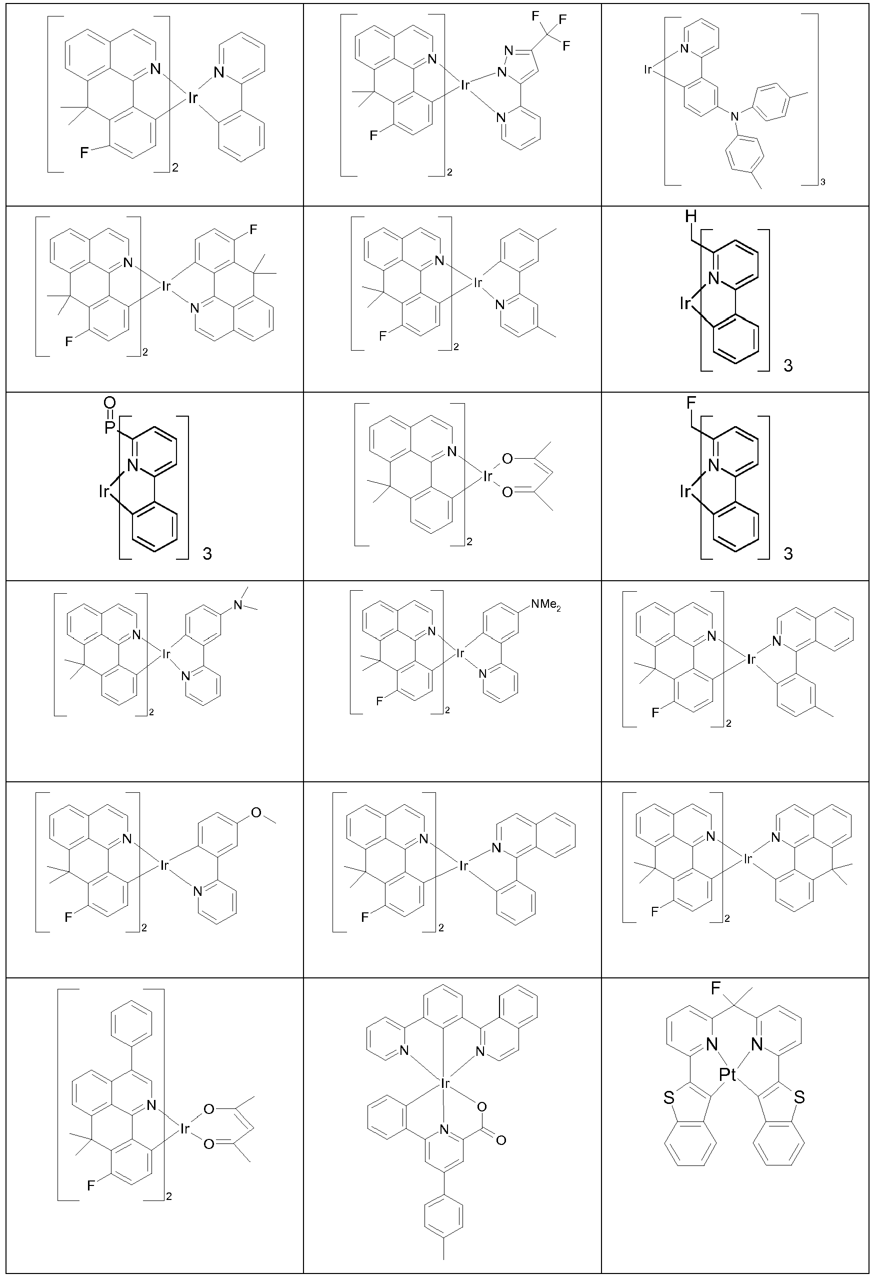

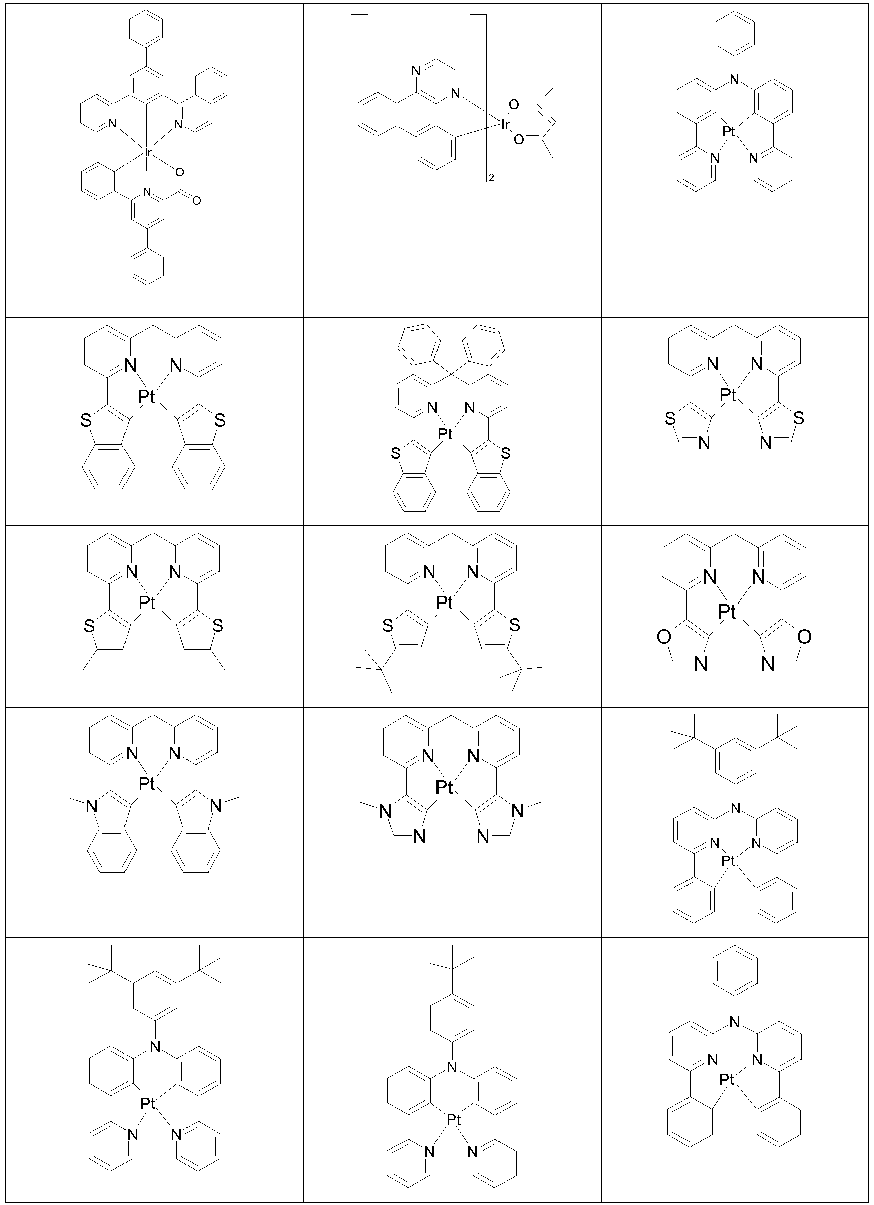

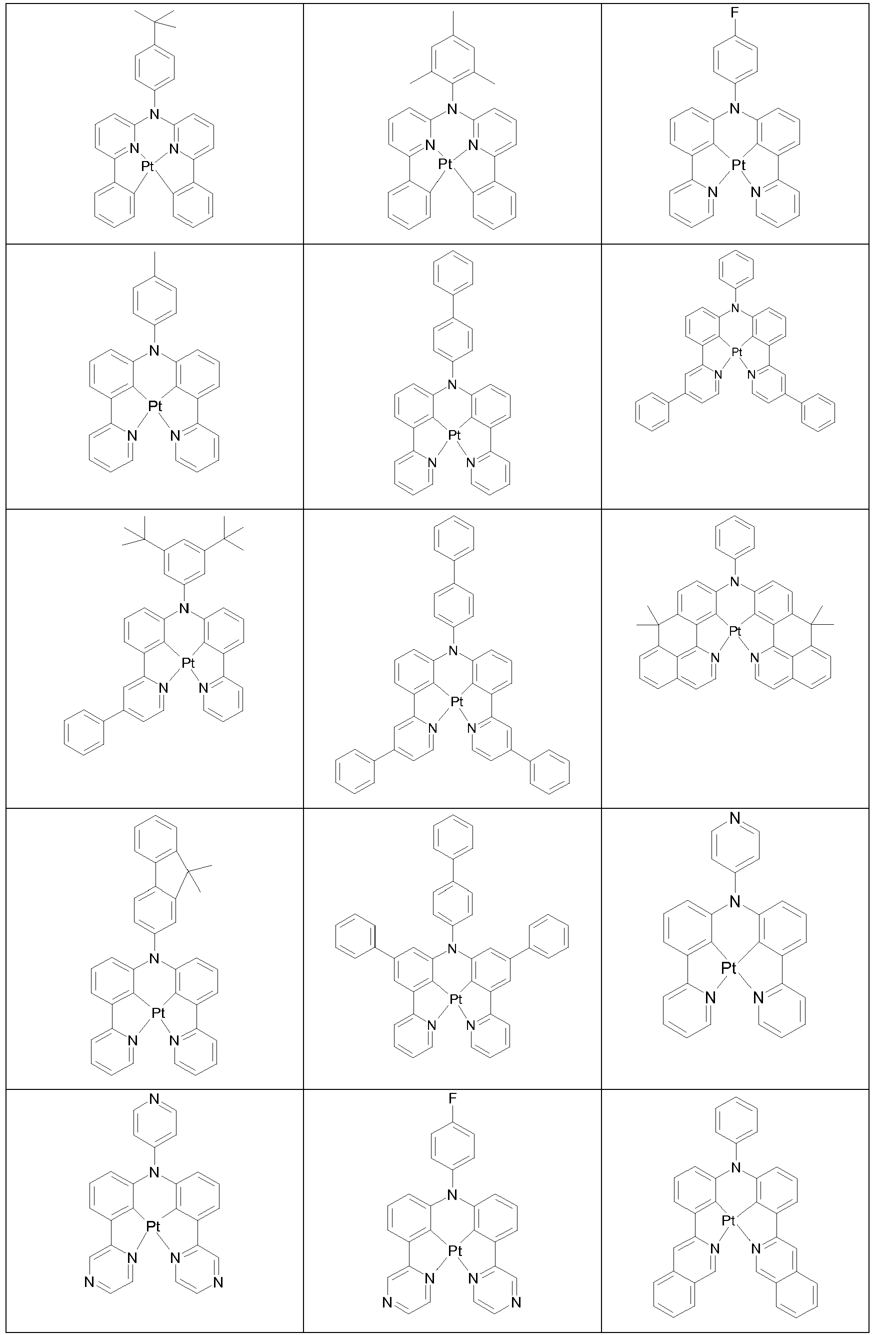

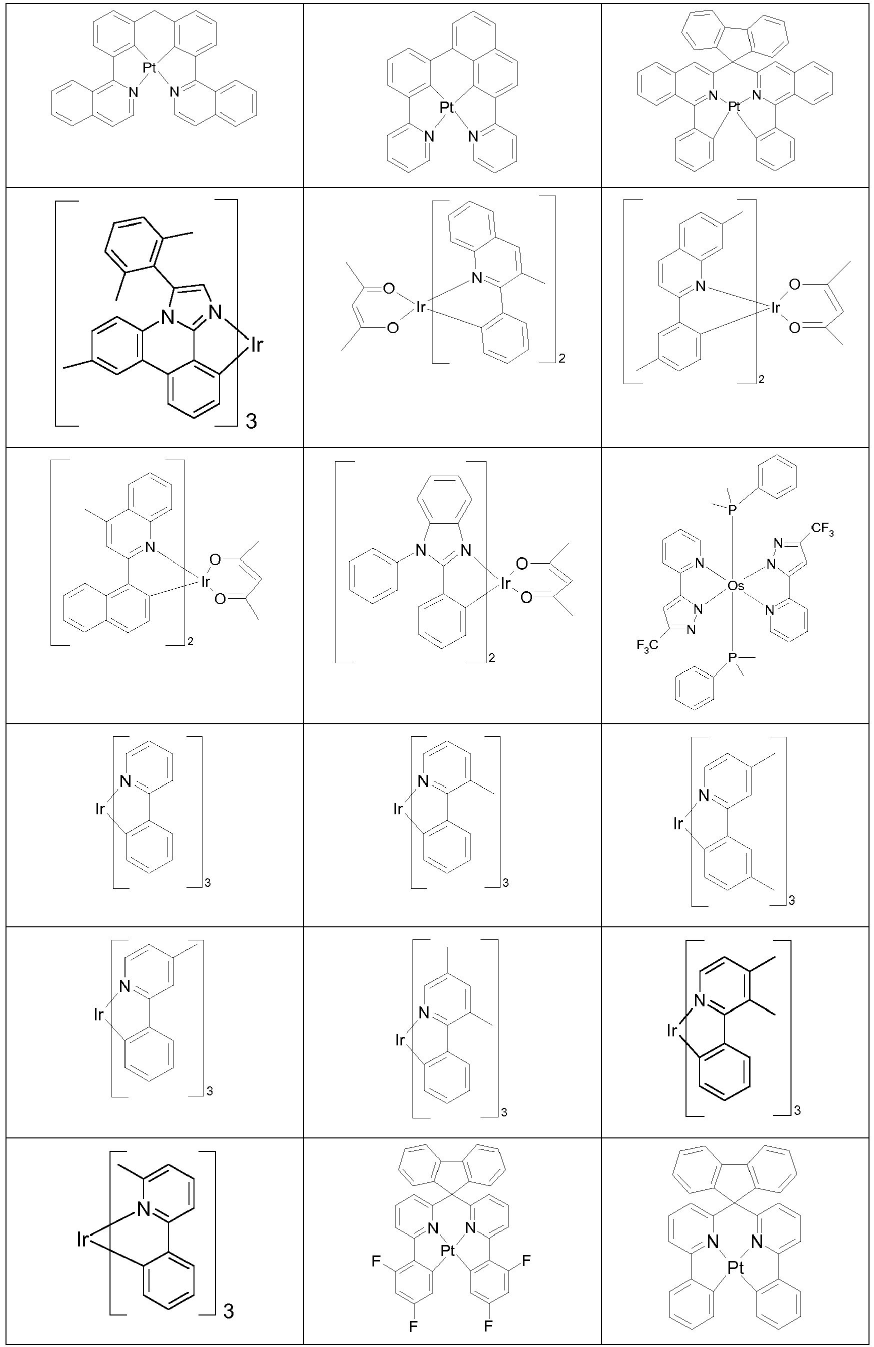

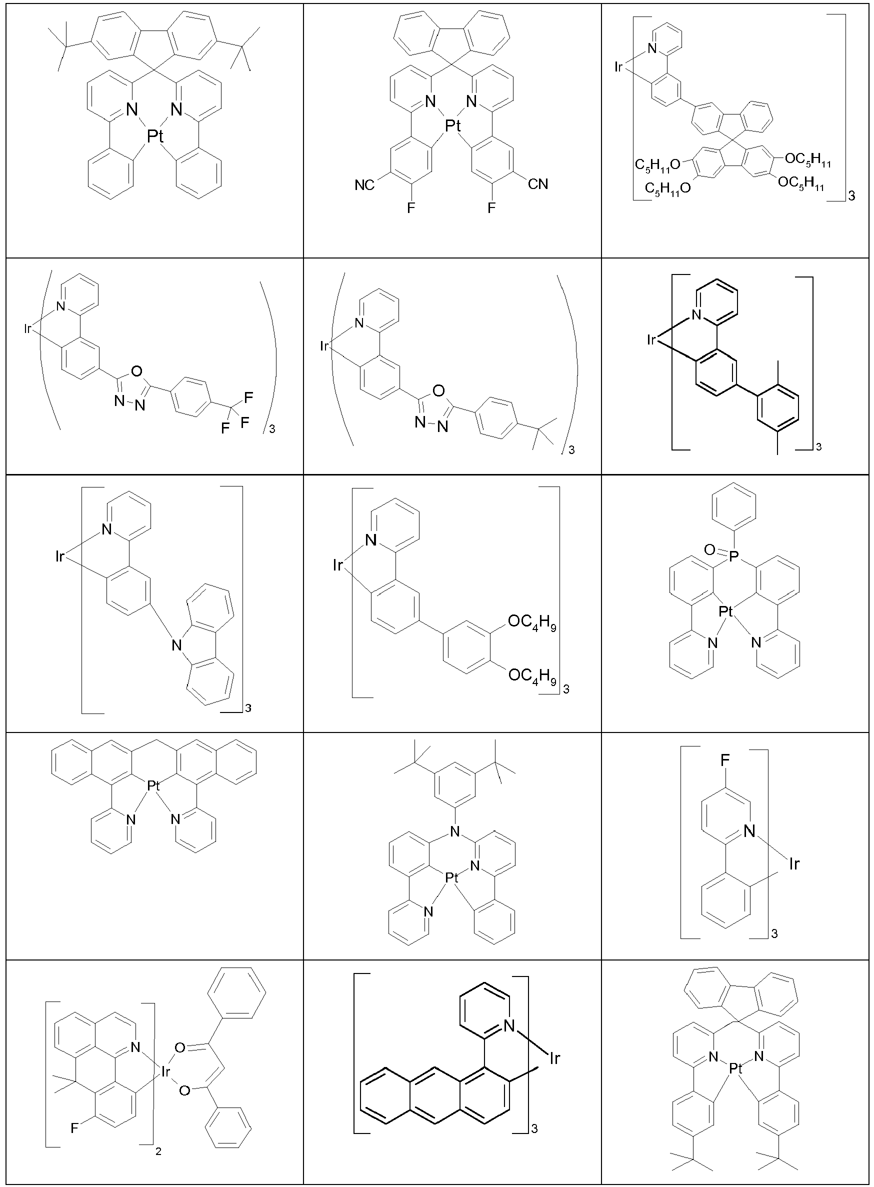

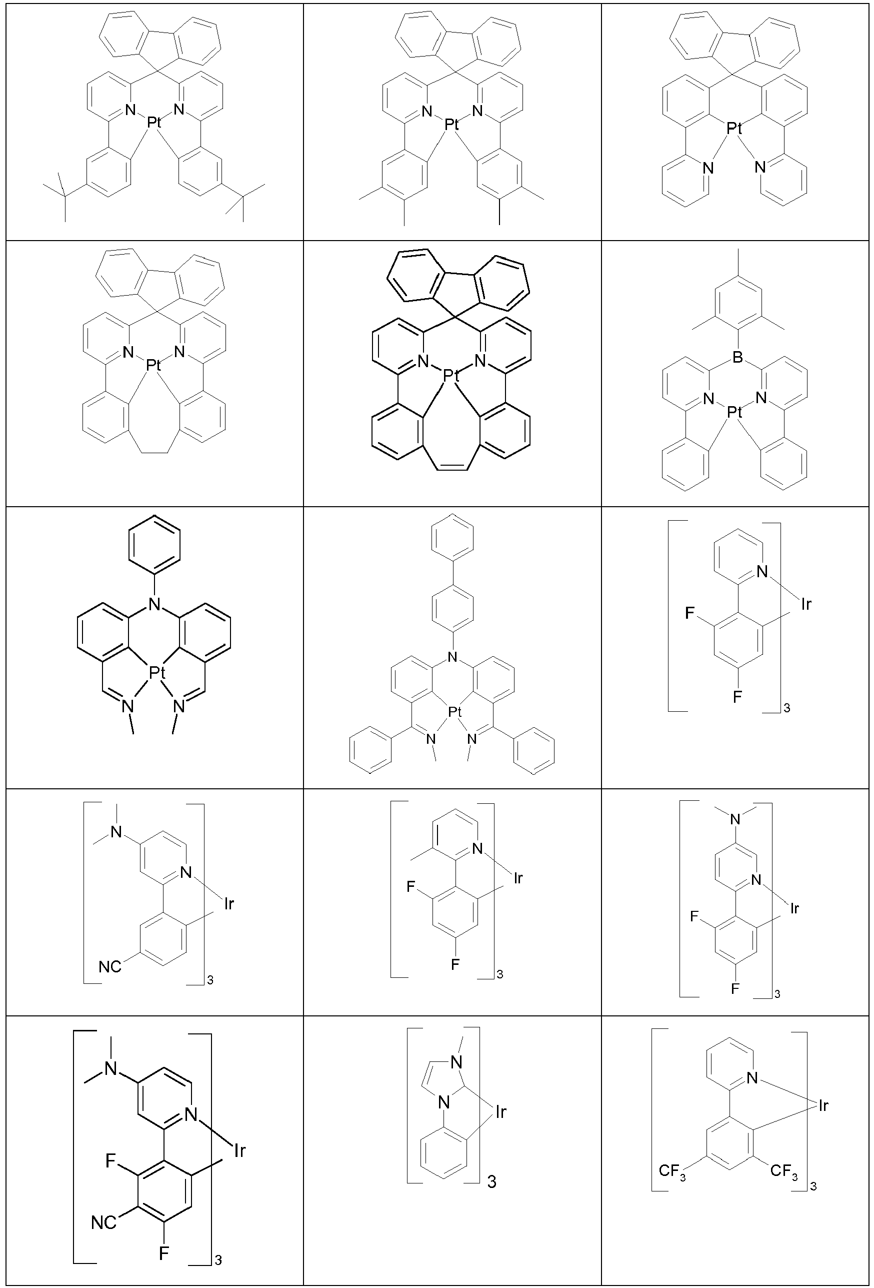

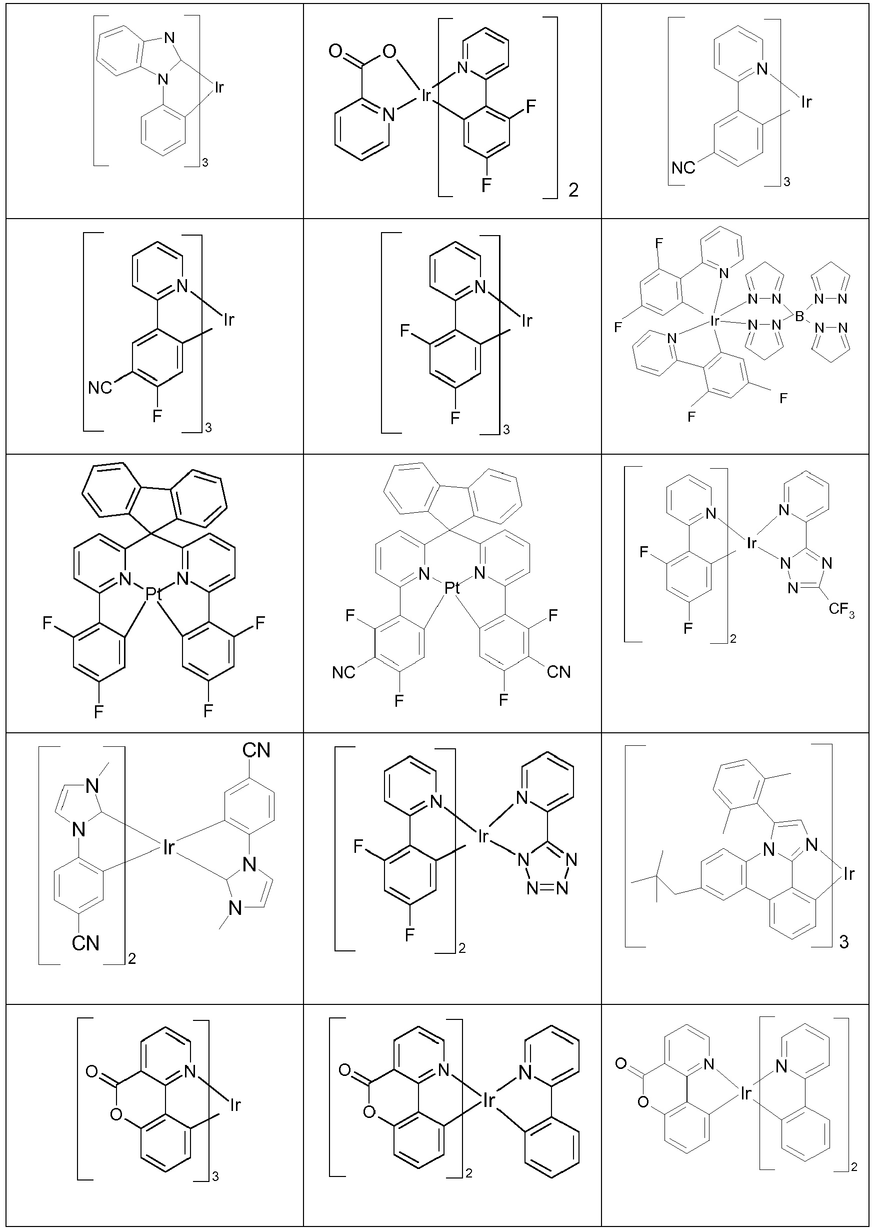

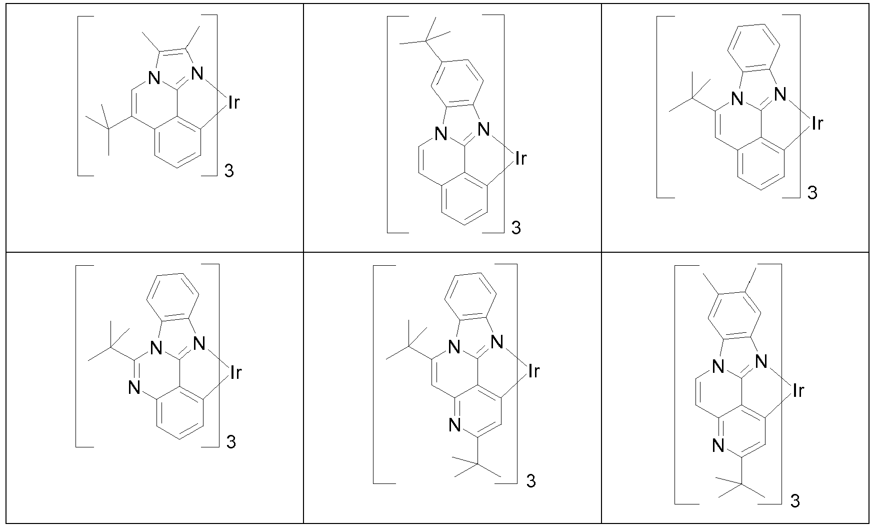

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 offenbarten Pyren-Arylamine.

Als Matrixmaterialien, bevorzugt für fluoreszierende Emitter, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Offenbarung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende Emitter sind neben den erfindungsgemäßen Verbindungen aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Es ist allgemein bevorzugt, dass eine oder mehrere der Lochtransportschichten p-dotiert ist und/oder dass eine oder mehrere der Elektronentransportschichten n-dotiert ist. Hierfür geeignete p-Dotanden bzw. n-Dotanden sind beispielsweise in Chem. Rev. 2007, 107, 1233-1271 beschrieben.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

### Ausführungsbeispiele

### A) Synthesebeispiele

### Schritt 1: Synthese des Amin-Bausteins:

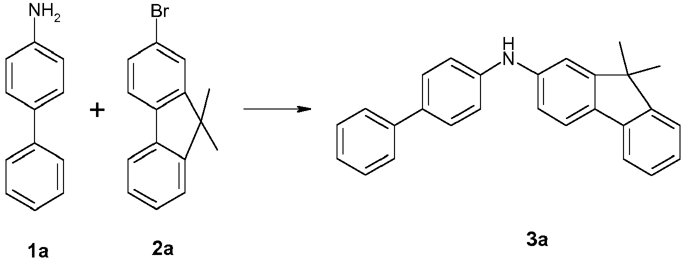

24.0g (142mmol, 1.2eq.) 4-Aminobiphenyl **1a** (CAS 92-67-1) werden zusammen mit 32.0g (117mmol, 1.0eq) 2-Bromo-9,9'-dimethylfluoren **2a** (CAS 28320-31-2) in 950 ml Toluol vorgelegt und 30 Minuten mit Argon gesättigt. Anschließend werden 1.0g (1.8mmol, 0.02eq) 1,1'-Bis(diphenylphosphino)ferrocen (CAS 12150-46-8), 350mg (1.6mmol, 0.01eq) Palladium(II)-acetat (CAS 3375-31-3) und 29g (300mmol, 2.6eq) Natrium-*tert*-butylat (CAS 865-48-5) zugegeben und über Nacht unter Rückfluss erhitzt. Nach beendeter Reaktion wird der Ansatz mit 300ml Toluol verdünnt und mit Wasser extrahiert. Die organische Phase wird über Natriumsulfat getrocknet und das Lösungsmittel am Rotationsverdampfer entfernt. Das braune Öl wird mit 50ml Ethylacetat versetzt und in eine Mischung aus Heptan/Essigester 20:1 gegeben. Der entstandene Feststoff wird abgesaugt und mit Heptan gewaschen. Nach Trocknung werden 29g (80mmol, 69%) des gewünschten Produkts **3a** mit einer HPLC-Reinheit von 99.1% erhalten.

Analog können folgende Verbindungen erhalten werden:

| **Nr.** | **Edukt 1** | **Edukt 2** | **Produkt** | **Ausbeute** |
|---|---|---|---|---|
| **3b** | 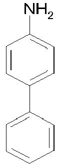 | 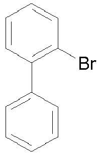 | 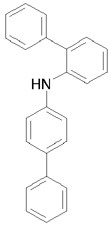 | 71% |
| | 92-67-1 | 2052-07-5 | | |
| **3c** | 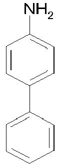 | 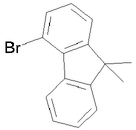 | 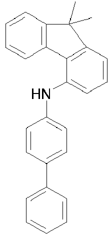 | 61% |
| | 92-67-1 | 942615-32-9 | | |
| **3d** | 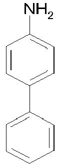 | 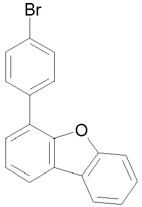 | 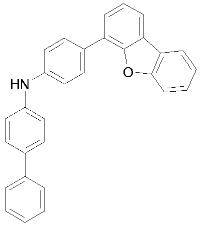 | 78% |
| | 92-67-1 | 955959-84-9 | | |
| **3e** | 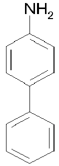 | 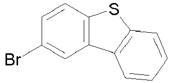 | 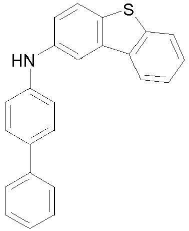 | 82% |
| | 92-67-1 | 22439-61-8 | | |
| **3f** | 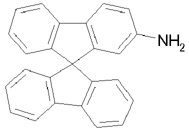 | 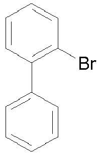 | 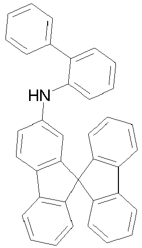 | 62% |
| | 118951-68-1 | 2052-07-5 | | |
| **3g** | 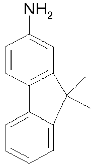 | 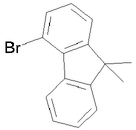 | 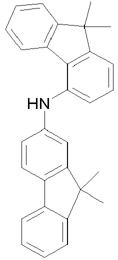 | 47% |
| | 108714-73-4 | 942615-32-9 | | |
| **3h** | 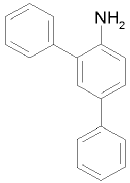 | 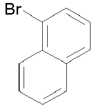 | 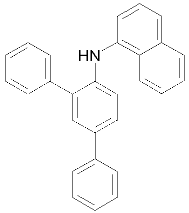 | 92% |
| | 63344-48-9 | 90-11-9 | | |
| **3i** | 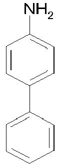 | 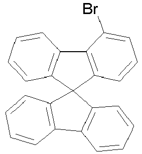 | 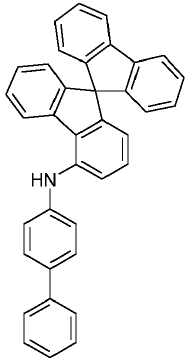 | 75% |
| | 92-67-1 | 171408-76-7 | | |
| **3j** | 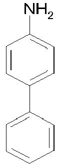 | 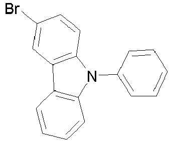 | 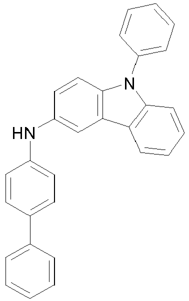 | 84% |
| | 92-67-1 | 1153-85-1 | | |
| **3k** | 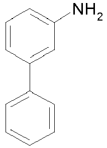 | 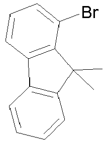 | 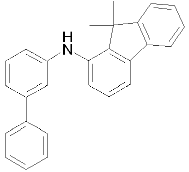 | 62% |
| | 90-41-5 | 1225053-54-2 | | |
| **3l** | 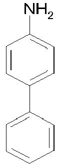 | 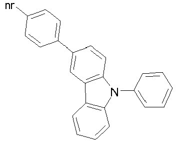 | 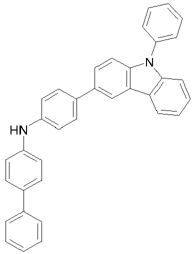 | 78% |
| | 92-67-1 | 1028647-93-9 | | |
| **3m** | 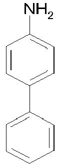 | 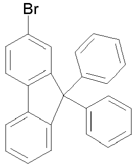 | 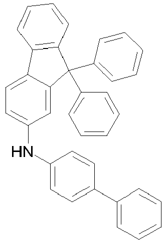 | 74% |
| | 92-67-1 | 474918-32-6 | | |
| **3n** | 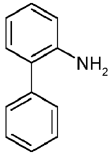 | 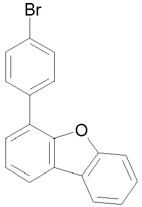 | 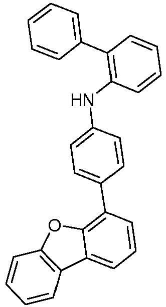 | 62% |
| | 90-41-5 | 955959-84-9 | | |
| **3o** | 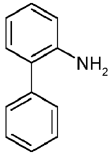 | 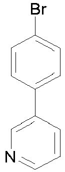 | 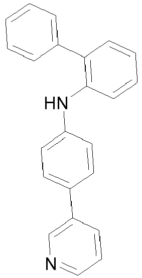 | 67% |
| | 90-41 -5 | 129013-83-8 | | |
| **3p** | 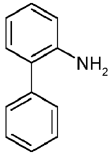 | 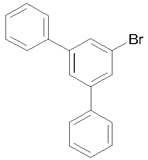 | 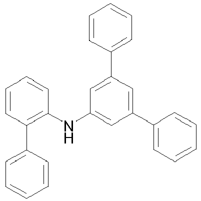 | 93% |
| | 90-41-5 | 103068-20-8 | | |
| **3q** | 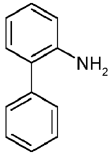 | 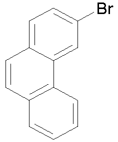 | 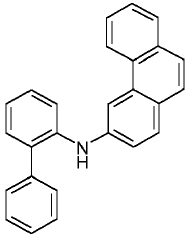 | 88% |
| | 90-41-5 | 715-50-4 | | |
| **3r** | 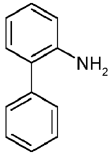 | 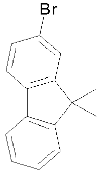 | 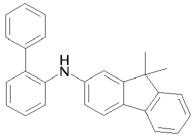 | 74% |
| | 90-41-5 | 28320-31-2 | | |

### Schritt 2: Einführung der Brücke

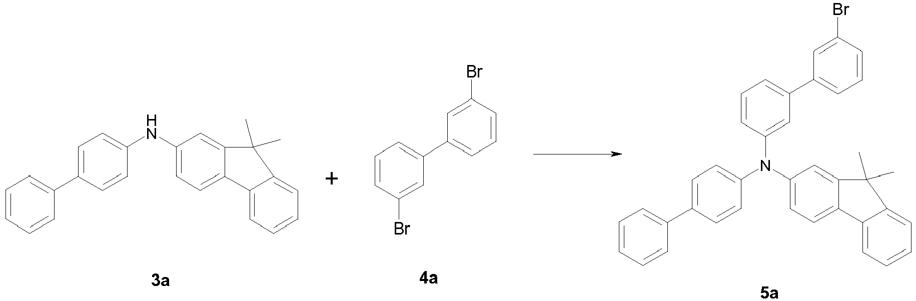

Es werden 29g (80mmol, 1.0 eq) des Zwischenprodukts **3a** zusammen mit 25g (80mmol, 1.0 eq) 3,3'-Dibromo-1,1'-biphenyl **4a** (CAS 16400-51-4) in 600ml Toluol gelöst und für 30 Minuten entgast. Anschließend werden 45g (240mmol, 3.0eq) Natrium-*tert*-butylat, 890mg (0.40mmol, 0.050eq) Palladium(II)-acetat und 8ml (8.0 mmol, 0.10eq.) einer 1M tri-*tert-*Butylphosphin-Lösung zugegeben. Der Ansatz wird über Nacht unter Rückfluss erhitzt und nach beendeter Reaktion zweimal über Aluminiumoxid mit Toluol filtriert. Nach Entfernung des Lösungsmittels am Rotationsverdampfer wird das Öl in etwas THF gelöst und in Heptan eingetragen. Der entstandene Feststoff wird abgesaugt und mittels Heißextraktion in Heptan/Toluol 1:1 aufgereinigt. Es werden 16.6g (28mmol, 35%) des gewünschten Produkts **5a** erhalten.

Analog können folgende Verbindungen erhalten werden:

| **Nr.** | **Edukt 3** | **Edukt 4** | **Produkt** | **Ausbeute** |
|---|---|---|---|---|
| **5b** | 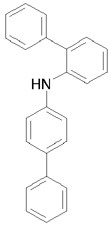 | 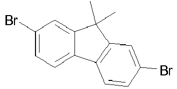 | 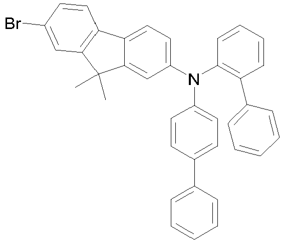 | 49% |
| | | 28320-32-3 | | |
| **5c** | 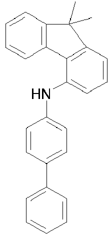 | 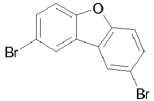 | 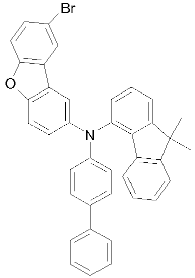 | 37% |
| | | 10016-52-1 | | |
| **5d** | 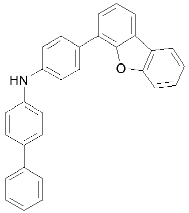 | 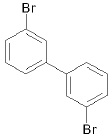 | 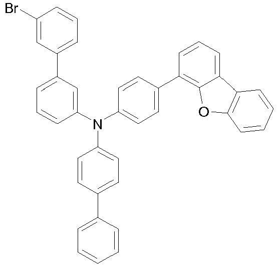 | 72% |
| | | 16400-51-4 | | |
| **5e** | 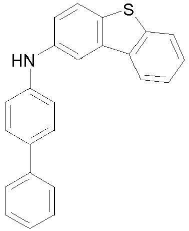 | 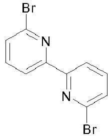 | 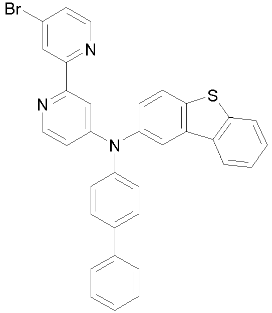 | 28% |
| | | 49669-22-9 | | |
| **5f** | 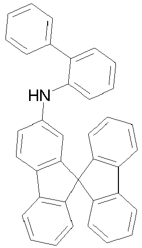 | 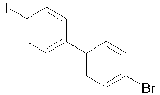 | 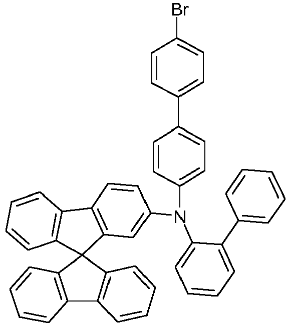 | 82% |
| | | 105946-82-5 | | |
| **5g** | 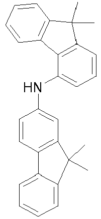 | 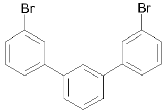 | 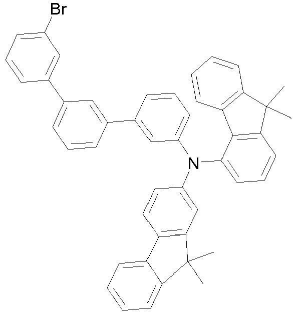 | 32% |
| | | 95962-62-2 | | |
| **5h** | 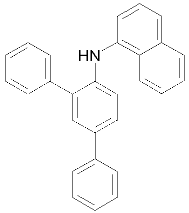 | 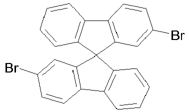 | 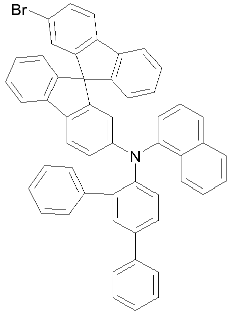 | 46% |
| | | 67665-47-8 | | |
| **5i** | 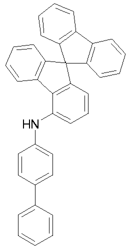 | 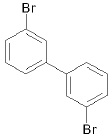 | 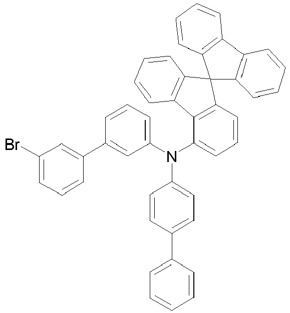 | 41% |
| | | 16400-51-4 | | |
| **5j** | 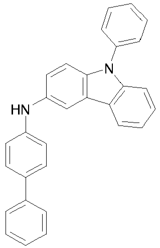 | 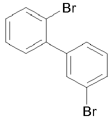 | 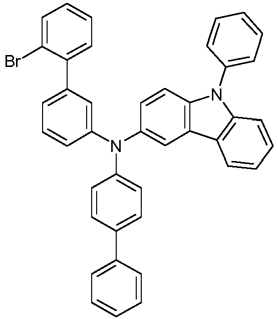 | 31% |
| | | 49602-90-6 | | |
| **5k** | 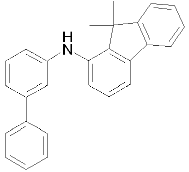 | 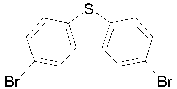 | 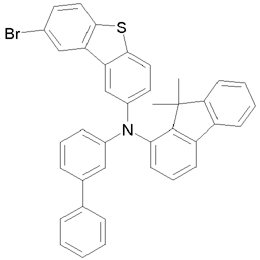 | 27% |
| | | 31574-87-5 | | |
| **5l** | 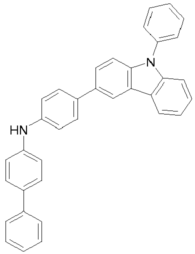 | 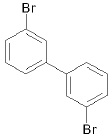 | 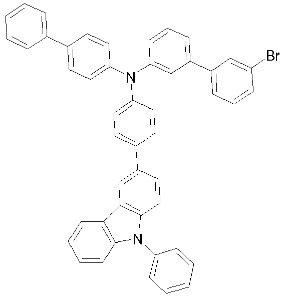 | 38% |
| | | 16400-51-4 | | |
| **5m** | 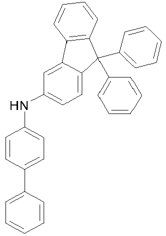 | 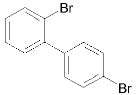 | 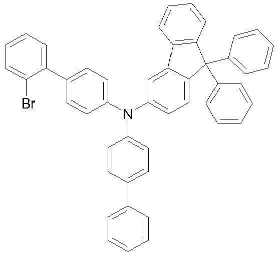 | 56% |
| | | 49602-91-7 | | |
| **5n** | 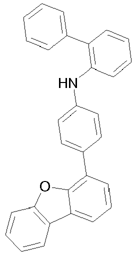 | 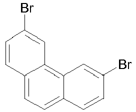 | 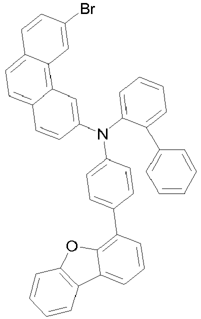 | 33% |
| | | 174735-02-5 | | |
| **5o** | 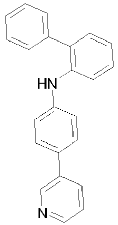 | 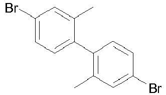 | 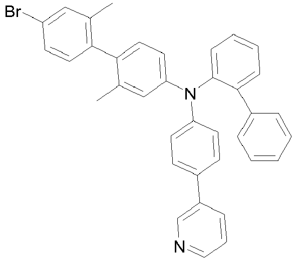 | 47% |
| | | 31458-17-0 | | |
| **5p** | 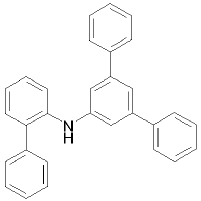 | 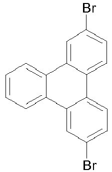 | 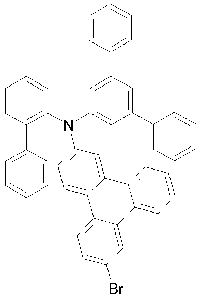 | 24% |
| | | 888041-37-0 | | |
| **5q** | 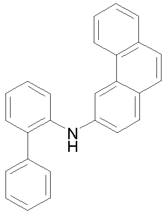 | 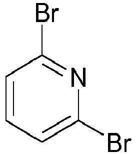 | 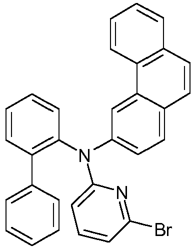 | 81% |
| | | 626-05-1 | | |
| **5r** | 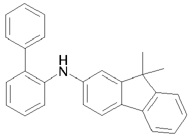 | 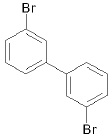 | 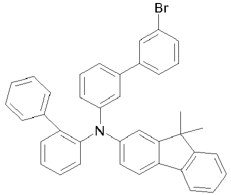 | 57% |
| | | 16400-51-4 | | |
| **5s** | 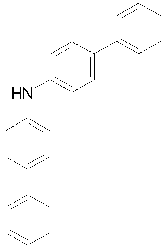 | 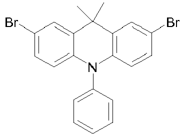 | 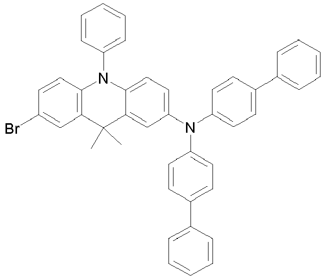 | 29% |
| | | 1333316-36-1 | | |
| **5t** | 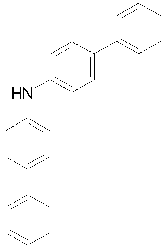 102113-98-4 | 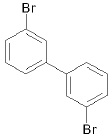 | 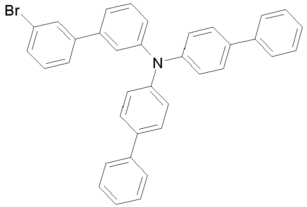 | 36% |
| | | 16400-51-4 | | |
| **5u** | 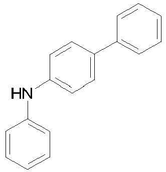 | 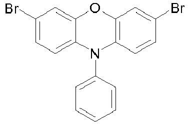 | 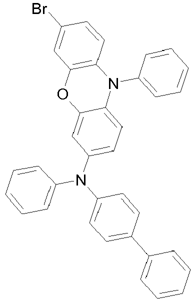 | 44% |
| | 32228-99-2 | 71041-12-8 | | |
| **5v** | 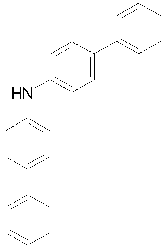 | 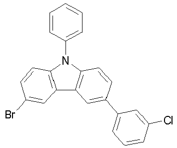 | 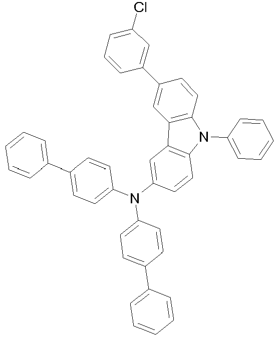 | 89% |
| | 102113-98-4 | | | |

### Zu Beispiel 5v:

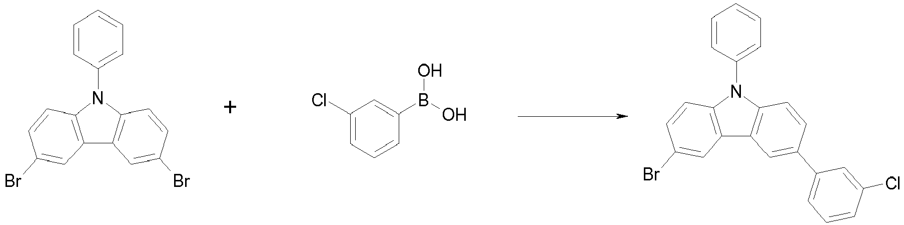

Es werden 50.0g (125mmol, 1.0eq) 3,6-Dibromo-9-phenylcarbazol (CAS 57103-20-5) zusammen mit 19.5g (125mmol, 1.0eq) 3-Chlorbenzolboronsäure (CAS 6350-60-6) in einem Gemisch aus 400ml Wasser, 400ml Dioxan und 400ml Toluol vorgelegt und für 30 Minuten entgast. Nach Zugabe von 280mg (1.25mmol, 1mol-%) Palladium(II)-acetat und 1.14g (3.75mmol, 3mol-%) Tri-o-tolylphoshin wird der Ansatz über Nacht unter Rücklfluss erhitzt und nach beendeter Reaktion mit etwas Wasser versetzt. Die organische Phase wird abgetrennt und zweifach mit Wasser extrahiert. Nach Trocknung der organischen Phase über Natriumsulfat wird der Rückstand aus Heptan/Toluol umkristallisiert. Es werden 44.8g (103mmol, 83%) eines beigen Feststoffs erhalten.

### Schritt 3: Boronierung

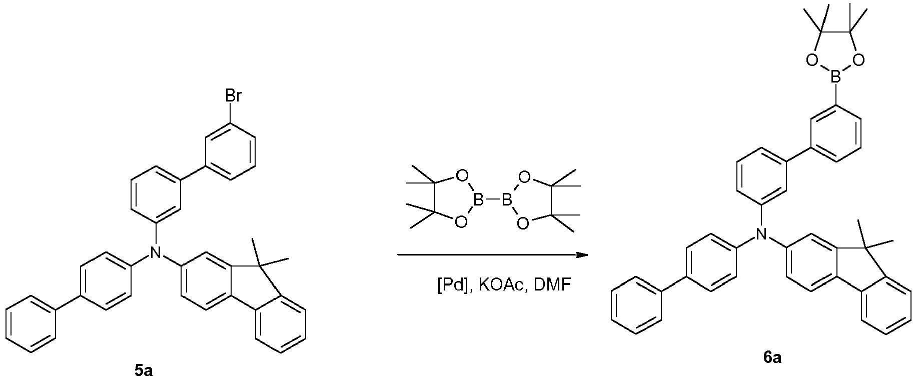

In einem 500ml-Kolben werden unter Schutzgas 16.6g (28mmol, 35%) des Bromids 5a zusammen mit 8.5g (34mmol, 1.2 eq) Bis-(pinacolato)-diboran (CAS 73183-34-3) in 120ml trockenem DMF gelöst und für 30 Minuten entgast. Anschließend werden 8.2g (84mmol, 3.0eq) Kaliumacetat und 690mg (0.84mmol, 3mol-%) [1,1'-Bis(diphenylphosphino)ferrocen]-dichlorpalladium(II)-Komplex mit Dichlormethan (CAS 95464-05-4) zugegeben und der Ansatz über Nacht auf 90°C erhitzt. Nach beendeter Reaktion wird mit 300ml Toluol verdünnt und das Gemisch mit Wasser extrahiert. Das Lösungsmittel wird am Rotationsverdampfer entfernt und der erhaltene Feststoff 14.7g (23mmol, 82%) getrocknet. Der Boronester **6a** wird ohne weitere Aufreinigung umgesetzt.

Analog können folgende Verbindungen erhalten werden:

| **Nr**. | **Edukt 5** | **Produkt 6** | **Ausbeute** |
|---|---|---|---|
| **6b** | 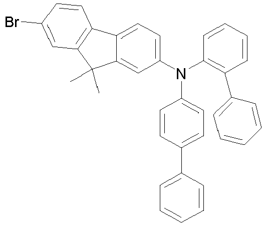 | 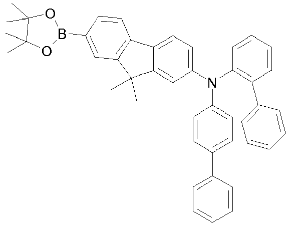 | 88% |
| **6c** | 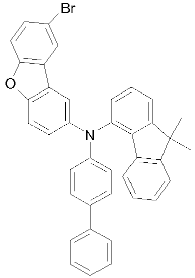 | 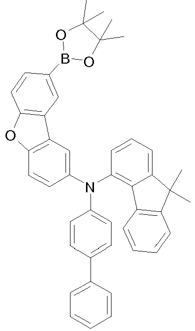 | 81% |
| **6d** | 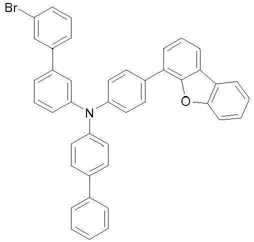 | 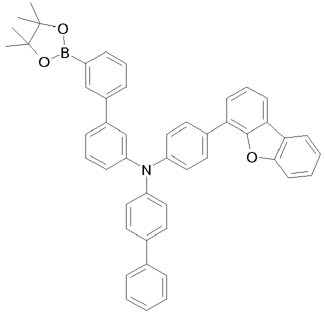 | 75% |
| **6e** | 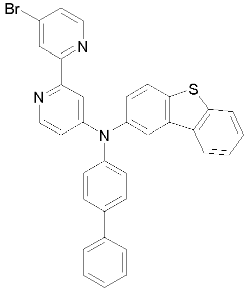 | 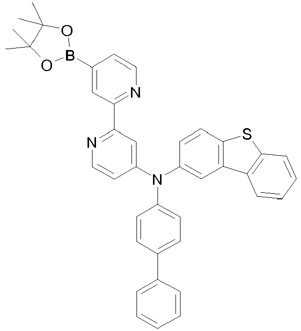 | 67% |
| **6f** | 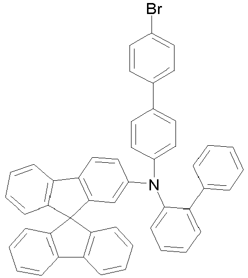 | 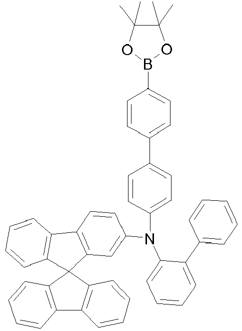 | 79% |
| **6g** | 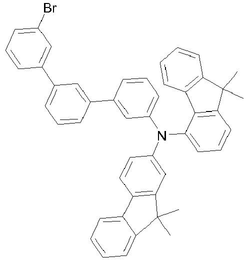 | 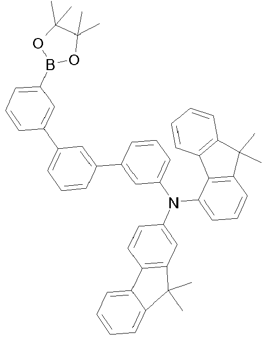 | 93% |
| **6h** | 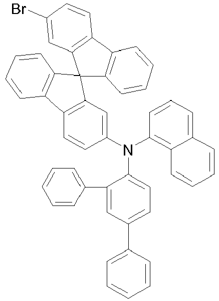 | 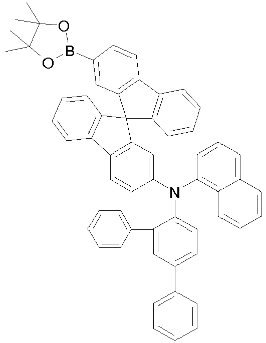 | 44% |
| **6i** | 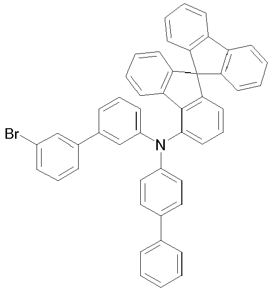 | 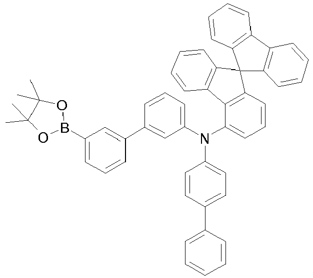 | 87% |
| **6j** | 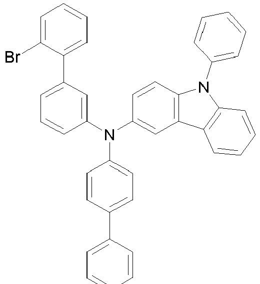 | 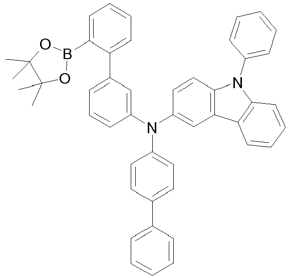 | 28% |
| **6k** | 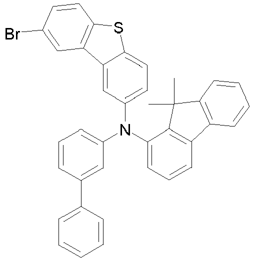 | 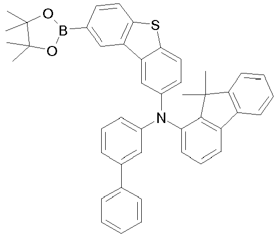 | 35% |
| **6l** | 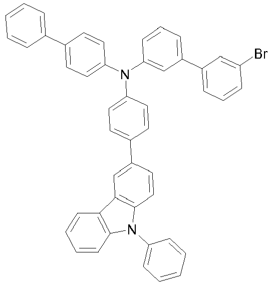 | 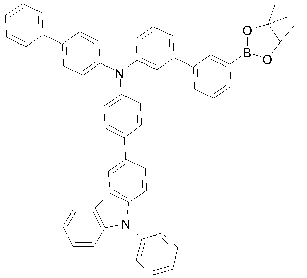 | 77% |
| **6m** | 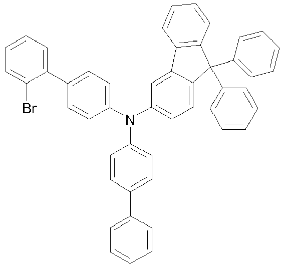 | 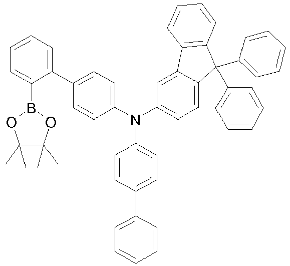 | 38% |
| **6n** | 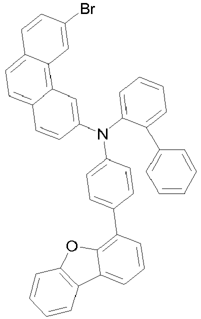 | 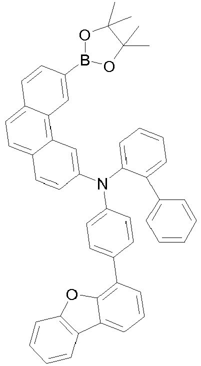 | 55% |
| **6o** | 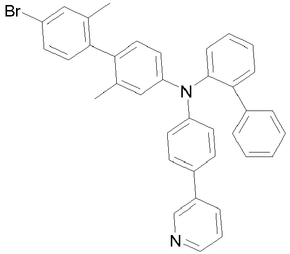 | 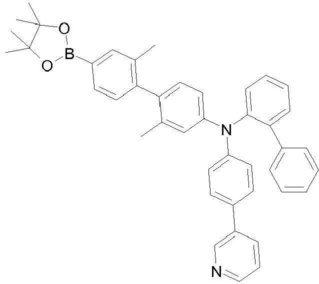 | 41% |
| **6p** | 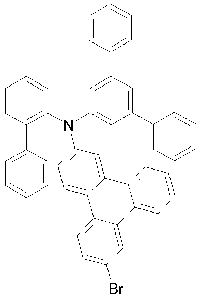 | 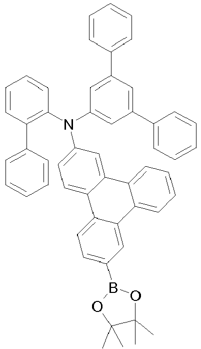 | 67% |
| **6q** | 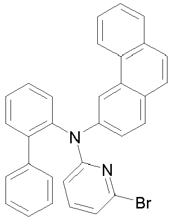 | 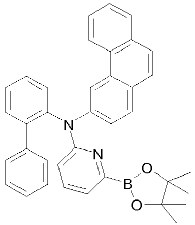 | 82% |
| **6r** | 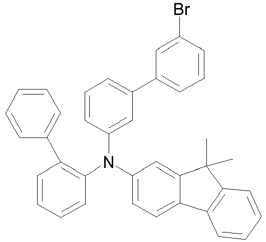 | 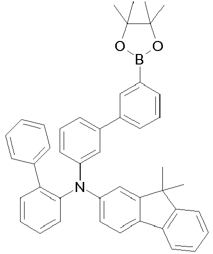 | 91% |
| **6s** | 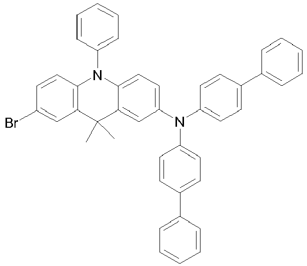 | 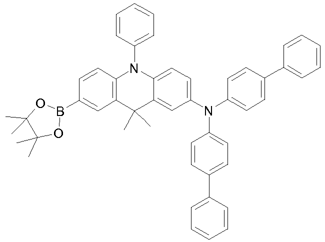 | 87% |
| **6t** | 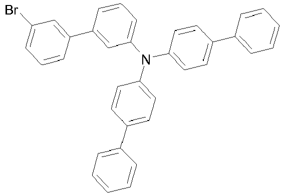 | 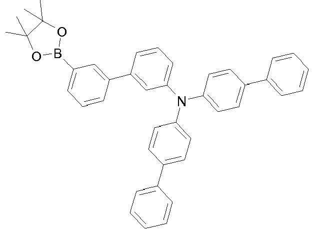 | 96% |
| **6u** | 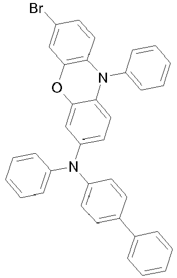 | 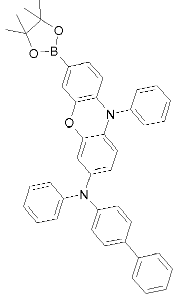 | 58% |
| **6v** | 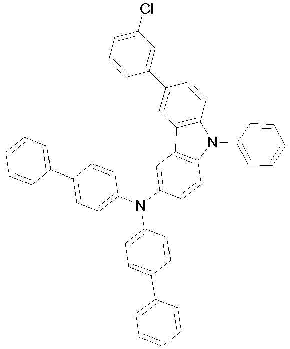 | 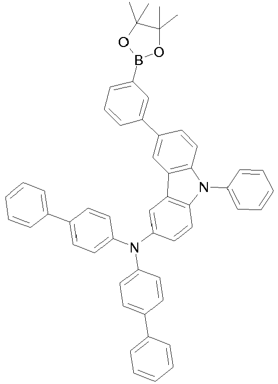 | 94% |

### Schritt 4:

### Synthese des Triazin-Bausteins Stufe 1

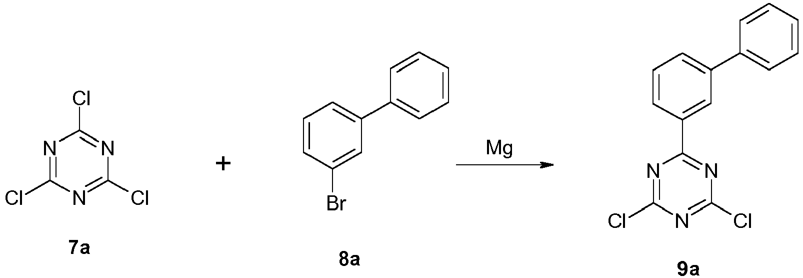

7.9g (330mmol, 1.2eq) Magnesiumspäne werden in einem 1L-Vierhalskolben vorgelegt und so langsam mit einer THF-Lösung aus 63g (270mmol, 1.0eq) 3-Bromobiphenyl **8a** (CAS 2113-57-7) versetzt, um den Rückfluss des Reaktionsgemisches zu erhalten. Nach beendeter Zugabe wird der Ansatz für zwei weitere Stunden unter Rückfluss erhitzt.
In einem 2L-Vierhalskolben werden 50g (270mmol, 1eq) 2,4,6-Trichloro-[1,3,5]triazin **7a** (CAS 108-77-0) in 500ml THF auf-10°C abgekühlt. Bei dieser Temperatur wird die Grignard-Lösung so langsam zugetropft, dass die Temperatur 0°C nicht übersteigt und der Ansatz schließlich über Nacht bei Raumtemperatur gerührt. Zur Aufarbeitung werden 270ml einer 1N Salzsäure zugetropft und das Gemisch für eine Stunde gerührt. Anschließend wird die wässrige Phase abgetrennt und mit Diethylether extrahiert. Die vereinten organischen Phasen werden über Natriumsulfat getrocknet und das Lösungsmittel am Rotationsverdampfer entfernt. Es werden 56g (69%) eines farblosen Öls **9a** erhalten.

Analog können folgende Verbindungen erhalten werden:

| **Nr.** | **Edukt 7** | **Edukt 8** | **Produkt 9** | **Ausbeute** |
|---|---|---|---|---|
| **9b** | 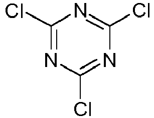 | 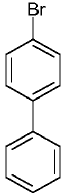 | 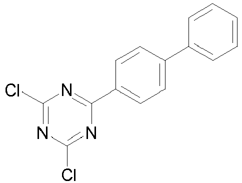 | 56% |
| | | 92-66-0 | | |
| **9c** | 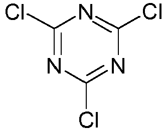 | 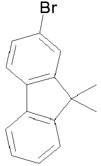 | 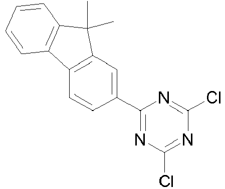 | 27% |
| | | 28320-31-2 | | |
| **9d** | 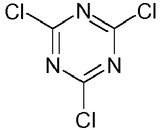 | 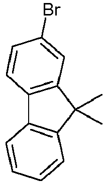 | 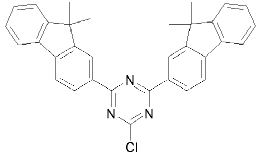 | 48% |
| | | 28320-31-2 Jeweils 2 Äq. eingesetzt | | |
| **9e** | 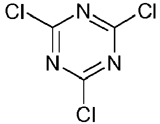 | 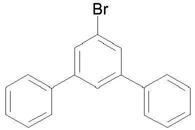 | 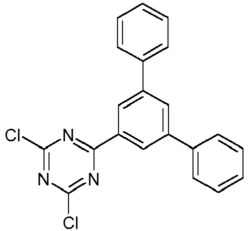 | 71% |
| | | 103068-20-8 | | |

### Synthese des Triazin-Bausteins Stufe 2

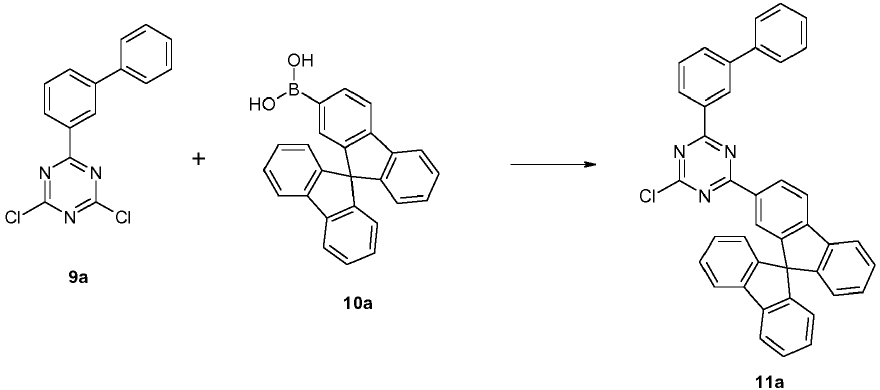

### Variante A:

18g (50mmol, 1eq) 9,9-Spirobifluoren-2-yl-boronsäure **10a** (CAS werden zusammen mit 15g (50mmol, 1eq) 2-Biphenyl-3-yl-4,6-dichloro-[1,3,5]triazin **9a** und 5.8g (55mmol, 1.1eq) Natriumcarbonat in einem Gemisch aus 200ml Dioxan, 200ml Toluol und 70ml Wasser gelöst und für 30 Minuten entgast. Anschließend werden 580mg (0.50 mmol, 1 mol-%) Tetrakis(triphenylphosphin) (CAS 14221-01-3) zugegeben und der Ansatz über Nacht am Rückfluss erhitzt. Das Reaktionsgemisch wird abgekühlt und mit 300ml Wasser versetzt. Die wässrige Phase wird dreimal mit Ethylacetat extrahiert, die organischen Phasen vereint und das Lösungsmittel am Rotationsverdampfer entfernt. Nach Heißextraktion in Heptan/Toluol 4:1 werden 15g (26mmol, 51%) eines farblosen Feststoffs erhalten.

### Variante B: analog Stufe 1

Analog sind folgende Verbindungen herzustellen:

| | **Variante** | **Edukt 9** | **Edukt 10** | **Produkt 11** | **Ausbeute** |
|---|---|---|---|---|---|
| **9b** | B | 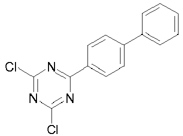 | 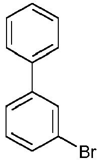 | 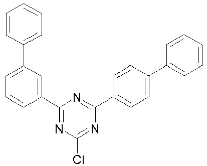 | 68% |
| | | | 2113-57 -7 | | |
| **9c** | A | 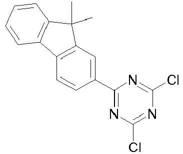 | 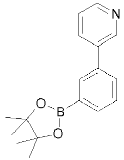 | 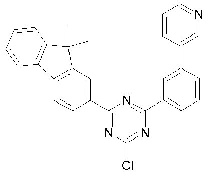 | 81% |
| | | | 939430-30-5 | | |
| **9d** | A | 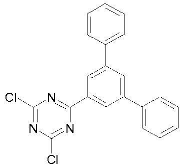 | 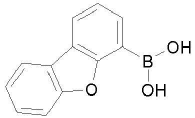 | 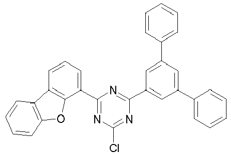 | 63% |
| | | | 100124-06-9 | | |
| **9e** | A | 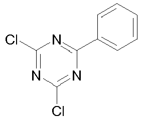 | 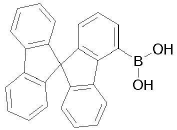 | 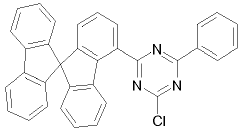 | 71% |
| | | | 1421789-05-0 | | |
| **9f** | A | 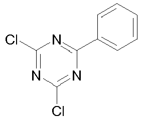 | 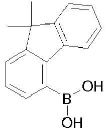 | 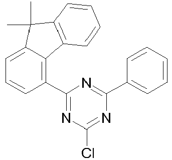 | 53% |
| | | | 1246022-50-3 | | |
| **9g** | A | 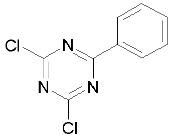 | 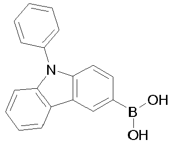 | 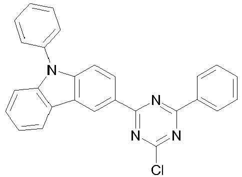 | 68% |
| | | | 854952-58-2 | | |
| **9h** | B | 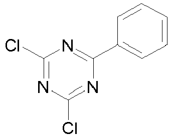 | 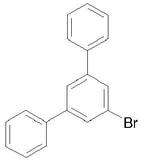 | 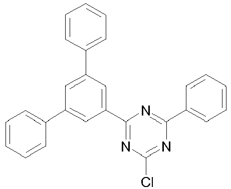 | 67% |
| | | | 103068-20-8 | | |

### Schritt 5: Darstellung des Endprodukts mittels Suzuki-Kupplung

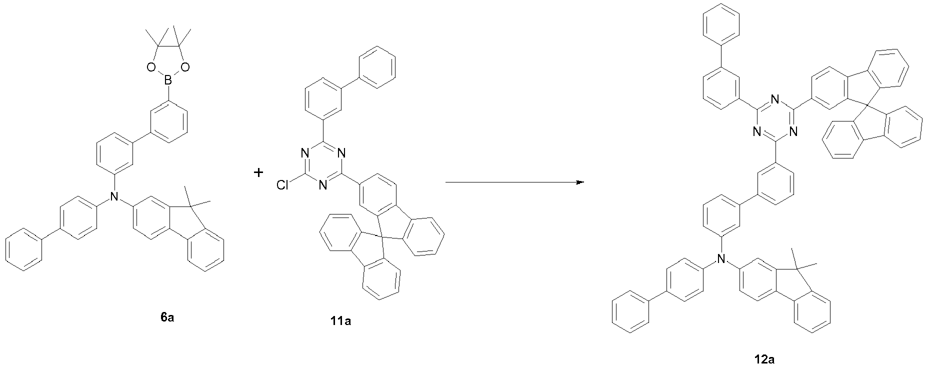

Es werden 14.7 (23mmol, 1.0eq) des Boronesters **6a** und 14.7g (25mmol, 1.1eq) des Triazin-Bausteins **11a** in je 190ml Toluol und Wasser suspendiert und für 30 Minuten entgast. Anschließend werden 7.0g (51mmol, 2.2eq) Kaliumcarbonat, 150mg (6.9mmol, 3mol-%) Palladium(II)-acetat und 300mg (1.2mmol, 5mol-%) Triphenylphosphin zugegeben und über Nacht unter Rückfluss erhitzt. Der ausgefallene Feststoff wird abgesaugt und mittels Heißextraktion mit Heptan/Toluol 1:1 und dreifacher Umkristallisation aus einem Heptan/Toluol-Gemisch aufgereinigt. Nach Sublimation werden 11.2g (11mmol, 46%) des gewünschten Produkts **12a** mit einer HPLC-Reinheit von >99.9% erhalten.

Analog können folgende Verbindungen erhalten werden:

| **Nr.** | **Edukt 6** | **Edukt 11** | **Produkt 12** | **Ausbeute** |
|---|---|---|---|---|
| **12b** | 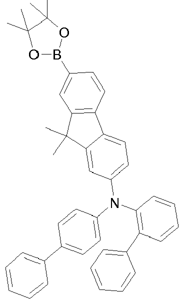 | 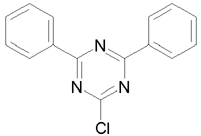 | 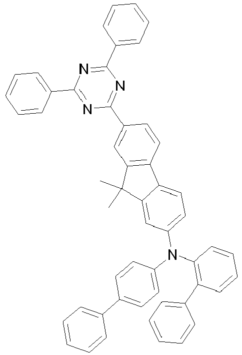 | 54% |
| | | 3842-55-5 | | |
| **12c** | 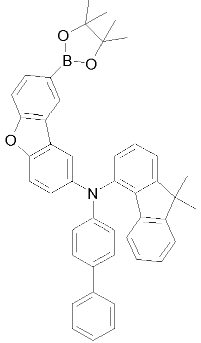 | 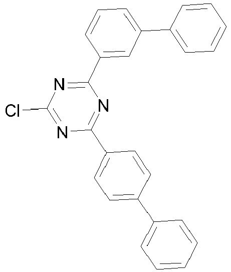 | 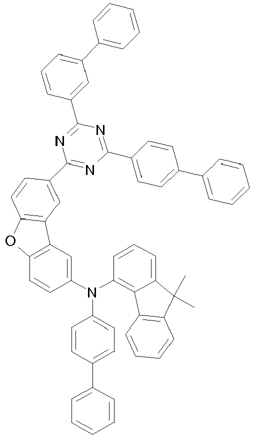 | 31% |
| **12d** | 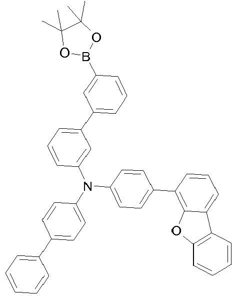 | 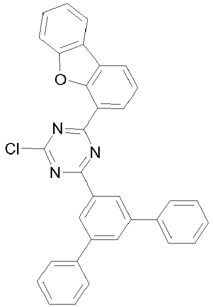 | 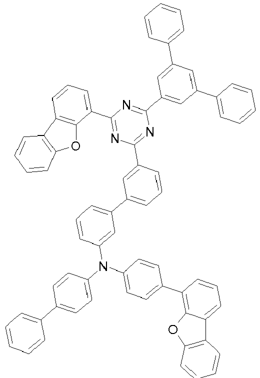 | 49% |
| **12e** | 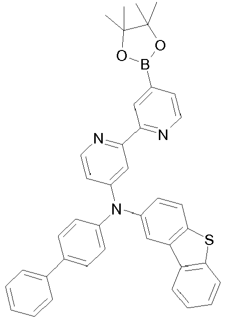 | 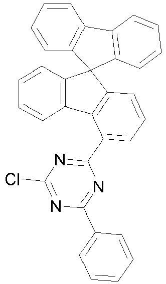 | 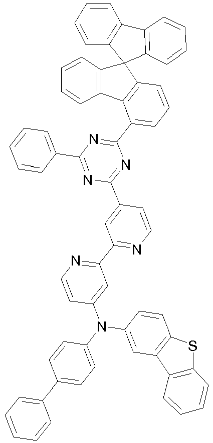 | 68% |
| **12f** | 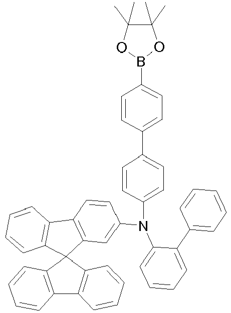 | 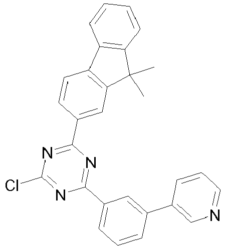 | 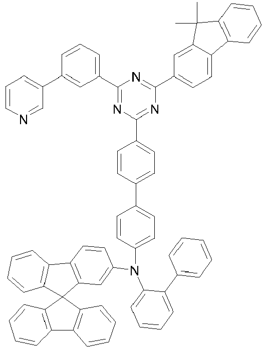 | 52% |
| **12g** | 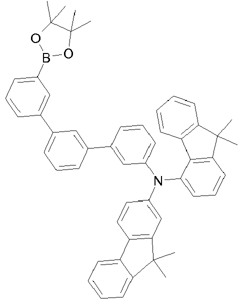 | 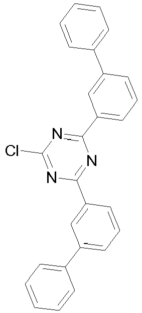 | 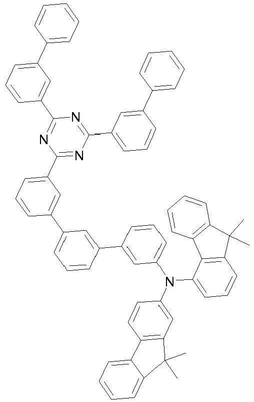 | 71% |
| | | 1205748-61-3 | | |
| **12h** | 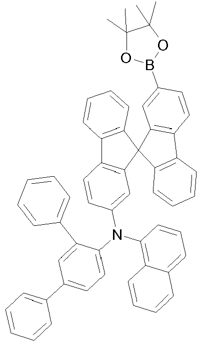 | 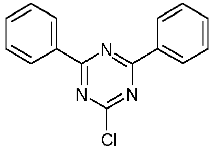 | 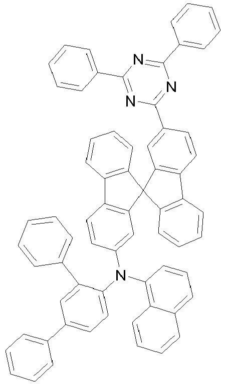 | 41% |
| | | 3842-55-5 | | |
| **12i** | 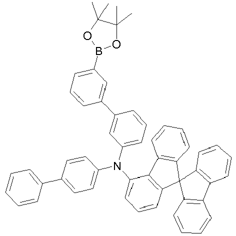 | 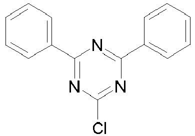 | 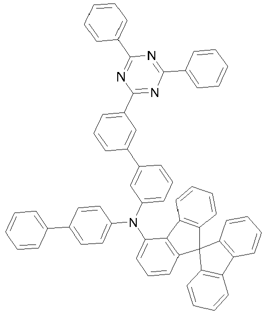 | 44% |
| | | 3842-55-5 | | |
| **12j** | 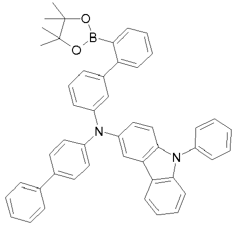 | 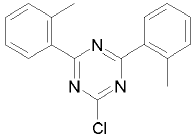 | 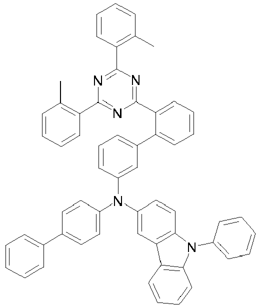 | 37% |
| | | 78941-34-1 | | |
| **12k** | 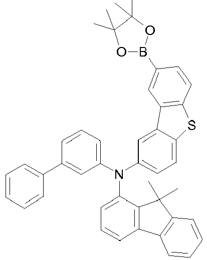 | 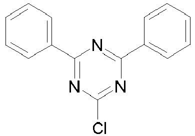 | 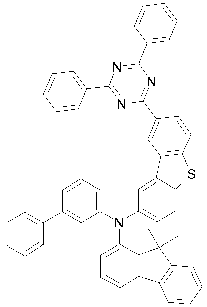 | 21% |
| | | 3842-55-5 | | |
| **12l** | 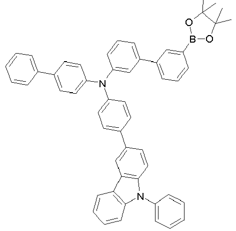 | 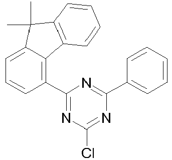 | 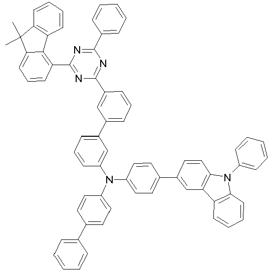 | 51% |
| **12m** | 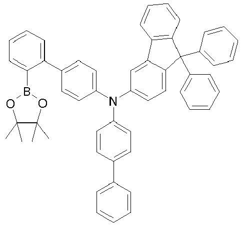 | 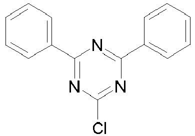 | 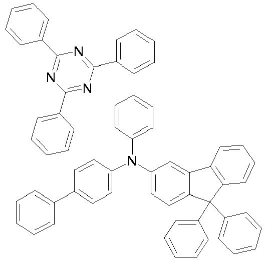 | 17% |
| | | 3842-55-5 | | |
| **12n** | 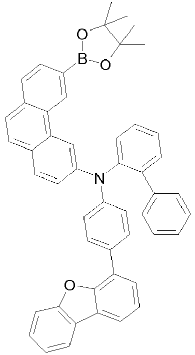 | 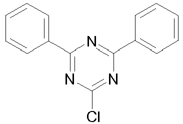 | 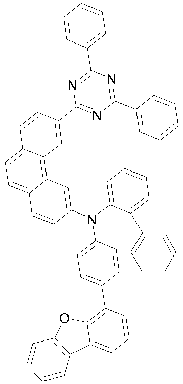 | 58% |
| | | 3842-55-5 | | |
| **12o** | 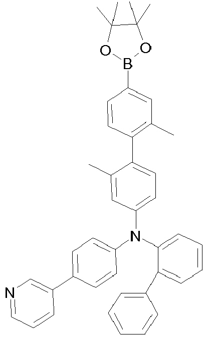 | 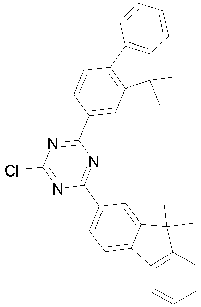 | 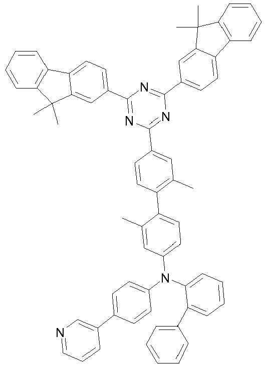 | 74% |
| **12p** | 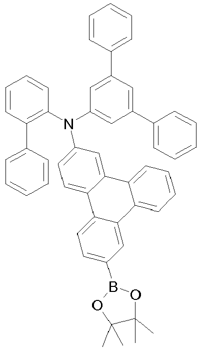 | 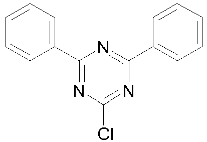 | 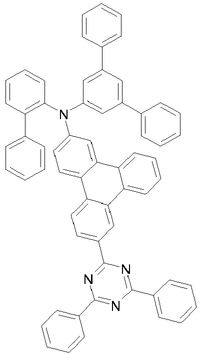 | 29% |
| | | 3842-55-5 | | |
| **12q** | 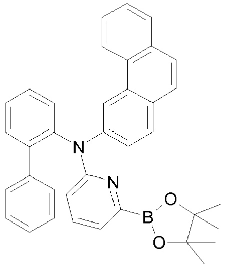 | 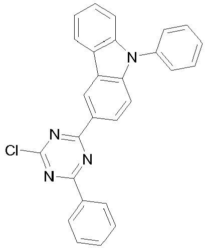 | 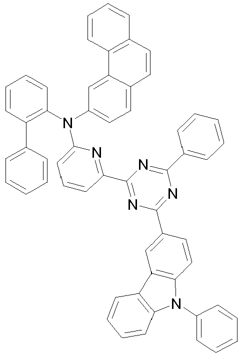 | 14% |
| **12r** | 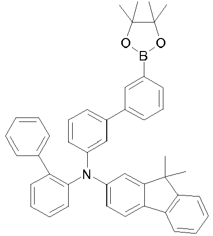 | 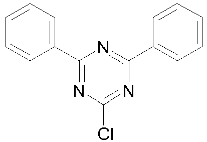 | 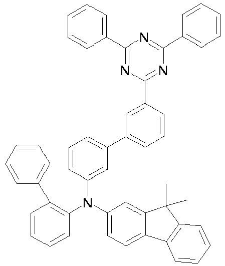 | 36% |
| | | 3842-55-5 | | |
| **12s** | 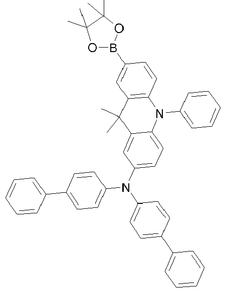 | 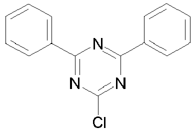 | 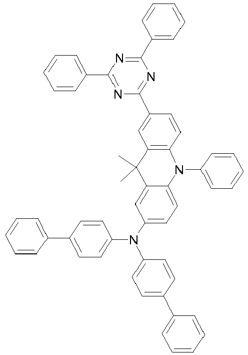 | 82% |
| | | 3842-55-5 | | |
| **12t** | 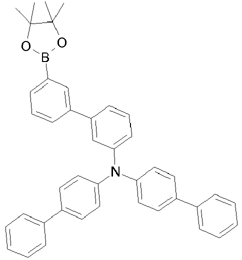 | 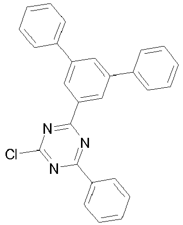 | 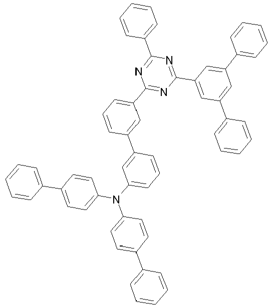 | 75% |
| **12u** | 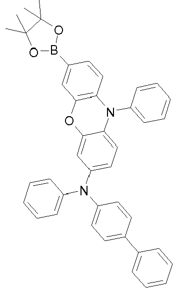 | 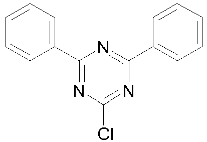 | 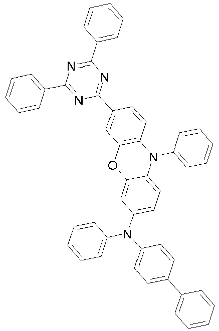 | 66% |
| | | 3842-55-5 | | |
| **12v** | 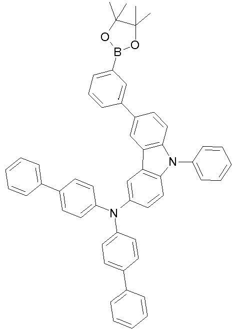 | 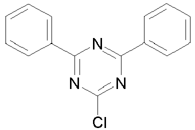 | 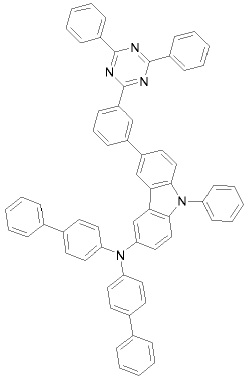 | 73% |
| | | 3842-55-5 | | |

### B) Device-Beispiele

### B-1) Herstellung der OLEDs

Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs verwendeten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie 12r:IC1:TEG1 (60%:30%:10%) bedeutet hierbei, dass das Material 12r in einem Volumenanteil von 60%, IC1 in einem Anteil von 30% und TEG1 in einem Anteil von 10% in der Schicht vorliegt. Analog besteht die Elektronentransportschicht aus einer Mischung von zwei Materialien.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 80% in Tabelle X2 bedeutet, dass die in Spalte LD angegebene Lebensdauer der Zeit entspricht, nach der die Anfangsleuchtdichte von 4000 cd/m² auf 3200 cd/m² absinkt. Analog bedeutet L0;j0 = 20mA/cm², L1 = 80%, dass die anfängliche Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiel V1-V3 sind Vergleichsbeispiele gemäß dem Stand der Technik, die Beispiele E1-E8 zeigen Daten von OLEDs mit erfindungsgemäßen Materialien.

Im folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

Mit den erfindungsgemäßen Verbindungen (s. Beispiele E1 bis E8) werden allgemein sehr gute Leistungsdaten der OLEDs erreicht, insbesondere sehr gute Lebensdauer, Leistungseffizienz und Betriebsspannung.

Im Vergleich zur Verbindung C1 zeigen die Verbindungen 12r und 12i, die an der Aminogruppe mit größeren aromatischen Systemen substituiert sind, bei Einsatz als Matrixmaterial Verbesserungen bezüglich Effizienz und Spannung, vor allem aber in Bezug auf Lebensdauer (Beispiele V1, E1 und E6).

Eine Verbesserung ergibt sich auch im Vergleich zur Verbindung C2, die ebenfalls nur kleine aromatische Systeme an der Aminogruppe aufweist (Beispiele V2, E1 und E6).

Im Vergleich zur Verbindung C3, in der Amin und Triazin über eine Phenylgruppe verknüpft sind, erhält man mit beispielsweise der ähnlichen erfindungsgemäßen Verbindung 12t wesentlich bessere Effizienz und vergleichbare Lebensdauer (Beispiele V3, E2).

**Tabelle 1: Aufbau der OLEDs**

| Bsp. | HTL | IL | EBL | EML | HBL | ETL |
|---|---|---|---|---|---|---|
| | Dicke | Dicke | Dicke | Dicke | Dicke | Dicke |
| V1 | SpA1 | HATCN | SpMA1 | C1:TEG1 | IC1 | ST2:LiQ (50%:50%) |
| | 70nm | 5nm | 90nm | (90%:10%) 30nm | 10nm | 30nm |
| V2 | SpA1 | HATCN | SpMA1 | C2:TEG1 | IC1 | ST2:LiQ (50%:50%) |
| | 70nm | 5nm | 90nm | (90%:10%) 30nm | 10nm | 30nm |
| V3 | SpA1 | HATCN | SpMA1 | C3:TEG1 | IC1 | ST2:LiQ (50%:50%) |
| | 70nm | 5nm | 90nm | (90%:10%) 30nm | 10nm | 30nm |
| E1 | SpA1 | HATCN | SpMA1 | 12r:TEG1 | IC1 | ST2:LiQ (50%:50%) |
| | 70nm | 5nm | 90nm | (90%:10%) 30nm | 10nm | 30nm |
| E2 | SpA1 | HATCN | SpMA1 | 12t:TEG1 | IC1 | ST2:LiQ (50%:50%) |
| | 70nm | 5nm | 90nm | (90%:10%) 30nm | 10nm | 30nm |
| E3 | SpA1 | HATCN | SpMA1 | 12b:TER3 | IC1 | ST2:LiQ (50%:50%) |
| | 90nm | 5nm | 130nm | (92%:8%)40nm | 10nm | 30nm |
| E4 | SpA1 | HATCN | SpMA1 | 12c:TEG1 | IC1 | ST2:LiQ (50%:50%) |
| | 70nm | 5nm | 90nm | (90%:10%) 30nm | 10nm | 30nm |
| E5 | SpA1 | HATCN | SpMA1 | 12d:TEG1 | IC1 | ST2:LiQ (50%:50%) |
| | 70nm | 5nm | 90nm | (90%:10%) 30nm | 10nm | 30nm |
| E6 | SpA1 | HATCN | SpMA1 | 12i:TEG1 | IC1 | ST2:LiQ (50%:50%) |
| | 70nm | 5nm | 90nm | (90%:10%) 30nm | 10nm | 30nm |
| E7 | SpA1 | HATCN | SpMA1 | 12p:TER3 | IC1 | ST2:LiQ (50%:50%) |
| | 90nm | 5nm | 130nm | (92%:8%)40nm | 10nm | 30nm |
| E8 | SpA1 | HATCN | SpMA1 | 12v:TER3 | IC1 | ST2:LiQ (50%:50%) |
| | 90nm | 5nm | 130nm | (92%:8%) 40nm | 10nm | 30nm |

**Tabelle 2: Daten der OLEDs**

| Bsp. | U1000 (V) | SE1000 (cd/A) | LE1000 (Im/W) | EQE 1000 | CIE x/y bei 1000 cd/m² | L0;j0 | L1 % | LD (h) |
|---|---|---|---|---|---|---|---|---|
| V1 | 3.3 | 60 | 57 | 16.9% | 0.34/0.62 | 20mA/cm² | 80 | 70 |
| V2 | 3.6 | 50 | 44 | 14.3% | 0.36/0.61 | 20mA/cm² | 80 | 95 |
| V3 | 3.4 | 55 | 50 | 15.4% | 0.34/0.62 | 20mA/cm² | 80 | 125 |
| E1 | 3.2 | 63 | 62 | 17.8% | 0.34/0.62 | 20mA/cm² | 80 | 110 |
| E2 | 3.3 | 61 | 58 | 17.1% | 0.34/0.62 | 20mA/cm² | 80 | 135 |
| E3 | 4.3 | 11.0 | 8.0 | 11.9% | 0.67/0.33 | 4000 cd/m² | 80 | 330 |
| E4 | 3.4 | 58 | 53 | 16.3% | 0.34/0.62 | 20mA/cm² | 80 | 90 |
| E5 | 3.3 | 64 | 61 | 18.1% | 0.34/0.62 | 20mA/cm² | 80 | 120 |
| E6 | 3.2 | 60 | 59 | 17.0% | 0.35/0.62 | 20mA/cm² | 80 | 115 |
| E7 | 4.8 | 10.4 | 6.8 | 11.3% | 0.67/0.33 | 4000 cd/m² | 80 | 270 |
| E8 | 4.4 | 12.4 | 8.8 | 12.8% | 0.66/0.34 | 4000 cd/m² | 80 | 305 |

**Tabelle 3: Strukturformeln der Materialien für die OLEDs**

| | |
|---|---|
| 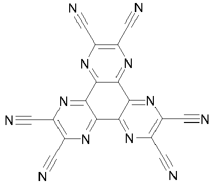 | 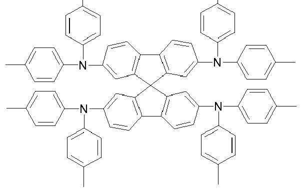 |
| HATCN | SpA1 |
| 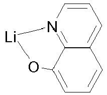 | 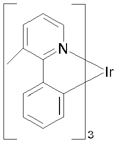 |
| LiQ | TEG1 |
| 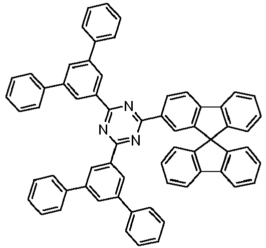 | 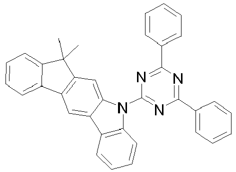 |
| ST2 | IC1 |
| 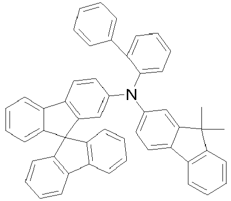 | 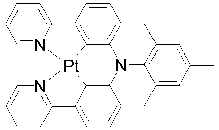 |
| SpMA1 | TER3 |
| 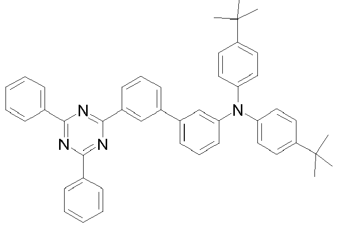 | 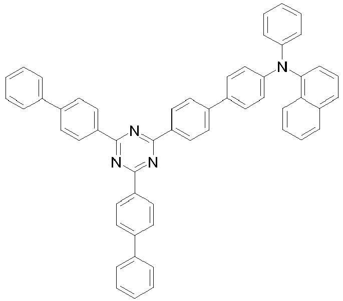 |
| C1 | C2 |
| 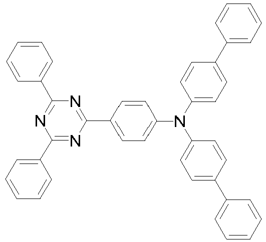 | |
| C3 | |
| 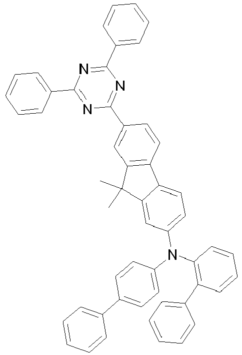 | 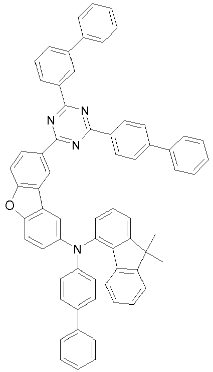 |
| 12b | 12c |
| 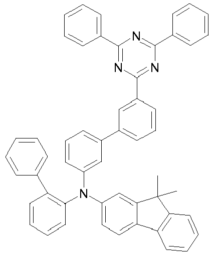 | 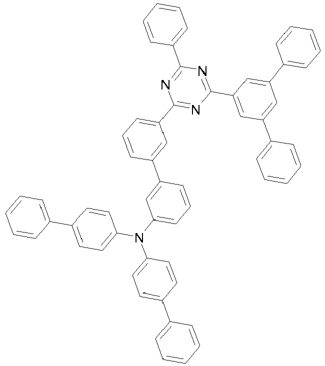 |
| 12r | 12t |
| 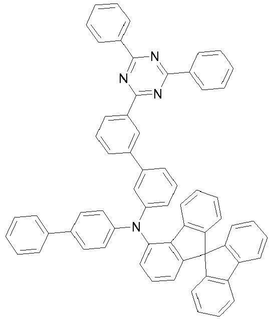 | 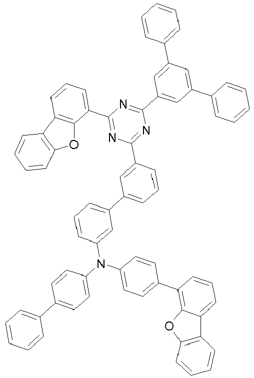 |
| 12i | 12d |
| 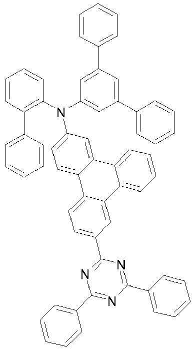 | 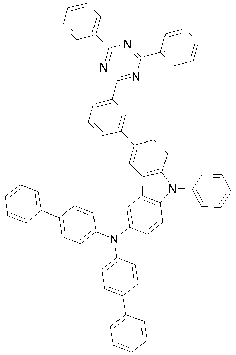 |
| 12p | 12v |

## Patentansprüche

1. Verbindung der Formel (I) 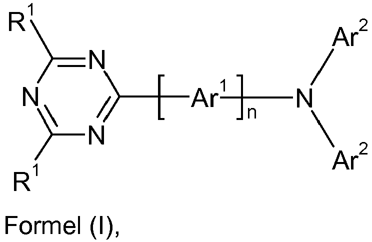 wobei gilt:
Ar¹ ist bei jedem Auftreten gleich oder verschieden ein aromatisches oder heteroaromatisches Ringsystem mit 5 bis 24 aromatischen Ringatomen, das mit einem oder mehreren Resten R² substituiert sein kann;
Ar² ist bei jedem Auftreten gleich oder verschieden ein aromatisches oder heteroaromatisches Ringsystem mit 5 bis 24 aromatischen Ringatomen, das mit einem oder mehreren Resten R² substituiert sein kann, wobei mindestens eine Gruppe Ar² in der Verbindung der Formel (I) eine Gruppe Ar²* darstellt;
Ar²* ist bei jedem Auftreten gleich oder verschieden ein aromatisches oder heteroaromatisches Ringsystem mit 12 bis 24 aromatischen Ringatomen, das mit einem oder mehreren Resten R² substituiert sein kann, wobei das aromatische Ringsystem keine kondensierte Aryl- oder Heteroarylgruppe mit mehr als 10 aromatischen Ringatomen umfasst;
R¹ ist bei jedem Auftreten gleich oder verschieden gewählt aus einem aromatischen oder heteroaromatischen Ringsystem mit 5 bis 30 aromatischen Ringatomen, das mit einem oder mehreren Resten R³ substituiert sein kann;
R² ist bei jedem Auftreten gleich oder verschieden gewählt aus H, einer geradkettigen Alkylgruppe mit 1 bis 10 C-Atomen oder einer verzweigten Alkylgruppe mit 3 bis 10 C-Atomen, wobei die oben genannten Gruppen jeweils mit einem oder mehreren Resten R³ substituiert sein können, oder einem aromatischen oder heteroaromatischen Ringsystem mit 5 bis 20 aromatischen Ringatomen, das jeweils mit einem oder mehreren Resten R³ substituiert sein kann;
R³ ist bei jedem Auftreten gleich oder verschieden gewählt aus H, einer geradkettigen Alkylgruppe mit 1 bis 10 C-Atomen oder einer verzweigten Alkylgruppe mit 3 bis 10 C-Atomen, oder einem aromatischen oder heteroaromatischen Ringsystem mit 5 bis 20 aromatischen Ringatomen;
n ist gleich 1, 2, 3 oder 4;
wobei für n=1 die Gruppe Ar¹ nicht wahlweise mit R² substituiertes Phenylen oder wahlweise mit R² substituiertes Carbazol ist; und
wobei Ar¹ nicht über die Positionen 9 und 9' gebundenes, wahlweise mit R² substituiertes Fluoren ist; und
wobei die Verbindung der Formel (I) genau eine Aminogruppe enthält.

2. Verbindung nach Anspruch 1, **dadurch gekennzeichnet, dass** sie genau eine Triazingruppe enthält.

3. Verbindung nach einem oder mehreren der Ansprüche 1 oder 2, **dadurch gekennzeichnet, dass** sie keine kondensierte Arylgruppe mit mehr als 14 aromatischen Ringatomen enthält.

4. Verbindung nach einem oder mehreren der Ansprüche 1 bis 3, **dadurch gekennzeichnet, dass** Ar¹ bei jedem Auftreten gleich oder verschieden gewählt ist aus wahlweise mit einem oder mehreren Resten R² substituiertem Phenyl, Biphenyl, Terphenyl, Fluorenyl, Spirobifluorenyl, Indenofluorenyl, Naphthyl, Anthracenyl, Phenanthrenyl, Chrysenyl, Benzanthracenyl, Pyrenyl, Fluoranthenyl, Triphenylenyl, Furanyl, Benzofuranyl, Dibenzofuranyl, Thiophenyl, Benzothiophenyl, Dibenzothiophenyl, Indolyl, Carbazolyl, Indolocarbazolyl, Indenocarbazolyl, Pyridyl, Chinolinyl, Acridyl, Dihydroacridyl, Pyrazolyl, Imidazolyl, Benzimidazolyl, Pyridazyl, Pyrimidyl, Pyrazinyl, Phenanthrolyl, Diphenylether, Diphenylthioether, Diphenylsilylen und Diphenylmethylen.

5. Verbindung nach einem oder mehreren der Ansprüche 1 bis 4, **dadurch gekennzeichnet, dass** die Einheit -(Ar¹)ₙ- gewählt ist aus wahlweise mit einem oder mehreren Resten R² substituiertem Fluorenyl, Spirobifluorenyl, Indenofluorenyl, Naphthyl, Anthracenyl, Phenanthrenyl, Chrysenyl, Benzanthracenyl, Pyrenyl, Fluoranthenyl, Furanyl, Benzofuranyl, Dibenzofuranyl, Thiophenyl, Benzothiophenyl, Dibenzothiophenyl, Indolyl, Indolocarbazolyl, Indenocarbazolyl, Pyridyl, Chinolinyl, Acridyl, Dihydroacridyl, Pyrazolyl, Imidazolyl, Benzimidazolyl, Pyridazyl, Pyrimidyl, Pyrazinyl, Phenanthrolyl, Diphenylether, Diphenylthioether, Diphenylsilylen und Diphenylmethylen und aus Kombinationen enthaltend eine oder mehrere der oben genannten Gruppen und eine oder mehrere weitere Gruppen, gewählt aus wahlweise mit einem oder mehreren Resten R² substituiertem Phenyl und Carbazolyl.

6. Verbindung nach einem oder mehreren der Ansprüche 1 bis 5, **dadurch gekennzeichnet, dass** die Einheit -(Ar¹)ₙ- eine vollständig konjugierte ungesättigte divalente Gruppe darstellt.

7. Verbindung nach einem oder mehreren der Ansprüche 1 bis 6, **dadurch gekennzeichnet, dass** beide Gruppen Ar² gewählt sind aus gleichen oder verschiedenen Gruppen Ar²*.

8. Verbindung nach einem oder mehreren der Ansprüche 1 bis 7, **dadurch gekennzeichnet, dass** Ar² bei jedem Auftreten gleich oder verschieden gewählt ist aus einem aromatischen oder heteroaromatischen Ringsystem mit 5 bis 20 aromatischen Ringatomen, das mit einem oder mehreren Resten R² substituiert sein kann.

9. Verbindung nach einem oder mehreren der Ansprüche 1 bis 8, **dadurch gekennzeichnet, dass** Ar² bei jedem Auftreten gleich oder verschieden gewählt ist aus wahlweise mit einem oder mehreren Resten R² substituiertem Phenyl, Biphenyl, Terphenyl, Quaterphenyl, Fluorenyl, Spirobifluorenyl, Indenofluorenyl, Naphthyl, Anthracenyl, Phenanthrenyl, Chrysenyl, Benzanthracenyl, Pyrenyl, Triphenylenyl, Fluoranthenyl, Furanyl, Benzofuranyl, Dibenzofuranyl, Thiophenyl, Benzothiophenyl, Dibenzothiophenyl, Indolyl, Carbazolyl, Indolocarbazolyl, Indenocarbazolyl, Pyridyl, Chinolinyl, Acridyl, Dihydroacridyl, Pyrazolyl, Imidazolyl, Benzimidazolyl, Pyridazyl, Pyrimidyl, Pyrazinyl und Phenanthrolyl.

10. Formulierung, enthaltend mindestens eine Verbindung nach einem oder mehreren der Ansprüche 1 bis 9 sowie mindestens ein Lösungsmittel.

11. Verwendung einer Verbindung nach einem oder mehreren der Ansprüche 1 bis 9 in einer organischen Elektrolumineszenzvorrichtung (OLED).

12. Organische Elektrolumineszenzvorrichtung, enthaltend Anode, Kathode sowie mindestens eine organische Schicht, wobei die organische Schicht mindestens eine Verbindung nach einem oder mehreren der Ansprüche 1 bis 9 enthält.

13. Organische Elektrolumineszenzvorrichtung nach Anspruch 12, **dadurch gekennzeichnet, dass** die Verbindung nach einem oder mehreren der Ansprüche 1 bis 9 als Matrixmaterial in einer emittierenden Schicht in Kombination mit einer oder mehreren Emitterverbindungen eingesetzt wird.

## Claims

1. Compound of the formula (I) 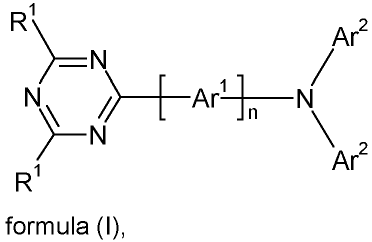 where:
Ar¹ is on each occurrence, identically or differently, an aromatic or heteroaromatic ring system having 5 to 24 aromatic ring atoms, which may be substituted by one or more radicals R²;
Ar² is on each occurrence, identically or differently, an aromatic or heteroaromatic ring system having 5 to 24 aromatic ring atoms, which may be substituted by one or more radicals R², where at least one group Ar² in the compound of the formula (I) represents a group Ar²*;
Ar²* is on each occurrence, identically or differently, an aromatic or heteroaromatic ring system having 12 to 24 aromatic ring atoms, which may be substituted by one or more radicals R², where the aromatic ring system contains no condensed aryl or heteroaryl group having more than 10 aromatic ring atoms;
R¹ is selected on each occurrence, identically or differently, from an aromatic or heteroaromatic ring system having 5 to 30 aromatic ring atoms, which may be substituted by one or more radicals R³;
R² is selected on each occurrence, identically or differently, from H, a straight-chain alkyl group having 1 to 10 C atoms or a branched alkyl group having 3 to 10 C atoms, where the above-mentioned groups may in each case be substituted by one or more radicals R³, or an aromatic or heteroaromatic ring system having 5 to 20 aromatic ring atoms, which may in each case be substituted by one or more radicals R³;
R³ is selected on each occurrence, identically or differently, from H, a straight-chain alkyl group having 1 to 10 C atoms or a branched alkyl group having 3 to 10 C atoms, or an aromatic or heteroaromatic ring system having 5 to 20 aromatic ring atoms;
n is equal to 1, 2, 3 or 4;
where, for n=1, the group Ar¹ is not phenylene which is optionally substituted by R² or carbazole which is optionally substituted by R²; and
where Ar¹ is not optionally R²-substituted fluorene bonded via positions 9 and 9' which is optionally substituted by R²; and
where the compound of the formula (I) contains precisely one amino group.

2. Compound according to Claim 1, **characterised in that** it contains precisely one triazine group.

3. Compound according to one or more of Claims 1 or 2, **characterised in that** it contains no condensed aryl group having more than 14 aromatic ring atoms.

4. Compound according to one or more of Claims 1 to 3, **characterised in that** Ar¹ is selected on each occurrence, identically or differently, from phenyl, biphenyl, terphenyl, fluorenyl, spirobifluorenyl, indenofluorenyl, naphthyl, anthracenyl, phenanthrenyl, chrysenyl, benzanthracenyl, pyrenyl, fluoranthenyl, triphenylenyl, furanyl, benzofuranyl, dibenzofuranyl, thiophenyl, benzothiophenyl, dibenzothiophenyl, indolyl, carbazolyl, indolocarbazolyl, indenocarbazolyl, pyridyl, quinolinyl, acridyl, dihydroacridyl, pyrazolyl, imidazolyl, benzimidazolyl, pyridazyl, pyrimidyl, pyrazinyl, phenanthrolyl, diphenyl ether, diphenyl thioether, diphenylsilylene and diphenylmethylene, each of which is optionally substituted by one or more radicals R².

5. Compound according to one or more of Claims 1 to 4, **characterised in that** the unit -(Ar¹)ₙ- is selected from fluorenyl, spirobifluorenyl, indenofluorenyl, naphthyl, anthracenyl, phenanthrenyl, chrysenyl, benzanthracenyl, pyrenyl, fluoranthenyl, furanyl, benzofuranyl, dibenzofuranyl, thiophenyl, benzothiophenyl, dibenzothiophenyl, indolyl, indolocarbazolyl, indenocarbazolyl, pyridyl, quinolinyl, acridyl, dihydroacridyl, pyrazolyl, imidazolyl, benzimidazolyl, pyridazyl, pyrimidyl, pyrazinyl, phenanthrolyl, diphenyl ether, diphenyl thioether, diphenylsilylene and diphenylmethylene, each of which is optionally substituted by one or more radicals R², and from combinations containing one or more of the above-mentioned groups and one or more further groups selected from phenyl and carbazolyl, each of which is optionally substituted by one or more radicals R².

6. Compound according to one or more of Claims 1 to 5, **characterised in that** the unit -(Ar¹)ₙ- represents a fully conjugated unsaturated divalent group.

7. Compound according to one or more of Claims 1 to 6, **characterised in that** both groups Ar² are selected from identical or different groups Ar²*.

8. Compound according to one or more of Claims 1 to 7, **characterised in that** Ar² is selected on each occurrence, identically or differently, from an aromatic or heteroaromatic ring system having 5 to 20 aromatic ring atoms, which may be substituted by one or more radicals R².

9. Compound according to one or more of Claims 1 to 8, **characterised in that** Ar² is selected on each occurrence, identically or differently, from phenyl, biphenyl, terphenyl, quaterphenyl, fluorenyl, spirobifluorenyl, indenofluorenyl, naphthyl, anthracenyl, phenanthrenyl, chrysenyl, benzanthracenyl, pyrenyl, triphenylenyl, fluoranthenyl, furanyl, benzofuranyl, dibenzofuranyl, thiophenyl, benzothiophenyl, dibenzothiophenyl, indolyl, carbazolyl, indolocarbazolyl, indenocarbazolyl, pyridyl, quinolinyl, acridyl, dihydroacridyl, pyrazolyl, imidazolyl, benzimidazolyl, pyridazyl, pyrimidyl, pyrazinyl and phenanthrolyl, each of which is optionally substituted by one or more radicals R².

10. Formulation comprising at least one compound according to one or more of Claims 1 to 9 and at least one solvent.

11. Use of a compound according to one or more of Claims 1 to 9 in an organic electroluminescent device (OLED).

12. Organic electroluminescent device comprising anode, cathode and at least one organic layer, where the organic layer comprises at least one compound according to one or more of Claims 1 to 9.

13. Organic electroluminescent device according to Claim 12, **characterised in that** the compound according to one or more of Claims 1 to 9 is employed as matrix material in an emitting layer in combination with one or more emitter compounds.

## Revendications

1. Composé de la formule (I) 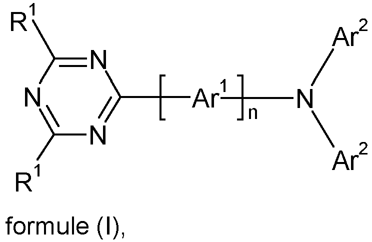 dans laquelle :
Ar¹ est pour chaque occurrence, de manière identique ou différente, un système de cycle aromatique ou hétéroaromatique qui comporte de 5 à 24 atomes de cycle aromatique, lequel peut être substitué par un radical ou par plusieurs radicaux R² ;
Ar² est pour chaque occurrence, de manière identique ou différente, un système de cycle aromatique ou hétéroaromatique qui comporte de 5 à 24 atomes de cycle aromatique, lequel peut être substitué par un radical ou par plusieurs radicaux R², où au moins un groupe Ar² dans le composé de la formule (I) représente un groupe Ar²* ;
Ar²* est pour chaque occurrence, de manière identique ou différente, un système de cycle aromatique ou hétéroaromatique qui comporte de 12 à 24 atomes de cycle aromatique, lequel peut être substitué par un radical ou par plusieurs radicaux R², où le système de cycle aromatique ne contient pas de groupe aryle ou hétéroaryle condensé qui comporte plus de 10 atomes de cycle aromatique ;
R¹ est sélectionné pour chaque occurrence, de manière identique ou différente, parmi un système de cycle aromatique ou hétéroaromatique qui comporte de 5 à 30 atomes de cycle aromatique, lequel peut être substitué par un radical ou par plusieurs radicaux R³ ;
R² est sélectionné pour chaque occurrence, de manière identique ou différente, parmi H, un groupe alkyle en chaîne droite qui comporte de 1 à 10 atome(s) de C ou un groupe alkyle ramifié qui comporte de 3 à 10 atomes de C, où les groupes qui ont été mentionnés ci-avant peuvent dans chaque cas être substitués par un radical ou par plusieurs radicaux R³, ou un système de cycle aromatique ou hétéroaromatique qui comporte de 5 à 20 atomes de cycle aromatique, lequel peut dans chaque cas être substitué par un radical ou par plusieurs radicaux R³ ;
R³ est sélectionné pour chaque occurrence, de manière identique ou différente, parmi H, un groupe alkyle en chaîne droite qui comporte de 1 à 10 atome(s) de C ou un groupe alkyle ramifié qui comporte de 3 à 10 atomes de C, ou un système de cycle aromatique ou hétéroaromatique qui comporte de 5 à 20 atomes de cycle aromatique ;
n est égal à 1, 2, 3 ou 4 ;
où, pour n = 1, le groupe Ar¹ n'est ni phénylène qui est en option substitué par R², ni carbazole qui est en option substitué par R² ; et
où Ar¹ n'est pas fluorène qui est lié via les positions 9 et 9', lequel est en option substitué par R² ; et
où le composé de la formule (I) contient de façon précise un seul groupe amino.

2. Composé selon la revendication 1, **caractérisé en ce qu'**il contient de façon précise un seul groupe triazine.

3. Composé selon une ou plusieurs des revendications 1 ou 2, **caractérisé en ce qu'**il ne contient pas de groupe aryle condensé qui comporte plus de 14 atomes de cycle aromatique.

4. Composé selon une ou plusieurs des revendications 1 à 3, **caractérisé en ce que** Ar¹ est sélectionné pour chaque occurrence, de manière identique ou différente, parmi phényle, biphényle, terphényle, fluorényle, spirobifluorényle, indénofluorényle, naphtyle, anthracényle, phénanthrényle, chrysényle, benzanthracényle, pyrényle, fluoranthényle, triphénylényle, furanyle, benzofuranyle, dibenzofuranyle, thiophényle, benzothiophényle, dibenzothiophényle, indolyle, carbazolyle, indolocarbazolyle, indénocarbazolyle, pyridyle, quinolinyle, acridyle, dihydroacridyle, pyrazolyle, imidazolyle, benzimidazolyle, pyridazyle, pyrimidyle, pyrazinyle, phénanthrolyle, éther de diphényle, thioéther de diphényle, diphénylsilylène et diphénylméthylène, dont chacun est en option substitué par un radical ou par plusieurs radicaux R².

5. Composé selon une ou plusieurs des revendications 1 à 4, **caractérisé en ce que** l'unité -(Ar¹)ₙ- est sélectionnée parmi fluorényle, spirobifluorényle, indénofluorényle, naphtyle, anthracényle, phénanthrényle, chrysényle, benzanthracényle, pyrényle, fluoranthényle, furanyle, benzofuranyle, dibenzofuranyle, thiophényle, benzothiophényle, dibenzothiophényle, indolyle, indolocarbazolyle, indénocarbazolyle, pyridyle, quinolinyle, acridyle, dihydroacridyle, pyrazolyle, imidazolyle, benzimidazolyle, pyridazyle, pyrimidyle, pyrazinyle, phénanthrolyle, éther de diphényle, thioéther de diphényle, diphénylsilylène et diphénylméthylène, dont chacun est en option substitué par un radical ou par plusieurs radicaux R², et parmi des combinaisons qui contiennent un ou plusieurs des groupes qui ont été mentionnés ci-avant et un ou plusieurs autre(s) groupe(s) qui est/sont sélectionné(s) parmi phényle et carbazolyle, dont chacun est en option substitué par un radical ou par plusieurs radicaux R².

6. Composé selon une ou plusieurs des revendications 1 à 5, **caractérisé en ce que** l'unité -(Ar¹)ₙ- représente un groupe divalent complètement conjugué non saturé.

7. Composé selon une ou plusieurs des revendications 1 à 6, **caractérisé en ce que** les deux groupes Ar² sont sélectionnés parmi des groupes Ar²* identiques ou différents.

8. Composé selon une ou plusieurs des revendications 1 à 7, **caractérisé en ce que** Ar² est sélectionné pour chaque occurrence, de manière identique ou différente, parmi un système de cycle aromatique ou hétéroaromatique qui comporte de 5 à 20 atomes de cycle aromatique, lequel peut être substitué par un radical ou par plusieurs radicaux R².

9. Composé selon une ou plusieurs des revendications 1 à 8, **caractérisé en ce que** Ar² est sélectionné pour chaque occurrence, de manière identique ou différente, parmi phényle, biphényle, terphényle, quater-phényle, fluorényle, spirobifluorényle, indénofluorényle, naphtyle, anthracényle, phénanthrényle, chrysényle, benzanthracényle, pyrényle, triphénylényle, fluoranthényle, furanyle, benzofuranyle, dibenzofuranyle, thiophényle, benzothiophényle, dibenzothiophényle, indolyle, carbazolyle, indolocarbazolyle, indénocarbazolyle, pyridyle, quinolinyle, acridyle, dihydroacridyle, pyrazolyle, imidazolyle, benzimidazolyle, pyridazyle, pyrimidyle, pyrazinyle et phénanthrolyle, dont chacun est en option substitué par un radical ou par plusieurs radicaux R².

10. Formulation comprenant au moins un composé selon une ou plusieurs des revendications 1 à 9 et au moins un solvant.

11. Utilisation d'un composé selon une ou plusieurs des revendications 1 à 9 dans un dispositif électroluminescent organique (OLED).

12. Dispositif électroluminescent organique comprenant une anode, une cathode et au moins une couche organique, dans lequel la couche organique comprend au moins un composé selon une ou plusieurs des revendications 1 à 9.

13. Dispositif électroluminescent organique selon la revendication 12, **caractérisé en ce que** le composé selon une ou plusieurs des revendications 1 à 9 est utilisé en tant que matériau de matrice dans une couche d'émission en combinaison avec un ou plusieurs composé(s) d'émetteur.
